# EUROPEAN PATENT APPLICATION

(11) **EP 1 640 365 A1**
(43) Date of publication of application: **29.03.2006**
(21) Application number: 04747158.6
(22) Date of filing: 01.07.2004
(51) Int. Cl.: C07D 213/57, C07D 213/79, C07D 217/14, C07D 215/12, C09K 11/06, H05B 33/14

(54) **METAL COMPLEX COMPOUND AND ORGANIC ELECTROLUMINESCENCE DEVICE CONTAINING THE SAME**

(30) Priority: 02.07.2003 JP 2003190374
(71) Applicant: IDEMITSU KOSAN CO., LTD., Tokyo 100-8321 (JP)
(72) Inventor: OKUDA, Fumio, Sodegaura-shi, Chiba 2990293 (JP); IWAKUMA, Toshihiro, Sodegaura-shi, Chiba 2990293 (JP); YAMAMICHI, Keiko, Sodegaura-shi, Chiba 2990293 (JP); HOSOKAWA, Chishio, Sodegaura-shi, Chiba 2990293 (JP)
(74) Representative: Gille Hrabal Struck Neidlein Prop Roos
(86) International application number: PCT/JP2004/009689
(87) International publication number: WO 2005/003095

(57) **Abstract**

A metal-complex compound having a special structure containing metals such as iridium. An organic electroluminescence device which comprises at least one organic thin film layer sandwiched between a pair of electrode consisting of an anode and a cathode, wherein the organic thin film layer comprises the metal-complex compound, which emits light by applying an electric voltage between the pair of electrode. An organic EL device employing the novel metal-complex compound emits various phosphorous light including blue light having an enhanced current efficiency and prolonged lifetime.

## Description

### TECHNICAL FIELD

The present invention relates to a novel metal-complex compound and an organic electroluminescence device using the compound. Particularly, the present invention relates to an organic electroluminescence device ("electroluminescence" will be referred to as "EL", hereinafter) having excellent efficiency of light emission and prolonged lifetime, and to a metal-complex compound realizing it.

### BACKGROUND ART

The organic EL devices have been expected to be applied to color wide screen image display devices replacing liquid crystal display devices, and have been intensively developed. Recently, although displays using the organic EL devices have now been used in practical applications, full-color image display devices using the same are still in the course of development because they lack in sufficient light emitting property. Very high-efficiency green organic light-emitting devices based on electrophosphorescence employing ortho metalized iridium complex (fac-tris(2-phenylpyridine) iridium) as a phosphorus light emitting material for improving properties of the organic EL device are proposed. (refer to, for example, D.F.O'Brien and M.A.Baldo et al "Improved energy transferring electrophosphorescent devices" Applied Physics letters Vol.74 No.3, pp442-444, January 18, 1999; and M.A.Baldo et al "Very high-efficiency green organic light emitting devices based on electrophosphorescence" Applied Physics letters Vol. 75 No.1, pp4-6, July 5, 1999)

Because the current organic EL devices employing the phosphorus photoluminescence are limited to emitting only green light, coverage as the color display devices is narrow. Therefore, it has been demanded to develop organic EL devices which emit light of different colors from green with improved light emission property. Regarding particularly with EL devices which emit blue light, those having an external quantum yield exceeding 5 % is not reported yet. Accordingly, an improvement in the EL devices which emit blue light, if possible, enables the display devices to display full colors or white light resultantly advancing toward practical use of phosphorus light EL device greatly.

### DISCLOSURE OF THE INVENTION

The present invention has been made to overcome the above problems and has an object of providing an organic EL device having excellent efficiency of light emission and prolonged lifetime, and an object of providing a metal-complex compound realizing it.

As a result of intensive researches and studies to achieve the above object by the present inventors, it was found that an employment of a metal-complex compound having a partial structure represented by a following general formula (I) provides the EL device having excellent efficiency of light emission and prolonged lifetime, resultantly completing the present invention.

Namely, the present invention provides a metal-complex compound having a partial structure represented by a following general formula (I): 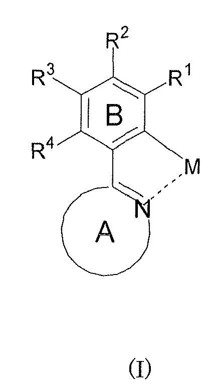 wherein structure B represents a benzene ring structure having R¹ to R⁴; R¹ to R⁴ each independently represents a hydrogen atom, a cyano group, a halogen atom, a substituted or unsubstituted alkyl group having 1 to 20 carbon atoms, a substituted or unsubstituted amino group, a substituted or unsubstituted alkoxyl group having 1 to 20 carbon atoms, a substituted or unsubstituted aromatic group having 1 to 30 carbon atoms; at least one among R¹ to R⁴ is a cyano group; and a couple of R¹ and R², a couple of R² and R³, and a couple of R³ and R⁴ may bond each other to form a ring structure;
structure A represents a ring structure having 3 to 20 carbon atoms, further having at least one carbon-nitrogen double bond and may have a substituent; which may form a ring structure having the foregoing R⁴; and
M represents any one metal atom selected from iridium (Ir) atom, rhodium (Rh) atom, platinum (Pt) atom or palladium (Pd) atom.

Further, the present invention provides an organic EL device which comprises at least one organic thin film layer sandwiched between a pair of electrode consisting of an anode and a cathode, wherein the organic thin film layer comprises the above metal-complex compound, which emits light by applying an electric voltage between the pair of electrode.

### THE PREFERRED EMBODIMENT TO CARRY OUT THE INVENTION

The present invention provides a metal-complex compound having a partial structure represented by a following general formula (I):

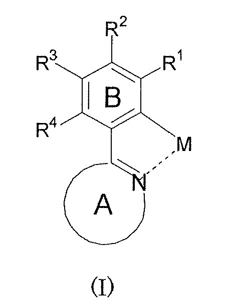

In the general formula (I), the structure B represents a benzene ring structure having R¹ to R⁴; R¹ to R⁴ each independently represents a hydrogen atom, a cyano group, a halogen atom, a substituted or unsubstituted alkyl group having 1 to 20 carbon atoms, a substituted or unsubstituted amino group, a substituted or unsubstituted alkoxyl group having 1 to 20 carbon atoms, a substituted or unsubstituted aromatic group having 1 to 30 carbon atoms; at least one among R¹ to R⁴ is a cyano group; and a couple of R¹ and R², a couple of R² and R³, and a couple of R³ and R⁴ may bond each other to form a ring structure.

Examples of the halogen atom include fluorine atom, chlorine atom, bromine atom and iodine atom, etc.

Examples of the alkyl group described above include methyl group, ethyl group, propyl group, isopropyl group, n-butyl group, s-butyl group, isobutyl group, t-butyl group, n-pentyl group, n-hexyl group, n-heptyl group, n-octyl group, etc. The alkoxy group is expressed as ―OY, wherein Y represents the same as the foregoing description about the alkyl group.

Examples of the above aromatic group include benzene, naphthalene, anthracene, phenanthrene, pyrene, coronene, biphenyl, terphenyl, pyrrole, furan, thiophene, benzothiophene, oxadi azoline, diphenylanthracene, indoline, carbazole, pyridine, benzoquinone, fluoranthene, acenaphtho fluoranthene, etc.

Further, examples of the substituent for those groups include halogen atom, hydroxyl group, substituted or unsubstituted amino group, nitro group, cyano group, substituted or unsubstituted alkyl group, fluorination alkyl group, substituted or unsubstituted alkenyl group, a substituted or unsubstituted cycloalkyl group, substituted or unsubstituted alkoxyl group, substituted or unsubstituted heterocyclic group, substituted or unsubstituted arylalkyl group, a substituted or unsubstituted aryloxy group, substituted or unsubstituted alkoxycarbonyl group, carboxyl group, etc.

It is preferable that R¹ to R⁴ each independently represents a hydrogen atom, a cyano group, a fluorine atom, a trifluoromethyl group, a dimethylamino group and a methoxy group.

Further, it is preferable that the above structure B represents a substituted benzene ring moiety represented by any one of following formulae:

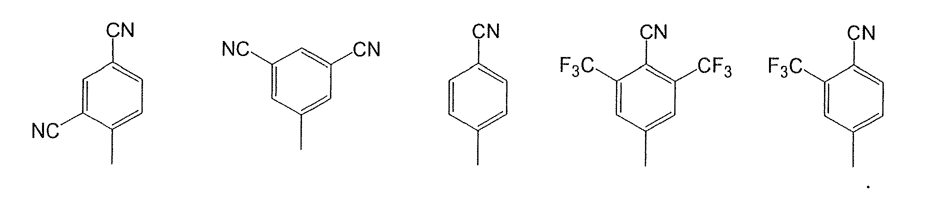

In the general formula (I), structure A represents a ring structure having 3 to 20 carbon atoms, further having at least one carbon-nitrogen double bond and may have a substituent; which may form a ring structure having the foregoing R⁴.

Examples of the ring structure include monovalent groups such as pyridine, chinoline, isoquinoline, pyrimidine, pyrazine, pyridazine, imidazole, oxaline, oxazole, isoxazole, thiazole, isothiazole, etc; and it is preferable that the above structure A represents a group represented by any one of following formulae:

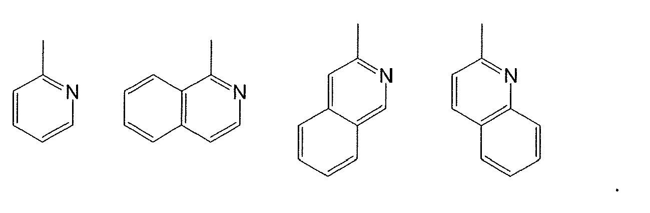

Substituents for the ring structure are the same as described about the substituents for the above structure B.

In the general formula (I), M represents any one metal atom selected from iridium (Ir) atom, rhodium (Rh) atom, platinum (Pt) atom or palladium (Pd) atom, while Ir being preferable.

Further, it is preferable that the partial structure represented by the general formula (I) is expressed by any one of following formulae:

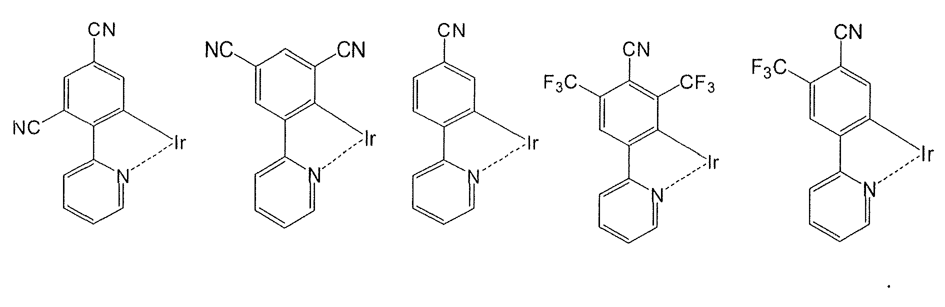

It is preferable that the metal-complex compound of the present invention has any one basic skeletal structure shown among following general formulae 1 to 8:

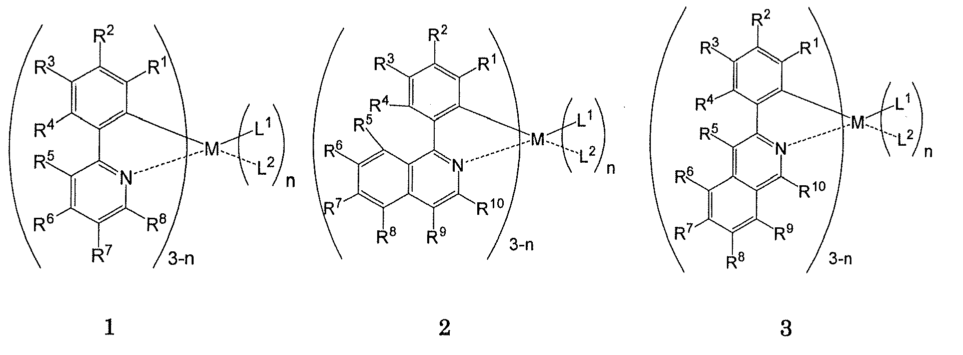

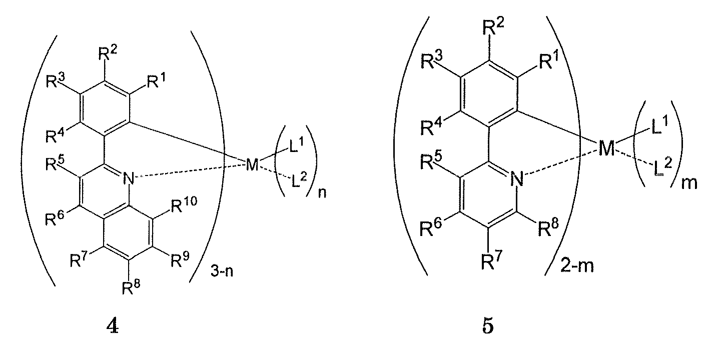

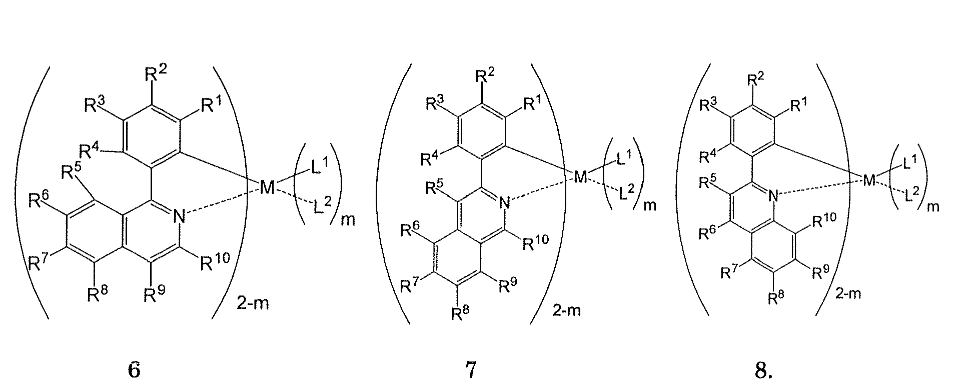

In the general formulae 1 to 8, R¹ to R¹⁰ each independently represents a hydrogen atom, a cyano group, a halogen atom, a substituted or unsubstituted alkyl group having 1 to 20 carbon atoms, a substituted or unsubstituted amino group having 1 to 20 carbon atoms, a substituted or unsubstituted alkoxyl group having 1 to 20 carbon atoms, a substituted or unsubstituted aromatic group having 1 to 30 carbon atoms; at least one among R¹ to R⁴ being a cyano group. Further, a couple of R¹ and R², a couple of R² and R³, a couple of R³ and R⁴, a couple of R⁴ and R⁵, a couple of R⁵ and R⁶, a couple of R⁶ and R⁷, a couple of R⁷ and R⁸, a couple of R⁸ and R⁹, or a couple of R⁹ and R¹⁰ may bond to form a ring structure.

Examples of the above halogen atom, alkyl group, alkoxyl group and aromatic group or those substituent are the same as explained about R¹ to R⁴ in the general formula (I), and preferable examples include similarly.

In the general formulae 1 to 8, M represents the same as the foregoing description.

In the general formulae 1 to 8, L¹ and L² each independently has a structure shown by any one of following structures:

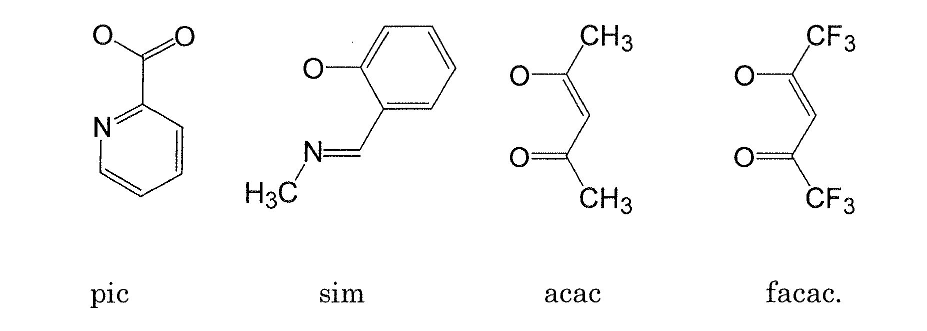

In the general formulae 1 to 8, n represents an integer of 0 to 2, preferably 0 or 1, and m represents an integer of 0 or 1.

Specific examples of the metal-complex compound having any one basic skeletal structure among the general formulae 1 to 8 will be shown in Tables below, though not limited thereto.

In the Tables below, R¹ to R⁸, L¹ and L² (in the cases of the basic skeletal structures 1 and 5) or R¹ to R¹⁰, L¹ and L² (except the cases of the basic skeletal structures 1 and 5) are described within the columns righter to the basic skeletal structures.

In the Tables below, BSS means Basic Skeletal Structure.

**Table 1**

| No. | M | n | BSS | R¹ | R² | R³ | R⁴ | R⁵ | R⁶ | R⁷ | R⁸ | L¹ | L² |
|---|---|---|---|---|---|---|---|---|---|---|---|---|---|
| 1-1 | Ir | 1 | 1 | H | CN | H | H | H | H | H | H | pic | |
| 1-1X | Ir | 1 | 1 | H | CN | H | H | H | H | H | H | acac | |
| 1-1Y | Ir | 0 | 1 | H | CN | H | H | H | H | H | H | - | - |
| 1-2 | Ir | 1 | 1 | H | H | CN | H | H | H | H | H | pic | |
| 1-2X | Ir | 1 | 1 | H | H | CN | H | H | H | H | H | acac | |
| 1-2Y | Ir | 0 | 1 | H | H | CN | H | H | H | H | H | - | - |
| 1-3 | Ir | 1 | 1 | H | H | H | CN | H | H | H | H | pic | |
| 1-3X | Ir | 1 | 1 | H | H | H | CN | H | H | H | H | acac | |
| 1-3Y | Ir | 0 | 1 | H | H | H | CN | H | H | H | H | - | - |
| 1-4 | Ir | 1 | 1 | CN | H | H | H | H | H | H | H | pic | |
| 1-4X | Ir | 1 | 1 | CN | H | H | H | H | H | H | H | acac | |
| 1-4Y | Ir | 0 | 1 | CN | H | H | H | H | H | H | H | - | - |
| 1-5 | lr | 1 | 1 | H | CN | H | CN | H | H | H | H | pic | |
| 1-5X | Ir | 1 | 1 | H | CN | H | CN | H | H | H | H | acac | |
| 1-5Y | Ir | 0 | 1 | H | CN | H | CN | H | H | H | H | - | - |
| 1-6 | Ir | 1 | 1 | CN | H | CN | H | H | H | H | H | pic | |
| 1-6X | Ir | 1 | 1 | CN | H | CN | H | H | H | H | H | acac | |
| 1-6Y | Ir | 0 | 1 | CN | H | CN | H | H | H | H | H | - | - |
| 1-7 | Ir | 1 | 1 | CF₃ | CN | CF₃ | H | H | H | H | H | pic | |
| 1-7X | Ir | 1 | 1 | CF₃ | CN | CF₃ | H | H | H | H | H | acac | |
| 1-7Y | Ir | 0 | 1 | CF₃ | CN | CF₃ | H | H | H | H | H | - | - |
| 1-8 | Ir | 1 | 1 | H | CN | CF₃ | H | H | H | H | H | pic | |
| 1-8X | Ir | 1 | 1 | H | CN | CF₃ | H | H | H | H | H | acac | |
| 1-8Y | Ir | 0 | 1 | H | CN | CF₃ | H | H | H | H | H | - | - |
| 1-9 | Ir | 1 | 1 | H | CN | H | F | H | H | H | H | pic | |
| 1-9X | Ir | 1 | 1 | H | CN | H | F | H | H | H | H | acac | |
| 1-9Y | Ir | 0 | 1 | H | CN | H | F | H | H | H | H | - | - |
| 1-10 | Ir | 1 | 1 | H | F | H | CN | H | H | H | H | pic | |
| 1-10X | Ir | 1 | 1 | H | F | H | CN | H | H | H | H | acac | |
| 1-10Y | Ir | 0 | 1 | H | F | H | CN | H | H | H | H | - | - |
| 1-11 | Ir | 1 | 1 | CN | F | CN | F | H | H | H | H | pic | |
| 1-11X | Ir | 1 | 1 | CN | F | CN | F | H | H | H | H | acac | |
| 1-11Y | Ir | 0 | 1 | CN | F | CN | F | H | H | H | H | - | - |
| 1-12 | Ir | 1 | 1 | CF₃ | CN | CF₃ | CN | H | H | H | H | pic | |
| 1-12X | Ir | 1 | 1 | CF₃ | CN | CF₃ | CN | H | H | H | H | acac | |
| 1-12Y | Ir | 0 | 1 | CF₃ | CN | CF₃ | CN | H | H | H | H | - | - |
| 1-13 | Ir | 1 | 1 | CF₃ | H | CF₃ | CN | H | H | H | H | pic | |
| 1-13X | Ir | 1 | 1 | CF₃ | H | CF₃ | CN | H | H | H | H | acac | |
| 1-13Y | Ir | 0 | 1 | CF₃ | H | CF₃ | CN | H | H | H | H | - | - |
| 1-14 | Ir | 1 | 1 | CF₃ | H | H | CN | H | H | H | H | pic | |
| 1-14X | Ir | 1 | 1 | CF₃ | H | H | CN | H | H | H | H | acac | |
| 1-14Y | Ir | 0 | 1 | CF₃ | H | H | CN | H | H | H | H | - | - |
| 1-15 | Ir | 1 | 1 | H | CF₃ | H | CN | H | H | H | H | pic | |
| 1-15X | Ir | 1 | 1 | H | CF₃ | H | CN | H | H | H | H | acac | |
| 1-15Y | Ir | 0 | 1 | H | CF₃ | H | CN | H | H | H | H | - | - |
| 1-16 | Ir | 1 | 1 | H | F | CN | F | H | H | H | H | pic | |
| 1-16X | Ir | 1 | 1 | H | F | CN | F | H | H | H | H | acac | |
| 1-16Y | Ir | 0 | 1 | H | F | CN | F | H | H | H | H | - | - |
| 1-17 | Ir | 1 | 1 | CF₃ | F | CN | F | H | H | H | H | pic | |
| 1-17X | Ir | 1 | 1 | CF₃ | F | CN | F | H | H | H | H | acac | |
| 1-17Y | Ir | 0 | 1 | CF₃ | F | CN | F | H | H | H | H | - | - |
| 1-18 | Ir | 1 | 1 | CN | CN | CN | CN | H | H | H | H | pic | |
| 1-18X | Ir | 1 | 1 | CN | CN | CN | CN | H | H | H | H | acac | |
| 1-18Y | Ir | 0 | 1 | CN | CN | CN | CN | H | H | H | H | - | - |
| 1-19 | Ir | 1 | 1 | H | H | H | CN | H | N(CH₃)₂ | H | H | | |
| 1-19X | Ir | 1 | 1 | H | H | H | CN | H | N(CH₃)₂ | H | H | acac | |
| 1-19Y | Ir | 0 | 1 | H | H | H | CN | H | N(CH₃)₂ | H | H | - | - |
| 1-20 | Ir | 1 | 1 | H | CN | H | CN | H | N(CH₃)₂ | H | H | pic | |
| 1-20X | Ir | 1 | 1 | H | CN | H | CN | H | N(CH₃)₂ | H | H | acac | |
| 1-20Y | Ir | 0 | 1 | H | CN | H | CN | H | N(CH₃)₂ | H | H | - | - |
| 1-21 | Ir | 1 | 1 | H | CN | CF₃ | H | H | N(CH₃)₂ | H | H | pic | |
| 1-21X | Ir | 1 | 1 | H | CN | CF₃ | H | H | N(CH₃)₂ | H | H | acac | |
| 1-21Y | Ir | 0 | 1 | H | CN | CF₃ | H | H | N(CH₃)₂ | H | H | - | - |
| 1-22 | Ir | 1 | 1 | CF₃ | CN | CF₃ | H | H | N(CH₃)₂ | H | H | pic | |
| 1-22X | Ir | 1 | 1 | CF₃ | CN | CF₃ | H | H | N(CH₃)₂ | H | H | acac | |
| 1-22Y | Ir | 0 | 1 | CF₃ | CN | CF₃ | H | H | N(CH₃)₂ | H | H | - | - |
| 1-23 | Ir | 1 | 1 | H | H | H | CN | H | OCH₃ | H | H | pic | |
| 1-23X | Ir | 1 | 1 | H | H | H | CN | H | OCH₃ | H | H | acac | |
| 1-23Y | Ir | 0 | 1 | H | H | H | CN | H | OCH₃ | H | H | - | - |
| 1-24 | Ir | 1 | 1 | H | CN | H | CN | H | OCH₃ | H | H | pic | |
| 1-24X | Ir | 1 | 1 | H | CN | H | CN | H | OCH₃ | H | H | acac | |
| 1-24Y | Ir | 0 | 1 | H | CN | H | CN | H | OCH₃ | H | H | - | - |
| 1-25 | Ir | 1 | 1 | H | CN | CF₃ | H | H | OCH₃ | H | H | pic | |
| 1-25X | Ir | 1 | 1 | H | CN | CF₃ | H | H | OCH₃ | H | H | acac | |
| 1-25Y | Ir | 0 | 1 | H | CN | CF₃ | H | H | OCH₃ | H | H | - | - |
| 1-26 | Ir | 1 | 1 | CF₃ | CN | CF₃ | H | H | OCH₃ | H | H | pic | |
| 1-26X | Ir | 1 | 1 | CF₃ | CN | CF₃ | H | H | OCH₃ | H | H | acac | |
| 1-26Y | Ir | O | 1 | CF₃ | CN | CF₃ | H | H | OCH₃ | H | H | - | - |

**Table 2**

| No. | M | n | BSS | R¹ | R² | R³ | R⁴ | R⁵ | R⁶ | R⁷ | R⁸ | L¹ | L² |
|---|---|---|---|---|---|---|---|---|---|---|---|---|---|
| 1'-1 | Rh | 1 | 1 | H | CN | H | H | H | H | H | H | pic | |
| 1'-1X | Rh | 1 | 1 | H | CN | H | H | H | H | H | H | acac | |
| 1'-1Y | Rh | 0 | 1 | H | CN | H | H | H | H | H | H | - | - |
| 1'-2 | Rh | 1 | 1 | H | H | CN | H | H | H | H | H | pic | |
| 1'-2X | Rh | 1 | 1 | H | H | CN | H | H | H | H | H | acac | |
| 1'-2Y | Rh | 0 | 1 | H | H | CN | H | H | H | H | H | - | - |
| 1'-3 | Rh | 1 | 1 | H | H | H | CN | H | H | H | H | pic | |
| 1'-3X | Rh | 1 | 1 | H | H | H | CN | H | H | H | H | acac | |
| 1'-3Y | Rh | 0 | 1 | H | H | H | CN | H | H | H | H | - | - |
| 1'-4 | Rh | 1 | 1 | CN | H | H | H | H | H | H | H | pic | |
| 1'-4X | Rh | 1 | 1 | CN | H | H | H | H | H | H | H | acac | |
| 1'-4Y | Rh | 0 | 1 | CN | H | H | H | H | H | H | H | - | - |
| 1'-5 | Rh | 1 | 1 | H | CN | H | CN | H | H | H | H | pic | |
| 1'-5X | Rh | 1 | 1 | H | CN | H | CN | H | H | H | H | acac | |
| 1'-5Y | Rh | 0 | 1 | H | CN | H | CN | H | H | H | H | - | - |
| 1'-6 | Rh | 1 | 1 | CN | H | CN | H | H | H | H | H | pic | |
| 1'-6X | Rh | 1 | 1 | CN | H | CN | H | H | H | H | H | acac | |
| 1'-6Y | Rh | O | 1 | CN | H | CN | H | H | H | H | H | - | - |
| 1'-7 | Rh | 1 | 1 | CF₃ | CN | CF₃ | H | H | H | H | H | pic | |
| 1'-7X | Rh | 1 | 1 | CF₃ | CN | CF₃ | H | H | H | H | H | acac | |
| 1'-7Y | Rh | O | 1 | CF₃ | CN | CF₃ | H | H | H | H | H | - | - |
| 1'-8 | Rh | 1 | 1 | H | CN | CF₃ | H | H | H | H | H | pic | |
| 1' -8X | Rh | 1 | 1 | H | CN | CF₃ | H | H | H | H | H | acac | |
| 1'-8Y | Rh | 0 | 1 | H | CN | CF₃ | H | H | H | H | H | - | - |
| 1'-9 | Rh | 1 | 1 | H | CN | H | F | H | H | H | H | pic | |
| 1'-9X | Rh | 1 | 1 | H | CN | H | F | H | H | H | H | acac | |
| 1'-9Y | Rh | 0 | 1 | H | CN | H | F | H | H | H | H | - | - |
| 1'-10 | Rh | 1 | 1 | H | F | H | CN | H | H | H | H | pic | |
| 1'-10X | Rh | 1 | 1 | H | F | H | CN | H | H | H | H | acac | |
| 1'-10Y | Rh | 0 | 1 | H | F | H | CN | H | H | H | H | - | - |
| 1'-11 | Rh | 1 | 1 | CN | F | CN | F | H | H | H | H | pic | |
| 1'-11X | Rh | 1 | 1 | CN | F | CN | F | H | H | H | H | acac | |
| 1'-11Y | Rh | 0 | 1 | CN | F | CN | F | H | H | H | H | - | - |
| 1'-12 | Rh | 1 | 1 | CF₃ | CN | CF₃ | CN | H | H | H | H | pic | |
| 1'-12X | Rh | 1 | 1 | CF₃ | CN | CF₃ | CN | H | H | H | H | acac | |
| 1'-12Y | Rh | 0 | 1 | CF₃ | CN | CF₃ | CN | H | H | H | H | - | - |
| 1'-13 | Rh | 1 | 1 | CF₃ | H | CF₃ | CN | H | H | H | H | pic | |
| 1'-13X | Rh | 1 | 1 | CF₃ | H | CF₃ | CN | H | H | H | H | acac | |
| 1'-13Y | Rh | 0 | 1 | CF₃ | H | CF₃ | CN | H | H | H | H | - | - |
| 1'-14 | Rh | 1 | 1 | CF₃ | H | H | CN | H | H | H | H | pic | |
| 1'-14X | Rh | 1 | 1 | CF₃ | H | H | CN | H | H | H | H | acac | |
| 1'-14Y | Rh | 0 | 1 | CF₃ | H | H | CN | H | H | H | H | ― | ― |
| 1'-15 | Rh | 1 | 1 | H | CF₃ | H | CN | H | H | H | H | pic | |
| 1'-15X | Rh | 1 | 1 | H | CF₃ | H | CN | H | H | H | H | acac | |
| 1'-15Y | Rh | 0 | 1 | H | CF₃ | H | CN | H | H | H | H | - | - |
| 1'-16 | Rh | 1 | 1 | H | F | CN | F | H | H | H | H | pic | |
| 1'-16X | Rh | 1 | 1 | H | F | CN | F | H | H | H | H | acac | |
| 1'-16Y | Rh | 0 | 1 | H | F | CN | F | H | H | H | H | - | - |
| 1'-17 | Rh | 1 | 1 | CF₃ | F | CN | F | H | H | H | H | pic | |
| 1'-17X | Rh | 1 | 1 | CF₃ | F | CN | F | H | H | H | H | acac | |
| 1'-17Y | Rh | 0 | 1 | CF₃ | F | CN | F | H | H | H | H | - | - |
| 1'-18 | Rh | 1 | 1 | CN | CN | CN | CN | H | H | H | H | pic | |
| 1'-18X | Rh | 1 | 1 | CN | CN | CN | CN | H | H | H | H | acac | |
| 1'-18Y | Rh | 0 | 1 | CN | CN | CN | CN | H | H | H | H | - | - |
| 1'-19 | Rh | 1 | 1 | H | H | H | CN | H | N(CH₃)₂ | H | H | | |
| 1'-19X | Rh | 1 | 1 | H | H | H | CN | H | N(CH₃)₂ | H | H | acac | |
| 1'-19Y | Rh | 0 | 1 | H | H | H | CN | H | N(CH₃)₂ | H | H | - | - |
| 1'-20 | Rh | 1 | 1 | H | CN | H | CN | H | N(CH₃)₂ | H | H | pic | |
| 1'-20X | Rh | 1 | 1 | H | CN | H | CN | H | N(CH₃)₂ | H | H | acac | |
| 1'-20Y | Rh | 0 | 1 | H | CN | H | CN | H | N(CH₃)₂ | H | H | - | - |
| 1'-21 | Rh | 1 | 1 | H | CN | CF₃ | H | H | N(CH₃)₂ | H | H | pic | |
| 1'-21X | Rh | 1 | 1 | H | CN | CF₃ | H | H | N(CH₃)₂ | H | H | acac | |
| 1'-21Y | Rh | 0 | 1 | H | CN | CF₃ | H | H | N(CH₃)₂ | H | H | - | - |
| 1'-22 | Rh | 1 | 1 | CF₃ | CN | CF₃ | H | H | N(CH₃)₂ | H | H | pic | |
| 1'-22X | Rh | 1 | 1 | CF₃ | CN | CF₃ | H | H | N(CH₃)₂ | H | H | acac | |
| 1'-22Y | Rh | 0 | 1 | CF₃ | CN | CF₃ | H | H | N(CH₃)₂ | H | H | - | - |
| 1'-23 | Rh | 1 | 1 | H | H | H | CN | H | OCH₃ | H | H | pic | |
| 1'-23X | Rh | 1 | 1 | H | H | H | CN | H | OCH₃ | H | H | acac | |
| 1'-23Y | Rh | 0 | 1 | H | H | H | CN | H | OCH₃ | H | H | - | - |
| 1'-24 | Rh | 1 | 1 | H | CN | H | CN | H | OCH₃ | H | H | pic | |
| 1'-24X | Rh | 1 | 1 | H | CN | H | CN | H | OCH₃ | H | H | acac | |
| 1'-24Y | Rh | 0 | 1 | H | CN | H | CN | H | OCH₃ | H | H | - | - |
| 1'-25 | Rh | 1 | 1 | H | CN | CF₃ | H | H | OCH₃ | H | H | pic | |
| 1'-25X | Rh | 1 | 1 | H | CN | CF₃ | H | H | OCH₃ | H | H | acac | |
| 1'-25Y | Rh | 0 | 1 | H | CN | CF₃ | H | H | OCH₃ | H | H | - | - |
| 1'-26 | Rh | 1 | 1 | CF₃ | CN | CF₃ | H | H | OCH₃ | H | H | pic | |
| 1'-26X | Rh | 1 | 1 | CF₃ | CN | CF₃ | H | H | OCH₃ | H | H | acac | |
| 1'-26Y | Rh | 0 | 1 | CF₃ | CN | CF₃ | H | H | OCH₃ | H | H | - | - |

**Table 3**

| No. | M | n | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 2-1 | Ir | 1 | 2 | H | CN | H | H | H | H | H | H | H | H | pic | |
| 2-1X | Ir | 1 | 2 | H | CN | H | H | H | H | H | H | H | H | acac | |
| 2-1Y | Ir | 0 | 2 | H | CN | H | H | H | H | H | H | H | H | - | - |
| 2-2 | Ir | 1 | 2 | H | H | CN | H | H | H | H | H | H | H | pic | |
| 2-2X | Ir | 1 | 2 | H | H | CN | H | H | H | H | H | H | H | acac | |
| 2-2Y | Ir | 0 | 2 | H | H | CN | H | H | H | H | H | H | H | - | - |
| 2-3 | Ir | 1 | 2 | H | H | H | CN | H | H | H | H | H | H | pic | |
| 2-3X | Ir | 1 | 2 | H | H | H | CN | H | H | H | H | H | H | acac | |
| 2-3Y | Ir | 0 | 2 | H | H | H | CN | H | H | H | H | H | H | - | - |
| 2-4 | Ir | 1 | 2 | CN | H | H | H | H | H | H | H | H | H | pic | |
| 2-4X | Ir | 1 | 2 | CN | H | H | H | H | H | H | H | H | H | acac | |
| 2-4Y | Ir | 0 | 2 | CN | H | H | H | H | H | H | H | H | H | - | - |
| 2-5 | Ir | 1 | 2 | H | CN | H | CN | H | H | H | H | H | H | pic | |
| 2-5X | Ir | 1 | 2 | H | CN | H | CN | H | H | H | H | H | H | acac | |
| 2-5Y | Ir | 0 | 2 | H | CN | H | CN | H | H | H | H | H | H | - | - |
| 2-6 | Ir | 1 | 2 | CN | H | CN | H | H | H | H | H | H | H | pic | |
| 2-6X | Ir | 1 | 2 | CN | H | CN | H | H | H | H | H | H | H | acac | |
| 2-6Y | Ir | 0 | 2 | CN | H | CN | H | H | H | H | H | H | H | - | - |
| 2-7 | Ir | 1 | 2 | CF₃ | CN | CF₃ | H | H | H | H | H | H | H | pic | |
| 2-7X | Ir | 1 | 2 | CF₃ | CN | CF₃ | H | H | H | H | H | H | H | acac | |
| 2-7Y | Ir | 0 | 2 | CF₃ | CN | CF₃ | H | H | H | H | H | H | H | - | - |
| 2-8 | Ir | 1 | 2 | H | CN | CF₃ | H | H | H | H | H | H | H | pic | |
| 2-8X | Ir | 1 | 2 | H | CN | CF₃ | H | H | H | H | H | H | H | acac | |
| 2-8Y | lr | 0 | 2 | H | CN | CF₃ | H | H | H | H | H | H | H | - | - |
| 2-9 | Ir | 1 | 2 | H | CN | H | F | H | H | H | H | H | H | pic | |
| 2-9X | Ir | 1 | 2 | H | CN | H | F | H | H | H | H | H | H | acac | |
| 2-9Y | Ir | 0 | 2 | H | CN | H | F | H | H | H | H | H | H | - | - |
| 2-10 | Ir | 1 | 2 | H | F | H | CN | H | H | H | H | H | H | pic | |
| 2-10X | Ir | 1 | 2 | H | F | H | CN | H | H | H | H | H | H | acac | |
| 2-10Y | Ir | 0 | 2 | H | F | H | CN | H | H | H | H | H | H | - | - |
| 2-11 | Ir | 1 | 2 | CN | F | CN | F | H | H | H | H | H | H | pic | |
| 2-11X | Ir | 1 | 2 | CN | F | CN | F | H | H | H | H | H | H | acac | |
| 2-11Y | Ir | 0 | 2 | CN | F | CN | F | H | H | H | H | H | H | - | - |
| 2-12 | Ir | 1 | 2 | CF₃ | CN | CF₃ | CN | H | H | H | H | H | H | pic | |
| 2-12X | Ir | 1 | 2 | CF₃ | CN | CF₃ | CN | H | H | H | H | H | H | acac | |
| 2-12Y | Ir | 0 | 2 | CF₃ | CN | CF₃ | CN | H | H | H | H | H | H | - | - |
| 2-13 | Ir | 1 | 2 | CF₃ | H | CF₃ | CN | H | H | H | H | H | H | pic | |
| 2-13X | Ir | 1 | 2 | CF₃ | H | CF₃ | CN | H | H | H | H | H | H | acac | |
| 2-13Y | Ir | 0 | 2 | CF₃ | H | CF₃ | CN | H | H | H | H | H | H | - | - |
| 2-14 | Ir | 1 | 2 | CF₃ | H | H | CN | H | H | H | H | H | H | pic | |
| 2-14X | Ir | 1 | 2 | CF₃ | H | H | CN | H | H | H | H | H | H | acac | |
| 2-14Y | Ir | 0 | 2 | CF₃ | H | H | CN | H | H | H | H | H | H | - | - |
| 2-15 | Ir | 1 | 2 | H | CF₃ | H | CN | H | H | H | H | H | H | pic | |
| 2-15X | Ir | 1 | 2 | H | CF₃ | H | CN | H | H | H | H | H | H | acac | |
| 2-15Y | Ir | 0 | 2 | H | CF₃ | H | CN | H | H | H | H | H | H | - | - |
| 2-16 | Ir | 1 | 2 | H | F | CN | F | H | H | H | H | H | H | pic | |
| 2-16X | Ir | 1 | 2 | H | F | CN | F | H | H | H | H | H | H | acac | |
| 2-16Y | Ir | 0 | 2 | H | F | CN | F | H | H | H | H | H | H | - | - |
| 2-17 | Ir | 1 | 2 | CF₃ | F | CN | F | H | H | H | H | H | H | pic | |
| 2-17X | Ir | 1 | 2 | CF₃ | F | CN | F | H | H | H | H | H | H | acac | |
| 2-17Y | Ir | 0 | 2 | CF₃ | F | CN | F | H | H | H | H | H | H | - | - |
| 2-18 | Ir | 1 | 2 | CN | CN | CN | CN | H | H | H | H | H | H | pic | |
| 2-18X | Ir | 1 | 2 | CN | CN | CN | CN | H | H | H | H | H | H | acac | |
| 2-18Y | Ir | 0 | 2 | CN | CN | CN | CN | H | H | H | H | H | H | - | - |
| 2-19 | Ir | 1 | 2 | CN | H | H | OCH₃ | H | H | H | H | H | H | pic | |
| 2-19X | Ir | 1 | 2 | CN | H | H | OCH₃ | H | H | H | H | H | H | acac | |
| 2-19Y | Ir | 0 | 2 | CN | H | H | OCH₃ | H | H | H | H | H | H | - | - |
| 2-20 | Ir | 1 | 2 | CN | H | H | CH₃ | H | H | H | H | H | H | pic | |
| 2-20X | Ir | 1 | 2 | CN | H | H | CH₃ | H | H | H | H | H | H | acac | |
| 2-20Y | Ir | 0 | 2 | CN | H | H | CH₃ | H | H | H | H | H | H | - | - |
| 2-21 | Ir | 1 | 2 | H | H | CN | OCH₃ | H | H | H | H | H | H | pic | |
| 2-21X | Ir | 1 | 2 | H | H | CN | OCH₃ | H | H | H | H | H | H | acac | |
| 2-21Y | Ir | 0 | 2 | H | H | CN | OCH₃ | H | H | H | H | H | H | - | - |
| 2-22 | Ir | 1 | 2 | H | H | CN | CH₃ | H | H | H | H | H | H | pic | |
| 2-22X | Ir | 1 | 2 | H | H | CN | CH₃ | H | H | H | H | H | H | acac | |
| 2-22Y | Ir | 0 | 2 | H | H | CN | CH₃ | H | H | H | H | H | H | - | - |
| 2-23 | Ir | 1 | 2 | CN | H | H | CN | H | H | H | H | H | H | pic | |
| 2-23X | Ir | 1 | 2 | CN | H | H | CN | H | H | H | H | H | H | acac | |
| 2-23Y | Ir | 0 | 2 | CN | H | H | CN | H | H | H | H | H | H | - | - |
| 2-24 | Ir | 1 | 2 | CN | H | H | F | H | H | H | H | H | H | pic | |
| 2-24X | Ir | 1 | 2 | CN | H | H | F | H | H | H | H | H | H | acac | |
| 2-24Y | Ir | 0 | 2 | CN | H | H | F | H | H | H | H | H | H | - | - |
| 2-25 | Ir | 1 | 2 | CN | F | H | F | H | H | H | H | H | H | pic | |
| 2-25X | Ir | 1 | 2 | CN | F | H | F | H | H | H | H | H | H | acac | |
| 2-25Y | Ir | 0 | 2 | CN | F | H | F | H | H | H | H | H | H | - | - |
| 2-26 | Ir | 1 | 2 | CN | H | CF₃ | CH₃ | H | H | H | H | H | H | pic | |
| 2-26X | Ir | 1 | 2 | CN | H | CF₃ | CH₃ | H | H | H | H | H | H | acac | |
| 2-26Y | Ir | 0 | 2 | CN | H | CF₃ | CH₃ | H | H | H | H | H | H | - | - |
| 2-27 | Ir | 1 | 2 | CN | H | H | CF₃ | H | H | H | H | H | H | pic | |
| 2-27X | Ir | 1 | 2 | CN | H | H | CF₃ | H | H | H | H | H | H | acac | |
| 2-27Y | Ir | 0 | 2 | CN | H | H | CF₃ | H | H | H | H | H | H | - | - |
| 2-28 | Ir | 1 | 2 | CN | F | CN | H | H | H | H | H | H | H | pic | |
| 2-28X | lr | 1 | 2 | CN | F | CN | H | H | H | H | H | H | H | acac | |
| 2-28Y | Ir | 0 | 2 | CN | F | CN | H | H | H | H | H | H | H | - | - |
| 2-29 | Ir | 1 | 2 | CN | CF₃ | CN | H | H | H | H | H | H | H | pic | |
| 2-29X | Ir | 1 | 2 | CN | CF₃ | CN | H | H | H | H | H | H | H | acac | |
| 2-29Y | Ir | 0 | 2 | CN | CF₃ | CN | H | H | H | H | H | H | H | - | - |

**Table 4**

| No. | M | n | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 2'-1 | Rh | 1 | 2 | H | CN | H | H | H | H | H | H | H | H | pic | |
| 2'-1X | Rh | 1 | 2 | H | CN | H | H | H | H | H | H | H | H | acac | |
| 2'-1Y | Rh | 0 | 2 | H | CN | H | H | H | H | H | H | H | H | - | - |
| 2'-2 | Rh | 1 | 2 | H | H | CN | H | H | H | H | H | H | H | pic | |
| 2'-2X | Rh | 1 | 2 | H | H | CN | H | H | H | H | H | H | H | acac | |
| 2'-2Y | Rh | 0 | 2 | H | H | CN | H | H | H | H | H | H | H | - | - |
| 2'-3 | Rh | 1 | 2 | H | H | H | CN | H | H | H | H | H | H | pic | |
| 2'-3X | Rh | 1 | 2 | H | H | H | CN | H | H | H | H | H | H | acac | |
| 2'-3Y | Rh | 0 | 2 | H | H | H | CN | H | H | H | H | H | H | ― | ― |
| 2'-4 | Rh | 1 | 2 | CN | H | H | H | H | H | H | H | H | H | pic | |
| 2'-4X | Rh | 1 | 2 | CN | H | H | H | H | H | H | H | H | H | acac | |
| 2'-4Y | Rh | 0 | 2 | CN | H | H | H | H | H | H | H | H | H | ― | ― |
| 2'-5 | Rh | 1 | 2 | H | CN | H | CN | H | H | H | H | H | H | pic | |
| 2'-5X | Rh | 1 | 2 | H | CN | H | CN | H | H | H | H | H | H | acac | |
| 2'-5Y | Rh | 0 | 2 | H | CN | H | CN | H | H | H | H | H | H | ― | ― |
| 2'-6 | Rh | 1 | 2 | CN | H | CN | H | H | H | H | H | H | H | pic | |
| 2'-6X | Rh | 1 | 2 | CN | H | CN | H | H | H | H | H | H | H | acac | |
| 2'-6Y | Rh | 0 | 2 | CN | H | CN | H | H | H | H | H | H | H | ― | ― |
| 2'-7 | Rh | 1 | 2 | CF₃ | CN | CF₃ | H | H | H | H | H | H | H | pic | |
| 2'-7X | Rh | 1 | 2 | CF₃ | CN | CF₃ | H | H | H | H | H | H | H | acac | |
| 2'-7Y | Rh | 0 | 2 | CF₃ | CN | CF₃ | H | H | H | H | H | H | H | ― | ― |
| 2'-8 | Rh | 1 | 2 | H | CN | CF₃ | H | H | H | H | H | H | H | pic | |
| 2'-8X | Rh | 1 | 2 | H | CN | CF₃ | H | H | H | H | H | H | H | acac | |
| 2'-8Y | Rh | 0 | 2 | H | CN | CF₃ | H | H | H | H | H | H | H | ― | ― |
| 2'-9 | Rh | 1 | 2 | H | CN | H | F | H | H | H | H | H | H | pic | |
| 2'-9X | Rh | 1 | 2 | H | CN | H | F | H | H | H | H | H | H | acac | |
| 2'-9Y | Rh | 0 | 2 | H | CN | H | F | H | H | H | H | H | H | - | - |
| 2'-10 | Rh | 1 | 2 | H | F | H | CN | H | H | H | H | H | H | pic | |
| 2'-10X | Rh | 1 | 2 | H | F | H | CN | H | H | H | H | H | H | acac | |
| 2'-10Y | Rh | 0 | 2 | H | F | H | CN | H | H | H | H | H | H | - | - |
| 2'-11 | Rh | 1 | 2 | CN | F | CN | F | H | H | H | H | H | H | pic | |
| 2'-11 | X Rh | 1 | 2 | CN | F | CN | F | H | H | H | H | H | H | acac | |
| 2'-11Y | Rh | 0 | 2 | CN | F | CN | F | H | H | H | H | H | H | - | - |
| 2'-12 | Rh | 1 | 2 | CF₃ | CN | CF₃ | CN | H | H | H | H | H | H | pic | |
| 2'-12X | Rh | 1 | 2 | CF₃ | CN | CF₃ | CN | H | H | H | H | H | H | acac | |
| 2'-12Y | Rh | 0 | 2 | CF₃ | CN | CF₃ | CN | H | H | H | H | H | H | ― | ― |
| 2'-13 | Rh | 1 | 2 | CF₃ | H | CF₃ | CN | H | H | H | H | H | H | pic | |
| 2'-13X | Rh | 1 | 2 | CF₃ | H | CF₃ | CN | H | H | H | H | H | H | acac | |
| 2'-13Y | Rh | 0 | 2 | CF₃ | H | CF₃ | CN | H | H | H | H | H | H | ― | ― |
| 2'-14 | Rh | 1 | 2 | CF₃ | H | H | CN | H | H | H | H | H | H | pic | |
| 2'-14X | Rh | 1 | 2 | CF₃ | H | H | CN | H | H | H | H | H | H | acac | |
| 2'-14Y | Rh | 0 | 2 | CF₃ | H | H | CN | H | H | H | H | H | H | ― | ― |
| 2'- 15 | Rh | 1 | 2 | H | CF₃ | H | CN | H | H | H | H | H | H | pic | |
| 2'-15X | Rh | 1 | 2 | H | CF₃ | H | CN | H | H | H | H | H | H | acac | |
| 2'-15Y | Rh | 0 | 2 | H | CF₃ | H | CN | H | H | H | H | H | H | - | - |
| 2'-16 | Rh | 1 | 2 | H | F | CN | F | H | H | H | H | H | H | pic | |
| 2'-16X | Rh | 1 | 2 | H | F | CN | F | H | H | H | H | H | H | acac | |
| 2'-16Y | Rh | 0 | 2 | H | F | CN | F | H | H | H | H | H | H | - | - |
| 2'-17 | Rh | 1 | 2 | CF₃ | F | CN | F | H | H | H | H | H | H | pic | |
| 2'-17X | Rh | 1 | 2 | CF₃ | F | CN | F | H | H | H | H | H | H | acac | |
| 2'-17Y | Rh | 0 | 2 | CF₃ | F | CN | F | H | H | H | H | H | H | - | - |
| 2'-18 | Rh | 1 | 2 | CN | CN | CN | CN | H | H | H | H | H | H | pic | |
| 2'-18X | Rh | 1 | 2 | CN | CN | CN | CN | H | H | H | H | H | H | acac | |
| 2'-18Y | Rh | 0 | 2 | CN | CN | CN | CN | H | H | H | H | H | H | - | - |

**Table 5**

| No. | M | n | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 3-1 | Ir | 1 | 3 | H | CN | H | H | H | H | H | H | H | H | pic | |
| 3-1X | Ir | 1 | 3 | H | CN | H | H | H | H | H | H | H | H | acac | |
| 3-1Y | Ir | 0 | 3 | H | CN | H | H | H | H | H | H | H | H | - | - |
| 3-2 | Ir | 1 | 3 | H | H | CN | H | H | H | H | H | H | H | pic | |
| 3-2X | Ir | 1 | 3 | H | H | CN | H | H | H | H | H | H | H | acac | |
| 3-2Y | Ir | 0 | 3 | H | H | CN | H | H | H | H | H | H | H | - | - |
| 3-3 | Ir | 1 | 3 | H | H | H | CN | H | H | H | H | H | H | pic | |
| 3-3X | Ir | 1 | 3 | H | H | H | CN | H | H | H | H | H | H | acac | |
| 3-3Y | Ir | 0 | 3 | H | H | H | CN | H | H | H | H | H | H | ― | ― |
| 3-4 | Ir | 1 | 3 | CN | H | H | H | H | H | H | H | H | H | pic | |
| 3-4X | Ir | 1 | 3 | CN | H | H | H | H | H | H | H | H | H | acac | |
| 3-4Y | Ir | 0 | 3 | CN | H | H | H | H | H | H | H | H | H | ― | ― |
| 3-5 | Ir | 1 | 3 | H | CN | H | CN | H | H | H | H | H | H | pic | |
| 3-5X | Ir | 1 | 3 | H | CN | H | CN | H | H | H | H | H | H | acac | |
| 3-5Y | Ir | 0 | 3 | H | CN | H | CN | H | H | H | H | H | H | ― | ― |
| 3-6 | Ir | 1 | 3 | CN | H | CN | H | H | H | H | H | H | H | pic | |
| 3-6X | Ir | 1 | 3 | CN | H | CN | H | H | H | H | H | H | H | acac | |
| 3-6Y | Ir | 0 | 3 | CN | H | CN | H | H | H | H | H | H | H | - | - |
| 3-7 | Ir | 1 | 3 | CF₃ | CN | CF₃ | H | H | H | H | H | H | H | pic | |
| 3-7X | Ir | 1 | 3 | CF₃ | CN | CF₃ | H | H | H | H | H | H | H | acac | |
| 3-7Y | Ir | 0 | 3 | CF₃ | CN | CF₃ | H | H | H | H | H | H | H | - | - |
| 3-8 | Ir | 1 | 3 | H | CN | CF₃ | H | H | H | H | H | H | H | pic | |
| 3-8X | Ir | 1 | 3 | H | CN | CF₃ | H | H | H | H | H | H | H | acac | |
| 3-8Y | Ir | 0 | 3 | H | CN | CF₃ | H | H | H | H | H | H | H | - | - |
| 3-9 | Ir | 1 | 3 | H | CN | H | F | H | H | H | H | H | H | pic | |
| 3-9X | Ir | 1 | 3 | H | CN | H | F | H | H | H | H | H | H | acac | |
| 3-9Y | Ir | 0 | 3 | H | CN | H | F | H | H | H | H | H | H | ― | ― |
| 3-10 | Ir | 1 | 3 | H | F | H | CN | H | H | H | H | H | H | pic | |
| 3-10X | Ir | 1 | 3 | H | F | H | CN | H | H | H | H | H | H | acac | |
| 3-10Y | Ir | 0 | 3 | H | F | H | CN | H | H | H | H | H | H | ― | ― |
| 3-11 | Ir | 1 | 3 | CN | F | CN | F | H | H | H | H | H | H | pic | |
| 3-11X | Ir | 1 | 3 | CN | F | CN | F | H | H | H | H | H | H | acac | |
| 3-11Y | Ir | 0 | 3 | CN | F | CN | F | H | H | H | H | H | H | ― | ― |
| 3-12 | Ir | 1 | 3 | CF₃ | CN | CF₃ | CN | H | H | H | H | H | H | pic | |
| 3-12X | Ir | 1 | 3 | CF₃ | CN | CF₃ | CN | H | H | H | H | H | H | acac | |
| 3-12Y | Ir | 0 | 3 | CF₃ | CN | CF₃ | CN | H | H | H | H | H | H | ― | ― |
| 3-13 | Ir | 1 | 3 | CF₃ | H | CF₃ | CN | H | H | H | H | H | H | pic | |
| 3-13X | Ir | 1 | 3 | CF₃ | H | CF₃ | CN | H | H | H | H | H | H | acac | |
| 3-13Y | Ir | 0 | 3 | CF₃ | H | CF₃ | CN | H | H | H | H | H | H | ― | ― |
| 3-14 | lr | 1 | 3 | CF₃ | H | H | CN | H | H | H | H | H | H | pic | |
| 3-14X | Ir | 1 | 3 | CF₃ | H | H | CN | H | H | H | H | H | H | acac | |
| 3-14Y | Ir | 0 | 3 | CF₃ | H | H | CN | H | H | H | H | H | H | ― | ― |
| 3-15 | Ir | 1 | 3 | H | CF₃ | H | CN | H | H | H | H | H | H | pic | |
| 3-15X | Ir | 1 | 3 | H | CF₃ | H | CN | H | H | H | H | H | H | acac | |
| 3-15Y | Ir | 0 | 3 | H | CF₃ | H | CN | H | H | H | H | H | H | ― | ― |
| 3-16 | Ir | 1 | 3 | H | F | CN | F | H | H | H | H | H | H | pic | |
| 3-16X | Ir | 1 | 3 | H | F | CN | F | H | H | H | H | H | H | acac | |
| 3-16Y | Ir | 0 | 3 | H | F | CN | F | H | H | H | H | H | H | ― | ― |
| 3-17 | Ir | 1 | 3 | CF₃ | F | CN | F | H | H | H | H | H | H | pic | |
| 3-17X | Ir | 1 | 3 | CF₃ | F | CN | F | H | H | H | H | H | H | acac | |
| 3-17Y | Ir | 0 | 3 | CF₃ | F | CN | F | H | H | H | H | H | H | ― | ― |
| 3-18 | Ir | 1 | 3 | CN | CN | CN | CN | H | H | H | H | H | H | pic | |
| 3-18X | Ir | 1 | 3 | CN | CN | CN | CN | H | H | H | H | H | H | acac | |
| 3-18Y | Ir | 0 | 3 | CN | CN | CN | CN | H | H | H | H | H | H | ― | ― |
| 3-19 | Ir | 1 | 3 | CN | H | H | OCH₃ | H | H | H | H | H | H | pic | |
| 3-19X | Ir | 1 | 3 | CN | H | H | OCH₃ | H | H | H | H | H | H | acac | |
| 3-19Y | Ir | 0 | 3 | CN | H | H | OCH₃ | H | H | H | H | H | H | - | - |
| 3-20 | Ir | 1 | 3 | CN | H | H | CH₃ | H | H | H | H | H | H | pic | |
| 3-20X | Ir | 1 | 3 | CN | H | H | CH₃ | H | H | H | H | H | H | acac | |
| 3-20Y | Ir | 0 | 3 | CN | H | H | CH₃ | H | H | H | H | H | H | ― | ― |
| 3-21 | Ir | 1 | 3 | H | H | CN | OCH₃ | H | H | H | H | H | H | pic | |
| 3-21X | Ir | 1 | 3 | H | H | CN | OCH₃ | H | H | H | H | H | H | acac | |
| 3-21Y | Ir | 0 | 3 | H | H | CN | OCH₃ | H | H | H | H | H | H | - | - |
| 3-22 | Ir | 1 | 3 | H | H | CN | CH₃ | H | H | H | H | H | H | pic | |
| 3-22X | Ir | 1 | 3 | H | H | CN | CH₃ | H | H | H | H | H | H | acac | |
| 3-22Y | Ir | 0 | 3 | H | H | CN | CH₃ | H | H | H | H | H | H | - | - |
| 3-23 | Ir | 1 | 3 | CN | H | H | CN | H | H | H | H | H | H | pic | |
| 3-23X | Ir | 1 | 3 | CN | H | H | CN | H | H | H | H | H | H | acac | |
| 3-23Y | Ir | 0 | 3 | CN | H | H | CN | H | H | H | H | H | H | - | - |
| 3-24 | Ir | 1 | 3 | CN | H | H | F | H | H | H | H | H | H | pic | |
| 3-24X | Ir | 1 | 3 | CN | H | H | F | H | H | H | H | H | H | acac | |
| 3-24Y | Ir | 0 | 3 | CN | H | H | F | H | H | H | H | H | H | ― | ― |
| 3-25 | Ir | 1 | 3 | CN | F | H | F | H | H | H | H | H | H | pic | |
| 3-25X | Ir | 1 | 3 | CN | F | H | F | H | H | H | H | H | H | acac | |
| 3-25Y | Ir | 0 | 3 | CN | F | H | F | H | H | H | H | H | H | ― | ― |
| 3-26 | Ir | 1 | 3 | CN | H | CF₃ | CH₃ | H | H | H | H | H | H | pic | |
| 3-26X | Ir | 1 | 3 | CN | H | CF₃ | CH₃ | H | H | H | H | H | H | acac | |
| 3-26Y | Ir | O | 3 | CN | H | CF₃ | CH₃ | H | H | H | H | H | H | ― | ― |
| 3-27 | Ir | 1 | 3 | CN | H | H | CF₃ | H | H | H | H | H | H | pic | |
| 3-27X | Ir | 1 | 3 | CN | H | H | CF₃ | H | H | H | H | H | H | acac | |
| 3-27Y | Ir | 0 | 3 | CN | H | H | CF₃ | H | H | H | H | H | H | ― | ― |
| 3-28 | lr | 1 | 3 | CN | F | CN | H | H | H | H | H | H | H | pic | |
| 3-28X | Ir | 1 | 3 | CN | F | CN | H | H | H | H | H | H | H | acac | |
| 3-28Y | Ir | 0 | 3 | CN | F | CN | H | H | H | H | H | H | H | - | - |
| 3-29 | Ir | 1 | 3 | CN | CF₃ | CN | H | H | H | H | H | H | H | pic | |
| 3-29X | Ir | 1 | 3 | CN | CF₃ | CN | H | H | H | H | H | H | H | acac | |
| 3-29Y | Ir | 0 | 3 | CN | CF₃ | CN | H | H | H | H | H | H | H | - | - |

**Table 6**

| No. | M | n | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 3'-1 | Rh | 1 | 3 | H | CN | H | H | H | H | H | H | H | H | pic | |
| 3'-1X | Rh | 1 | 3 | H | CN | H | H | H | H | H | H | H | H | acac | |
| 3'-1Y | Rh | 0 | 3 | H | CN | H | H | H | H | H | H | H | H | ― | ― |
| 3'-2 | Rh | 1 | 3 | H | H | CN | H | H | H | H | H | H | H | pic | |
| 3'-2X | Rh | 1 | 3 | H | H | CN | H | H | H | H | H | H | H | acac | |
| 3'-2Y | Rh | 0 | 3 | H | H | CN | H | H | H | H | H | H | H | ― | ― |
| 3'-3 | Rh | 1 | 3 | H | H | H | CN | H | H | H | H | H | H | pic | |
| 3'-3X | Rh | 1 | 3 | H | H | H | CN | H | H | H | H | H | H | acac | |
| 3'-3Y | Rh | 0 | 3 | H | H | H | CN | H | H | H | H | H | H | ― | ― |
| 3'-4 | Rh | 1 | 3 | CN | H | H | H | H | H | H | H | H | H | pic | |
| 3'-4X | Rh | 1 | 3 | CN | H | H | H | H | H | H | H | H | H | acac | |
| 3'-4Y | Rh | 0 | 3 | CN | H | H | H | H | H | H | H | H | H | ― | ― |
| 3'-5 | Rh | 1 | 3 | H | CN | H | CN | H | H | H | H | H | H | pic | |
| 3'-6X | Rh | 1 | 3 | H | CN | H | CN | H | H | H | H | H | H | acac | |
| 3'-5Y | Rh | 0 | 3 | H | CN | H | CN | H | H | H | H | H | H | ― | ― |
| 3'-6 | Rh | 1 | 3 | CN | H | CN | H | H | H | H | H | H | H | pic | |
| 3'-6X | Rh | 1 | 3 | CN | H | CN | H | H | H | H | H | H | H | acac | |
| 3'-6Y | Rh | 0 | 3 | CN | H | CN | H | H | H | H | H | H | H | - | - |
| 3'-7 | Rh | 1 | 3 | CF₃ | CN | CF₃ | H | H | H | H | H | H | H | pic | |
| 3'-7X | Rh | 1 | 3 | CF₃ | CN | CF₃ | H | H | H | H | H | H | H | acac | |
| 3'-7Y | Rh | 0 | 3 | CF₃ | CN | CF₃ | H | H | H | H | H | H | H | - | - |
| 3'-8 | Rh | 1 | 3 | H | CN | CF₃ | H | H | H | H | H | H | H | pic | |
| 3'-8X | Rh | 1 | 3 | H | CN | CF₃ | H | H | H | H | H | H | H | acac | |
| 3'-8Y | Rh | 0 | 3 | H | CN | CF₃ | H | H | H | H | H | H | H | - | - |
| 3'-9 | Rh | 1 | 3 | H | CN | H | F | H | H | H | H | H | H | pic | |
| 3'-9X | Rh | 1 | 3 | H | CN | H | F | H | H | H | H | H | H | acac | |
| 3'-9Y | Rh | 0 | 3 | H | CN | H | F | H | H | H | H | H | H | ― | ― |
| 3'-10 | Rh | 1 | 3 | H | F | H | CN | H | H | H | H | H | H | pic | |
| 3'-10X | Rh | 1 | 3 | H | F | H | CN | H | H | H | H | H | H | acac | |
| 3'-10Y | Rh | 0 | 3 | H | F | H | CN | H | H | H | H | H | H | ― | ― |
| 3'-11 | Rh | 1 | 3 | CN | F | CN | F | H | H | H | H | H | H | pic | |
| 3'-11X | Rh | 1 | 3 | CN | F | CN | F | H | H | H | H | H | H | acac | |
| 3'-11Y | Rh | 0 | 3 | CN | F | CN | F | H | H | H | H | H | H | ― | ― |
| 3'-12 | Rh | 1 | 3 | CF₃ | CN | CF₃ | CN | H | H | H | H | H | H | pic | |
| 3'-12X | Rh | 1 | 3 | CF₃ | CN | CF₃ | CN | H | H | H | H | H | H | acac | |
| 3' -12Y | Rh | 0 | 3 | CF₃ | CN | CF₃ | CN | H | H | H | H | H | H | - | - |
| 3'-13 | Rh | 1 | 3 | CF₃ | H | CF₃ | CN | H | H | H | H | H | H | pic | |
| 3' -13X | Rh | 1 | 3 | CF₃ | H | CF₃ | CN | H | H | H | H | H | H | acac | |
| 3'-13Y | Rh | 0 | 3 | CF₃ | H | CF₃ | CN | H | H | H | H | H | H | - | - |
| 3'-14 | Rh | 1 | 3 | CF₃ | H | H | CN | H | H | H | H | H | H | pic | |
| 3'-14X | Rh | 1 | 3 | CF₃ | H | H | CN | H | H | H | H | H | H | acac | |
| 3'-14Y | Rh | 0 | 3 | CF₃ | H | H | CN | H | H | H | H | H | H | - | - |
| 3'-15 | Rh | 1 | 3 | H | CF₃ | H | CN | H | H | H | H | H | H | pic | |
| 3'-15X | Rh | 1 | 3 | H | CF₃ | H | CN | H | H | H | H | H | H | acac | |
| 3'-15Y | Rh | 0 | 3 | H | CF₃ | H | CN | H | H | H | H | H | H | ― | ― |
| 3'-16 | Rh | 1 | 3 | H | F | CN | F | H | H | H | H | H | H | pic | |
| 3'-16X | Rh | 1 | 3 | H | F | CN | F | H | H | H | H | H | H | acac | |
| 3'-16Y | Rh | 0 | 3 | H | F | CN | F | H | H | H | H | H | H | ― | ― |
| 3'-17 | Rh | 1 | 3 | CF₃ | F | CN | F | H | H | H | H | H | H | pic | |
| 3'-17X | Rh | 1 | 3 | CF₃ | F | CN | F | H | H | H | H | H | H | acac | |
| 3'-17Y | Rh | 0 | 3 | CF₃ | F | CN | F | H | H | H | H | H | H | ― | ― |
| 3'- 18 | Rh | 1 | 3 | CN | CN | CN | CN | H | H | H | H | H | H | pic | |
| 3'-18X | Rh | 1 | 3 | CN | CN | CN | CN | H | H | H | H | H | H | acac | |
| 3'-18Y | Rh | 0 | 3 | CN | CN | CN | CN | H | H | H | H | H | H | ― | ― |

**Table 7**

| No. | M | n | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 4-1 | Ir | 1 | 4 | H | CN | H | H | H | H | H | H | H | H | pic | |
| 4-1X | Ir | 1 | 4 | H | CN | H | H | H | H | H | H | H | H | acac | |
| 4-1Y | Ir | 0 | 4 | H | CN | H | H | H | H | H | H | H | H | - | - |
| 4-2 | Ir | 1 | 4 | H | H | CN | H | H | H | H | H | H | H | pic | |
| 4-2X | Ir | 1 | 4 | H | H | CN | H | H | H | H | H | H | H | acac | |
| 4-2Y | tr | 0 | 4 | H | H | CN | H | H | H | H | H | H | H | - | - |
| 4-3 | Ir | 1 | 4 | H | H | H | CN | H | H | H | H | H | H | pic | |
| 4-3X | Ir | 1 | 4 | H | H | H | CN | H | H | H | H | H | H | acac | |
| 4-3Y | Ir | 0 | 4 | H | H | H | CN | H | H | H | H | H | H | ― | ― |
| 4-4 | Ir | 1 | 4 | CN | H | H | H | H | H | H | H | H | H | pic | |
| 4-4X | Ir | 1 | 4 | CN | H | H | H | H | H | H | H | H | H | acac | |
| 4-4Y | Ir | 0 | 4 | CN | H | H | H | H | H | H | H | H | H | ― | ― |
| 4-5 | Ir | 1 | 4 | H | CN | H | CN | H | H | H | H | H | H | pic | |
| 4-5X | Ir | 1 | 4 | H | CN | H | CN | H | H | H | H | H | H | acac | |
| 4-5Y | Ir | 0 | 4 | H | CN | H | CN | H | H | H | H | H | H | ― | ― |
| 4-6 | Ir | 1 | 4 | CN | H | CN | H | H | H | H | H | H | H | pic | |
| 4-6X | Ir | 1 | 4 | CN | H | CN | H | H | H | H | H | H | H | acac | |
| 4-6Y | Ir | 0 | 4 | CN | H | CN | H | H | H | H | H | H | H | ― | ― |
| 4-7 | Ir | 1 | 4 | CF₃ | CN | CF₃ | H | H | H | H | H | H | H | pic | |
| 4-7X | Ir | 1 | 4 | CF₃ | CN | CF₃ | H | H | H | H | H | H | H | acac | |
| 4-7Y | Ir | 0 | 4 | CF₃ | CN | CF₃ | H | H | H | H | H | H | H | ― | ― |
| 4-8 | Ir | 1 | 4 | H | CN | CF₃ | H | H | H | H | H | H | H | pic | |
| 4-8X | Ir | 1 | 4 | H | CN | CF₃ | H | H | H | H | H | H | H | acac | |
| 4-8Y | Ir | 0 | 4 | H | CN | CF₃ | H | H | H | H | H | H | H | ― | ― |
| 4-9 | Ir | 1 | 4 | H | CN | H | F | H | H | H | H | H | H | pic | |
| 4-9X | Ir | 1 | 4 | H | CN | H | F | H | H | H | H | H | H | acac | |
| 4-9Y | Ir | 0 | 4 | H | CN | H | F | H | H | H | H | H | H | ― | ― |
| 4-10 | Ir | 1 | 4 | H | F | H | CN | H | H | H | H | H | H | pic | |
| 4-10X | Ir | 1 | 4 | H | F | H | CN | H | H | H | H | H | H | acac | |
| 4-10Y | Ir | 0 | 4 | H | F | H | CN | H | H | H | H | H | H | ― | ― |
| 4-11 | Ir | 1 | 4 | CN | F | CN | F | H | H | H | H | H | H | pic | |
| 4-11X | Ir | 1 | 4 | CN | F | CN | F | H | H | H | H | H | H | acac | |
| 4-11Y | Ir | 0 | 4 | CN | F | CN | F | H | H | H | H | H | H | ― | ― |
| 4-12 | Ir | 1 | 4 | CF₃ | CN | CF₃ | CN | H | H | H | H | H | H | pic | |
| 4-12X | Ir | 1 | 4 | CF₃ | CN | CF₃ | CN | H | H | H | H | H | H | acac | |
| 4-12Y | Ir | 0 | 4 | CF₃ | CN | CF₃ | CN | H | H | H | H | H | H | - | - |
| 4-13 | Ir | 1 | 4 | CF₃ | H | CF₃ | CN | H | H | H | H | H | H | pic | |
| 4-13X | Ir | 1 | 4 | CF₃ | H | CF₃ | CN | H | H | H | H | H | H | acac | |
| 4-13Y | Ir | 0 | 4 | CF₃ | H | CF₃ | CN | H | H | H | H | H | H | - | - |
| 4-14 | Ir | 1 | 4 | CF₃ | H | H | CN | H | H | H | H | H | H | pic | |
| 4-14X | Ir | 1 | 4 | CF₃ | H | H | CN | H | H | H | H | H | H | acac | |
| 4-14Y | Ir | 0 | 4 | CF₃ | H | H | CN | H | H | H | H | H | H | - | - |
| 4-15 | Ir | 1 | 4 | H | CF₃ | H | CN | H | H | H | H | H | H | pic | |
| 4-15X | Ir | 1 | 4 | H | CF₃ | H | CN | H | H | H | H | H | H | acac | |
| 4-15Y | Ir | 0 | 4 | H | CF₃ | H | CN | H | H | H | H | H | H | ― | ― |
| 4-16 | Ir | 1 | 4 | H | F | CN | F | H | H | H | H | H | H | pic | |
| 4-16X | Ir | 1 | 4 | H | F | CN | F | H | H | H | H | H | H | acac | |
| 4-16Y | Ir | 0 | 4 | H | F | CN | F | H | H | H | H | H | H | ― | ― |
| 4-17 | Ir | 1 | 4 | CF₃ | F | CN | F | H | H | H | H | H | H | pic | |
| 4-17X | Ir | 1 | 4 | CF₃ | F | CN | F | H | H | H | H | H | H | acac | |
| 4-17Y | Ir | 0 | 4 | CF₃ | F | CN | F | H | H | H | H | H | H | ― | ― |
| 4-18 | Ir | 1 | 4 | CN | CN | CN | CN | H | H | H | H | H | H | pic | |
| 4-18X | Ir | 1 | 4 | CN | CN | CN | CN | H | H | H | H | H | H | acac | |
| 4-18Y | Ir | 0 | 4 | CN | CN | CN | CN | H | H | H | H | H | H | ― | ― |
| 4-19 | Ir | 1 | 4 | CN | H | H | OCH₃ | H | H | H | H | H | H | pic | |
| 4-19X | Ir | 1 | 4 | CN | H | H | OCH₃ | H | H | H | H | H | H | acac | |
| 4-19Y | Ir | 0 | 4 | CN | H | H | OCH₃ | H | H | H | H | H | H | ― | ― |
| 4-20 | Ir | 1 | 4 | CN | H | H | CH₃ | H | H | H | H | H | H | pic | |
| 4-20X | Ir | 1 | 4 | CN | H | H | CH₃ | H | H | H | H | H | H | acac | |
| 4-20Y | Ir | 0 | 4 | CN | H | H | CH₃ | H | H | H | H | H | H | ― | ― |
| 4-21 | Ir | 1 | 4 | H | H | CN | OCH₃ | H | H | H | H | H | H | pic | |
| 4-21X | Ir | 1 | 4 | H | H | CN | OCH₃ | H | H | H | H | H | H | acac | |
| 4-21Y | Ir | 0 | 4 | H | H | CN | OCH₃ | H | H | H | H | H | H | - | - |
| 4-22 | Ir | 1 | 4 | H | H | CN | CH₃ | H | H | H | H | H | H | pic | |
| 4-22X | Ir | 1 | 4 | H | H | CN | CH₃ | H | H | H | H | H | H | acac | |
| 4-22Y | Ir | 0 | 4 | H | H | CN | CH₃ | H | H | H | H | H | H | - | - |
| 4-23 | Ir | 1 | 4 | CN | H | H | CN | H | H | H | H | H | H | pic | |
| 4-23X | Ir | 1 | 4 | CN | H | H | CN | H | H | H | H | H | H | acac | |
| 4-23Y | Ir | 0 | 4 | CN | H | H | CN | H | H | H | H | H | H | - | - |
| 4-24 | Ir | 1 | 4 | CN | H | H | F | H | H | H | H | H | H | pic | |
| 4-24X | Ir | 1 | 4 | CN | H | H | F | H | H | H | H | H | H | acac | |
| 4-24Y | Ir | 0 | 4 | CN | H | H | F | H | H | H | H | H | H | - | - |
| 4-25 | Ir | 1 | 4 | CN | F | H | F | H | H | H | H | H | H | pic | |
| 4-25X | Ir | 1 | 4 | CN | F | H | F | H | H | H | H | H | H | acac | |
| 4-25Y | Ir | 0 | 4 | CN | F | H | F | H | H | H | H | H | H | - | - |
| 4-26 | Ir | 1 | 4 | CN | H | CF₃ | CH₃ | H | H | H | H | H | H | pic | |
| 4-26X | Ir | 1 | 4 | CN | H | CF₃ | CH₃ | H | H | H | H | H | H | acac | |
| 4-26Y | Ir | 0 | 4 | CN | H | CF₃ | CH₃ | H | H | H | H | H | H | - | - |
| 4-27 | Ir | 1 | 4 | CN | H | H | CF₃ | H | H | H | H | H | H | pic | |
| 4-27X | Ir | 1 | 4 | CN | H | H | CF₃ | H | H | H | H | H | H | acac | |
| 4-27Y | Ir | 0 | 4 | CN | H | H | CF₃ | H | H | H | H | H | H | ― | ― |
| 4-28 | Ir | 1 | 4 | CN | F | CN | H | H | H | H | H | H | H | pic | |
| 4-28X | Ir | 1 | 4 | CN | F | CN | H | H | H | H | H | H | H | acac | |
| 4-28Y | Ir | 0 | 4 | CN | F | CN | H | H | H | H | H | H | H | ― | ― |
| 4-29 | Ir | 1 | 4 | CN | CF₃ | CN | H | H | H | H | H | H | H | pic | |
| 4-29X | Ir | 1 | 4 | CN | CF₃ | CN | H | H | H | H | H | H | H | acac | |
| 4-29Y | Ir | 0 | 4 | CN | CF₃ | CN | H | H | H | H | H | H | H | ― | ― |

**Table 8**

| No. | M | n | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 4'-1 | Rh | 1 | 4 | H | CN | H | H | H | H | H | H | H | H | pic | |
| 4'-1X | Rh | 1 | 4 | H | CN | H | H | H | H | H | H | H | H | acac | |
| 4'-1Y | Rh | 0 | 4 | H | CN | H | H | H | H | H | H | H | H | ― | ― |
| 4'-2 | Rh | 1 | 4 | H | H | CN | H | H | H | H | H | H | H | pic | |
| 4'-2X | Rh | 1 | 4 | H | H | CN | H | H | H | H | H | H | H | acac | |
| 4'-2Y | Rh | 0 | 4 | H | H | CN | H | H | H | H | H | H | H | ― | ― |
| 4'-3 | Rh | 1 | 4 | H | H | H | CN | H | H | H | H | H | H | pic | |
| 4'-3X | Rh | 1 | 4 | H | H | H | CN | H | H | H | H | H | H | acac | |
| 4'-3Y | Rh | 0 | 4 | H | H | H | CN | H | H | H | H | H | H | ― | ― |
| 4'-4 | Rh | 1 | 4 | CN | H | H | H | H | H | H | H | H | H | pic | |
| 4'-4X | Rh | 1 | 4 | CN | H | H | H | H | H | H | H | H | H | acac | |
| 4'-4Y | Rh | 0 | 4 | CN | H | H | H | H | H | H | H | H | H | ― | ― |
| 4'-5 | Rh | 1 | 4 | H | CN | H | CN | H | H | H | H | H | H | pic | |
| 4'-5X | Rh | 1 | 4 | H | CN | H | CN | H | H | H | H | H | H | acac | |
| 4'-5Y | Rh | 0 | 4 | H | CN | H | CN | H | H | H | H | H | H | ― | ― |
| 4'-6 | Rh | 1 | 4 | CN | H | CN | H | H | H | H | H | H | H | pic | |
| 4'-6X | Rh | 1 | 4 | CN | H | CN | H | H | H | H | H | H | H | acac | |
| 4'-6Y | Rh | 0 | 4 | CN | H | CN | H | H | H | H | H | H | H | ― | ― |
| 4'-7 | Rh | 1 | 4 | CF₃ | CN | CF₃ | H | H | H | H | H | H | H | pic | |
| 4'-7X | Rh | 1 | 4 | CF₃ | CN | CF₃ | H | H | H | H | H | H | H | acac | |
| 4'-7Y | Rh | 0 | 4 | CF₃ | CN | CF₃ | H | H | H | H | H | H | H | ― | ― |
| 4'-8 | Rh | 1 | 4 | H | CN | CF₃ | H | H | H | H | H | H | H | pic | |
| 4'-8X | Rh | 1 | 4 | H | CN | CF₃ | H | H | H | H | H | H | H | acac | |
| 4'-8Y | Rh | 0 | 4 | H | CN | CF₃ | H | H | H | H | H | H | H | ― | ― |
| 4'-9 | Rh | 1 | 4 | H | CN | H | F | H | H | H | H | H | H | pic | |
| 4'-9X | Rh | 1 | 4 | H | CN | H | F | H | H | H | H | H | H | acac | |
| 4'-9Y | Rh | 0 | 4 | H | CN | H | F | H | H | H | H | H | H | ― | ― |
| 4'-10 | Rh | 1 | 4 | H | F | H | CN | H | H | H | H | H | H | pic | |
| 4'-10X | Rh | 1 | 4 | H | F | H | CN | H | H | H | H | H | H | acac | |
| 4'-10Y | Rh | 0 | 4 | H | F | H | CN | H | H | H | H | H | H | ― | ― |
| 4'-11 | Rh | 1 | 4 | CN | F | CN | F | H | H | H | H | H | H | pic | |
| 4'-11X | Rh | 1 | 4 | CN | F | CN | F | H | H | H | H | H | H | acac | |
| 4'-11Y | Rh | 0 | 4 | CN | F | CN | F | H | H | H | H | H | H | ― | ― |
| 4'-12 | Rh | 1 | 4 | CF₃ | CN | CF₃ | CN | H | H | H | H | H | H | pic | |
| 4'-12X | Rh | 1 | 4 | CF₃ | CN | CF₃ | CN | H | H | H | H | H | H | acac | |
| 4'-12Y | Rh | 0 | 4 | CF₃ | CN | CF₃ | CN | H | H | H | H | H | H | - | - |
| 4'-13 | Rh | 1 | 4 | CF₃ | H | CF₃ | CN | H | H | H | H | H | H | pic | |
| 4'-13X | Rh | 1 | 4 | CF₃ | H | CF₃ | CN | H | H | H | H | H | H | acac | |
| 4'-13Y | Rh | 0 | 4 | CF₃ | H | CF₃ | CN | H | H | H | H | H | H | - | - |
| 4'-14 | Rh | 1 | 4 | CF₃ | H | H | CN | H | H | H | H | H | H | pic | |
| 4'-14X | Rh | 1 | 4 | CF₃ | H | H | CN | H | H | H | H | H | H | acac | |
| 4'-14Y | Rh | 0 | 4 | CF₃ | H | H | CN | H | H | H | H | H | H | - | - |
| 4'-15 | Rh | 1 | 4 | H | CF₃ | H | CN | H | H | H | H | H | H | pic | |
| 4'-15X | Rh | 1 | 4 | H | CF₃ | H | CN | H | H | H | H | H | H | acac | |
| 4'-15Y | Rh | 0 | 4 | H | CF₃ | H | CN | H | H | H | H | H | H | - | - |
| 4'-16 | Rh | 1 | 4 | H | F | CN | F | H | H | H | H | H | H | pic | |
| 4'-16X | Rh | 1 | 4 | H | F | CN | F | H | H | H | H | H | H | acac | |
| 4'-16Y | Rh | 0 | 4 | H | F | CN | F | H | H | H | H | H | H | - | - |
| 4'-17 | Rh | 1 | 4 | CF₃ | F | CN | F | H | H | H | H | H | H | pic | |
| 4'-17X | Rh | 1 | 4 | CF₃ | F | CN | F | H | H | H | H | H | H | acac | |
| 4'-17Y | Rh | 0 | 4 | CF₃ | F | CN | F | H | H | H | H | H | H | - | - |
| 4'-18 | Rh | 1 | 4 | CN | CN | CN | CN | H | H | H | H | H | H | pic | |
| 4'-18X | Rh | 1 | 4 | CN | CN | CN | CN | H | H | H | H | H | H | acac | |
| 4'-18Y | Rh | 0 | 4 | CN | CN | CN | CN | H | H | H | H | H | H | - | - |

**Table 9**

| No. | M | m | BSS | R¹ | R² | R³ | R⁴ | R⁵ | R⁶ | R⁷ | R⁸ | L¹ | L² |
|---|---|---|---|---|---|---|---|---|---|---|---|---|---|
| 5-1 | Pt | 1 | 5 | H | CN | H | H | H | H | H | H | pic | |
| 5-1X | Pt | 1 | 5 | H | CN | H | H | H | H | H | H | acac | |
| 5-1Y | Pt | 0 | 5 | H | CN | H | H | H | H | H | H | ― | ― |
| 5-2 | Pt | 1 | 5 | H | H | CN | H | H | H | H | H | pic | |
| 5-2X | Pt | 1 | 5 | H | H | CN | H | H | H | H | H | acac | |
| 5-2Y | Pt | 0 | 5 | H | H | CN | H | H | H | H | H | ― | ― |
| 5-3 | Pt | 1 | 5 | H | H | H | CN | H | H | H | H | pic | |
| 5-3X | Pt | 1 | 5 | H | H | H | CN | H | H | H | H | acac | |
| 5-3Y | Pt | 0 | 5 | H | H | H | CN | H | H | H | H | ― | ― |
| 5-4 | Pt | 1 | 5 | CN | H | H | H | H | H | H | H | pic | |
| 5-4X | Pt | 1 | 5 | CN | H | H | H | H | H | H | H | acac | |
| 5-4Y | Pt | 0 | 5 | CN | H | H | H | H | H | H | H | ― | ― |
| 5-5 | Pt | 1 | 5 | H | CN | H | CN | H | H | H | H | pic | |
| 5-5X | Pt | 1 | 5 | H | CN | H | CN | H | H | H | H | acac | |
| 5-5Y | Pt | 0 | 5 | H | CN | H | CN | H | H | H | H | ― | ― |
| 5-6 | Pt | 1 | 5 | CN | H | CN | H | H | H | H | H | pic | |
| 5-6X | Pt | 1 | 5 | CN | H | CN | H | H | H | H | H | acac | |
| 5-6Y | Pt | 0 | 5 | CN | H | CN | H | H | H | H | H | - | - |
| 5-7 | Pt | 1 | 5 | CF₃ | CN | CF₃ | H | H | H | H | H | pic | |
| 5-7X | Pt | 1 | 5 | CF₃ | CN | CF₃ | H | H | H | H | H | acac | |
| 5-7Y | Pt | 0 | 5 | CF₃ | CN | CF₃ | H | H | H | H | H | - | - |
| 5-8 | Pt | 1 | 5 | H | CN | CF₃ | H | H | H | H | H | pic | |
| 5-8X | Pt | 1 | 5 | H | CN | CF₃ | H | H | H | H | H | acac | |
| 5-8Y | Pt | 0 | 5 | H | CN | CF₃ | H | H | H | H | H | - | - |
| 5-9 | Pt | 1 | 5 | H | CN | H | F | H | H | H | H | pic | |
| 5-9X | Pt | 1 | 5 | H | CN | H | F | H | H | H | H | acac | |
| 5-9Y | Pt | 0 | 5 | H | CN | H | F | H | H | H | H | - | - |
| 5-10 | Pt | 1 | 5 | H | F | H | CN | H | H | H | H | pic | |
| 5-10X | Pt | 1 | 5 | H | F | H | CN | H | H | H | H | acac | |
| 5-10Y | Pt | 0 | 5 | H | F | H | CN | H | H | H | H | - | - |
| 5-11 | Pt | 1 | 5 | CN | F | CN | F | H | H | H | H | pic | |
| 5-11X | Pt | 1 | 5 | CN | F | CN | F | H | H | H | H | acac | |
| 5-11Y | Pt | 0 | 5 | CN | F | CN | F | H | H | H | H | - | - |
| 5-12 | Pt | 1 | 5 | CF₃ | CN | CF₃ | CN | H | H | H | H | pic | |
| 5-12X | Pt | 1 | 5 | CF₃ | CN | CF₃ | CN | H | H | H | H | acac | |
| 5-12Y | Pt | 0 | 5 | CF₃ | CN | CF₃ | CN | H | H | H | H | - | - |
| 5-13 | Pt | 1 | 5 | CF₃ | H | CF₃ | CN | H | H | H | H | pic | |
| 5-13X | Pt | 1 | 5 | CF₃ | H | CF₃ | CN | H | H | H | H | acac | |
| 5-13Y | Pt | 0 | 5 | CF₃ | H | CF₃ | CN | H | H | H | H | - | - |
| 5-14 | Pt | 1 | 5 | CF₃ | H | H | CN | H | H | H | H | pic | |
| 5-14X | Pt | 1 | 5 | CF₃ | H | H | CN | H | H | H | H | acac | |
| 5-14Y | Pt | 0 | 5 | CF₃ | H | H | CN | H | H | H | H | ― | ― |
| 5-15 | Pt | 1 | 5 | H | CF₃ | H | CN | H | H | H | H | pic | |
| 5-15X | Pt | 1 | 5 | H | CF₃ | H | CN | H | H | H | H | acac | |
| 5-15Y | Pt | 0 | 5 | H | CF₃ | H | CN | H | H | H | H | ― | ― |
| 5-16 | Pt | 1 | 5 | H | F | CN | F | H | H | H | H | pic | |
| 5-16X | Pt | 1 | 5 | H | F | CN | F | H | H | H | H | acac | |
| 5-16Y | Pt | 0 | 5 | H | F | CN | F | H | H | H | H | ― | ― |
| 5-17 | Pt | 1 | 5 | CF₃ | F | CN | F | H | H | H | H | pic | |
| 5-17X | Pt | 1 | 5 | CF₃ | F | CN | F | H | H | H | H | acac | |
| 5-17Y | Pt | 0 | 5 | CF₃ | F | CN | F | H | H | H | H | - | - |
| 5-18 | Pt | 1 | 5 | CN | CN | CN | CN | H | H | H | H | pic | |
| 5-18X | Pt | 1 | 5 | CN | CN | CN | CN | H | H | H | H | acac | |
| 5-18Y | Pt | 0 | 5 | CN | CN | CN | CN | H | H | H | H | - | - |
| 5-19 | Pt | 1 | 5 | H | H | H | CN | H | N(CH₃)₂ | H | H | | |
| 5-19X | Pt | 1 | 5 | H | H | H | CN | H | N(CH₃)₂ | H | H | acac | |
| 5-19Y | Pt | 0 | 5 | H | H | H | CN | H | N(CH₃)₂ | H | H | - | - |
| 5-20 | Pt | 1 | 5 | H | CN | H | CN | H | N(CH₃)₂ | H | H | pic | |
| 5-20X | Pt | 1 | 5 | H | CN | H | CN | H | N(CH₃)₂ | H | H | acac | |
| 5-20Y | Pt | 0 | 5 | H | CN | H | CN | H | N(CH₃)₂ | H | H | ― | ― |
| 5-21 | Pt | 1 | 5 | H | CN | CF₃ | H | H | N(CH₃)₂ | H | H | pic | |
| 5-21X | Pt | 1 | 5 | H | CN | CF₃ | H | H | N(CH₃)₂ | H | H | acac | |
| 5-21Y | Pt | 0 | 5 | H | CN | CF₃ | H | H | N(CH₃)₂ | H | H | ― | ― |
| 5-22 | Pt | 1 | 5 | CF₃ | CN | CF₃ | H | H | N(CH₃)₂ | H | H | pic | |
| 5-22X | Pt | 1 | 5 | CF₃ | CN | CF₃ | H | H | N(CH₃)₂ | H | H | acac | |
| 5-22Y | Pt | 0 | 5 | CF₃ | CN | CF₃ | H | H | N(CH₃)₂ | H | H | ― | ― |
| 5-23 | Pt | 1 | 5 | H | H | H | CN | H | OCH₃ | H | H | pic | |
| 5-23X | Pt | 1 | 5 | H | H | H | CN | H | OCH₃ | H | H | acac | |
| 5-23Y | Pt | 0 | 5 | H | H | H | CN | H | OCH₃ | H | H | - | - |
| 5-24 | Pt | 1 | 5 | H | CN | H | CN | H | OCH₃ | H | H | pic | |
| 5-24X | Pt | 1 | 5 | H | CN | H | CN | H | OCH₃ | H | H | acac | |
| 5-24Y | Pt | 0 | 5 | H | CN | H | CN | H | OCH₃ | H | H | - | - |
| 5-25 | Pt | 1 | 5 | H | CN | CF₃ | H | H | OCH₃ | H | H | pic | |
| 5-25X | Pt | 1 | 5 | H | CN | CF₃ | H | H | OCH₃ | H | H | acac | |
| 5-25Y | Pt | 0 | 5 | H | CN | CF₃ | H | H | OCH₃ | H | H | - | - |
| 5-26 | Pt | 1 | 5 | CF₃ | CN | CF₃ | H | H | OCH₃ | H | H | pic | |
| 5-26X | Pt | 1 | 5 | CF₃ | CN | CF₃ | H | H | OCH₃ | H | H | acac | |
| 5-26Y | Pt | 0 | 5 | CF₃ | CN | CF₃ | H | H | OCH₃ | H | H | - | - |
| 5-27 | Pt | 1 | 5 | CN | H | H | OCH₃ | H | H | H | H | pic | |
| 5-27X | Pt | 1 | 5 | CN | H | H | OCH₃ | H | H | H | H | acac | |
| 5-27Y | Pt | 0 | 5 | CN | H | H | OCH₃ | H | H | H | H | - | - |
| 5-28 | Pt | 1 | 5 | CN | H | H | CH₃ | H | H | H | H | pic | |
| 5-28X | Pt | 1 | 5 | CN | H | H | CH₃ | H | H | H | H | acac | |
| 5-28Y | Pt | 0 | 5 | CN | H | H | CH₃ | H | H | H | H | - | - |
| 5-29 | Pt | 1 | 5 | H | H | CN | OCH₃ | H | H | H | H | pic | |
| 5-29X | Pt | 1 | 5 | H | H | CN | OCH₃ | H | H | H | H | acac | |
| 5-29Y | Pt | 0 | 5 | H | H | CN | OCH₃ | H | H | H | H | ― | ― |
| 5-30 | Pt | 1 | 5 | H | H | CN | CH₃ | H | H | H | H | pic | |
| 5-30X | Pt | 1 | 5 | H | H | CN | CH₃ | H | H | H | H | acac | |
| 5-30Y | Pt | 0 | 5 | H | H | CN | CH₃ | H | H | H | H | ― | ― |
| 5-31 | Pt | 1 | 5 | CN | H | H | CN | H | H | H | H | pic | |
| 6-31X | Pt | 1 | 5 | CN | H | H | CN | H | H | H | H | acac | |
| 5-31Y | Pt | 0 | 5 | CN | H | H | CN | H | H | H | H | - | - |
| 5-32 | Pt | 1 | 5 | CN | H | H | F | H | H | H | H | pic | - |
| 5-32X | Pt | 1 | 5 | CN | H | H | F | H | H | H | H | acac | - |
| 5-32Y | Pt | 0 | 5 | CN | H | H | F | H | H | H | H | - | - |
| 5-33 | Pt | 1 | 5 | CN | F | H | F | H | H | H | H | pic | ― |
| 5-33X | Pt | 1 | 5 | CN | F | H | F | H | H | H | H | acac | ― |
| 5-33Y | Pt | 0 | 5 | CN | F | H | F | H | H | H | H | - | - |
| 5-34 | Pt | 1 | 5 | CN | H | CF₃ | CH₃ | H | H | H | H | pic | ― |
| 5-34X | Pt | 1 | 5 | CN | H | CF₃ | CH₃ | H | H | H | H | acac | ― |
| 5-34Y | Pt | 0 | 5 | CN | H | CF₃ | CH₃ | H | H | H | H | - | - |
| 5-35 | Pt | 1 | 5 | CN | H | H | CF₃ | H | H | H | H | pic | |
| 5-35X | Pt | 1 | 5 | CN | H | H | CF₃ | H | H | H | H | acac | |
| 5-35Y | Pt | 0 | 5 | CN | H | H | CF₃ | H | H | H | H | - | - |
| 5-36 | Pt | 1 | 5 | CN | F | CN | H | H | H | H | H | pic | |
| 5-36X | Pt | 1 | 5 | CN | F | CN | H | H | H | H | H | acac | |
| 5-36Y | Pt | 0 | 5 | CN | F | CN | H | H | H | H | H | - | - |
| 5-37 | Pt | 1 | 5 | CN | CF₃ | CN | H | H | H | H | H | pic | |
| 5-37X | Pt | 1 | 5 | CN | CF₃ | CN | H | H | H | H | H | acac | |
| 5-37Y | IPt | 0 | 5 | CN | CF₃ | CN | H | H | H | H | H | - | - |

**Table 10**

| No. | M | m | BSS | R¹ | R² | R³ | R⁴ | R⁵ | R⁶ | R⁷ | R⁸ | L¹ | L² |
|---|---|---|---|---|---|---|---|---|---|---|---|---|---|
| 5'-1 | Pd | 1 | 5 | H | CN | H | H | H | H | H | H | pic | |
| 5'-1X | Pd | 1 | 5 | H | CN | H | H | H | H | H | H | acac | |
| 5'-1Y | Pd | 0 | 5 | H | CN | H | H | H | H | H | H | - | - |
| 5' -2 | Pd | 1 | 5 | H | H | CN | H | H | H | H | H | pic | |
| 5'-2X | Pd | 1 | 5 | H | H | CN | H | H | H | H | H | acac | |
| 5'-2Y | Pd | 0 | 5 | H | H | CN | H | H | H | H | H | - | - |
| 5'-3 | Pd | 1 | 5 | H | H | H | CN | H | H | H | H | pic | |
| 5'-3X | Pd | 1 | 5 | H | H | H | CN | H | H | H | H | acac | |
| 5'-3Y | Pd | 0 | 5 | H | H | H | CN | H | H | H | H | - | - |
| 5'-4 | Pd | 1 | 5 | CN | H | H | H | H | H | H | H | pic | |
| 5'-4X | Pd | 1 | 5 | CN | H | H | H | H | H | H | H | acac | |
| 5-4Y | Pd | 0 | 5 | CN | H | H | H | H | H | H | H | ― | ― |
| 5'-5 | Pd | 1 | 5 | H | CN | H | CN | H | H | H | H | pic | |
| 5'-5X | Pd | 1 | 5 | H | CN | H | CN | H | H | H | H | acac | |
| 5'-5Y | Pd | 0 | 5 | H | CN | H | CN | H | H | H | H | - | - |
| 5'-6 | Pd | 1 | 5 | CN | H | CN | H | H | H | H | H | pic | |
| 5'-6X | Pd | 1 | 5 | CN | H | CN | H | H | H | H | H | acac | |
| 5'-6Y | Pd | 0 | 5 | CN | H | CN | H | H | H | H | H | - | - |
| 5'-7 | Pd | 1 | 5 | CF₃ | CN | CF₃ | H | H | H | H | H | pic | |
| 5'-7X | Pd | 1 | 5 | CF₃ | CN | CF₃ | H | H | H | H | H | acac | |
| 5'-7Y | Pd | 0 | 5 | CF₃ | CN | CF₃ | H | H | H | H | H | - | - |
| 5'-8 | Pd | 1 | 5 | H | CN | CF₃ | H | H | H | H | H | pic | |
| 5'-8X | Pd | 1 | 5 | H | CN | CF₃ | H | H | H | H | H | acac | |
| 5'-8Y | Pd | 0 | 5 | H | CN | CF₃ | H | H | H | H | H | - | - |
| 5'-9 | Pd | 1 | 5 | H | CN | H | F | H | H | H | H | pic | |
| 5'-9X | Pd | 1 | 5 | H | CN | H | F | H | H | H | H | acac | |
| 5'-9Y | Pd | 0 | 5 | H | CN | H | F | H | H | H | H | - | - |
| 5'-10 | Pd | 1 | 5 | H | F | H | CN | H | H | H | H | pic | |
| 5'-10X | Pd | 1 | 5 | H | F | H | CN | H | H | H | H | acac | |
| 5'-10Y | Pd | 0 | 5 | H | F | H | CN | H | H | H | H | - | - |
| 5'-11 | Pd | 1 | 5 | CN | F | CN | F | H | H | H | H | pic | |
| 5'-11X | Pd | 1 | 5 | CN | F | CN | F | H | H | H | H | acac | |
| 5'-11Y | Pd | 0 | 5 | CN | F | CN | F | H | H | H | H | - | - |
| 5-12 | Pd | 1 | 5 | CF₃ | CN | CF₃ | CN | H | H | H | H | pic | |
| 5'-12X | Pd | 1 | 5 | CF₃ | CN | CF₃ | CN | H | H | H | H | acac | |
| 5'-12Y | Pd | 0 | 5 | CF₃ | CN | CF₃ | CN | H | H | H | H | ― | ― |
| 5'-13 | Pd | 1 | 5 | CF₃ | H | CF₃ | CN | H | H | H | H | pic | |
| 5'-13X | Pd | 1 | 5 | CF₃ | H | CF₃ | CN | H | H | H | H | acac | |
| 5'-13Y | Pd | 0 | 5 | CF₃ | H | CF₃ | CN | H | H | H | H | - | - |
| 5'-14 | Pd | 1 | 5 | CF₃ | H | H | CN | H | H | H | H | pic | |
| 5'-14X | Pd | 1 | 5 | CF₃ | H | H | CN | H | H | H | H | acac | |
| 5'-14Y | Pd | 0 | 5 | CF₃ | H | H | CN | H | H | H | H | - | - |
| 5'-15 | Pd | 1 | 5 | H | CF₃ | H | CN | H | H | H | H | pic | |
| 5'-15X | Pd | 1 | 5 | H | CF₃ | H | CN | H | H | H | H | acac | |
| 5'-15Y | Pd | 0 | 5 | H | CF₃ | H | CN | H | H | H | H | - | - |
| 5'-16 | Pd | 1 | 5 | H | F | CN | F | H | H | H | H | pic | |
| 5'-16X | Pd | 1 | 5 | H | F | CN | F | H | H | H | H | acac | |
| 5'-16Y | Pd | 0 | 5 | H | F | CN | F | H | H | H | H | - | - |
| 5'-17 | Pd | 1 | 5 | CF₃ | F | CN | F | H | H | H | H | pic | |
| 5'-17X | Pd | 1 | 5 | CF₃ | F | CN | F | H | H | H | H | acac | |
| 5'-17Y | Pd | 0 | 5 | CF₃ | F | CN | F | H | H | H | H | - | - |
| 5'-18 | Pd | 1 | 5 | CN | CN | CN | CN | H | H | H | H | pic | |
| 5'-18X | Pd | 1 | 5 | CN | CN | CN | CN | H | H | H | H | acac | |
| 5'-18Y | Pd | 0 | 5 | CN | CN | CN | CN | H | H | H | H | - | - |
| 5'-19 | Pd | 1 | 5 | H | H | H | CN | H | N(CH₃)₂ | H | H | | |
| 5'-19X | Pd | 1 | 5 | H | H | H | CN | H | N(CH₃)₂ | H | H | acac | |
| 5'-19Y | Pd | 0 | 5 | H | H | H | CN | H | N(CH₃)₂ | H | H | - | - |
| 5'-20 | Pd | 1 | 5 | H | CN | H | CN | H | N(CH₃)₂ | H | H | pic | |
| 5'-20X | Pd | 1 | 5 | H | CN | H | CN | H | N(CH₃)₂ | H | H | acac | |
| 5'-20Y | Pd | 0 | 5 | H | CN | H | CN | H | N(CH₃)₂ | H | H | - | - |
| 5'-21 | Pd | 1 | 5 | H | CN | CF₃ | H | H | N(CH₃)₂ | H | H | pic | |
| 5'-21X | Pd | 1 | 5 | H | CN | CF₃ | H | H | N(CH₃)₂ | H | H | acac | |
| 5'-21Y | Pd | 0 | 5 | H | CN | CF₃ | H | H | N(CH₃)₂ | H | H | - | - |
| 5'-22 | Pd | 1 | 5 | CF₃ | CN | CF₃ | H | H | N(CH₃)₂ | H | H | pic | |
| 5'-22X | Pd | 1 | 5 | CF₃ | CN | CF₃ | H | H | N(CH₃)₂ | H | H | acac | |
| 5'-22Y | Pd | 0 | 5 | CF₃ | CN | CF₃ | H | H | N(CH₃)₂ | H | H | - | - |
| 5'-23 | Pd | 1 | 5 | H | H | H | CN | H | OCH₃ | H | H | pic | |
| 5'-23X | Pd | 1 | 5 | H | H | H | CN | H | OCH₃ | H | H | acac | |
| 5'-23Y | Pd | 0 | 5 | H | H | H | CN | H | OCH₃ | H | H | - | - |
| 5'-24 | Pd | 1 | 5 | H | CN | H | CN | H | OCH₃ | H | H | pic | |
| 5'-24X | Pd | 1 | 5 | H | CN | H | CN | H | OCH₃ | H | H | acac | |
| 5'-24Y | Pd | 0 | 5 | H | CN | H | CN | H | OCH₃ | H | H | - | - |
| 5'-25 | Pd | 1 | 5 | H | CN | CF₃ | H | H | OCH₃ | H | H | pic | |
| 5'-25X | Pd | 1 | 5 | H | CN | CF₃ | H | H | OCH₃ | H | H | acac | |
| 5'-25Y | Pd | 0 | 5 | H | CN | CF₃ | H | H | OCH₃ | H | H | - | - |
| 5'-26 | Pd | 1 | 5 | CF₃ | CN | CF₃ | H | H | OCH₃ | H | H | pic | |
| 5'-26X | Pd | 1 | 5 | CF₃ | CN | CF₃ | H | H | OCH₃ | H | H | acac | |
| 5'-26Y | Pd | 0 | 5 | CF₃ | CN | CF₃ | H | H | OCH₃ | H | H | - | - |

**Table 11**

| No. | M | m | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 6-1 | Pt | 1 | 6 | H | CN | H | H | H | H | H | H | H | H | pic | |
| 6-1X | Pt | 1 | 6 | H | CN | H | H | H | H | H | H | H | H | acac | |
| 6-1Y | Pt | 0 | 6 | H | CN | H | H | H | H | H | H | H | H | ― | ― |
| 6-2 | Pt | 1 | 6 | H | H | CN | H | H | H | H | H | H | H | pic | |
| 6-2X | Pt | 1 | 6 | H | H | CN | H | H | H | H | H | H | H | acac | |
| 6-2Y | Pt | 0 | 6 | H | H | CN | H | H | H | H | H | H | H | ― | ― |
| 6-3 | Pt | 1 | 6 | H | H | H | CN | H | H | H | H | H | H | pic | |
| 6-3X | Pt | 1 | 6 | H | H | H | CN | H | H | H | H | H | H | acac | |
| 6-3Y | Pt | 0 | 6 | H | H | H | CN | H | H | H | H | H | H | ― | ― |
| 6-4 | Pt | 1 | 6 | CN | H | H | H | H | H | H | H | H | H | pic | |
| 6-4X | Pt | 1 | 6 | CN | H | H | H | H | H | H | H | H | H | acac | |
| 6-4Y | Pt | 0 | 6 | CN | H | H | H | H | H | H | H | H | H | ― | ― |
| 6-5 | Pt | 1 | 6 | H | CN | H | CN | H | H | H | H | H | H | pic | |
| 6-5X | Pt | 1 | 6 | H | CN | H | CN | H | H | H | H | H | H | acac | |
| 6-5Y | Pt | 0 | 6 | H | CN | H | CN | H | H | H | H | H | H | ― | ― |
| 6-6 | Pt | 1 | 6 | CN | H | CN | H | H | H | H | H | H | H | pic | |
| 6-6X | Pt | 1 | 6 | CN | H | CN | H | H | H | H | H | H | H | acac | |
| 6-6Y | Pt | 0 | 6 | CN | H | CN | H | H | H | H | H | H | H | ― | ― |
| 6-7 | Pt | 1 | 6 | CF₃ | CN | CF₃ | H | H | H | H | H | H | H | pic | |
| 6-7X | Pt | 1 | 6 | CF₃ | CN | CF₃ | H | H | H | H | H | H | H | acac | |
| 6-7Y | Pt | 0 | 6 | CF₃ | CN | CF₃ | H | H | H | H | H | H | H | ― | ― |
| 6-8 | Pt | 1 | 6 | H | CN | CF₃ | H | H | H | H | H | H | H | pic | |
| 6-8X | Pt | 1 | 6 | H | CN | CF₃ | H | H | H | H | H | H | H | acac | |
| 6-8Y | Pt | 0 | 6 | H | CN | CF₃ | H | H | H | H | H | H | H | ― | ― |
| 6-9 | Pt | 1 | 6 | H | CN | H | F | H | H | H | H | H | H | pic | |
| 6-9X | Pt | 1 | 6 | H | CN | H | F | H | H | H | H | H | H | acac | |
| 6-9Y | Pt | 0 | 6 | H | CN | H | F | H | H | H | H | H | H | - | - |
| 6-10 | Pt | 1 | 6 | H | F | H | CN | H | H | H | H | H | H | pic | |
| 6-10X | Pt | 1 | 6 | H | F | H | CN | H | H | H | H | H | H | acac | |
| 6-10Y | Pt | 0 | 6 | H | F | H | CN | H | H | H | H | H | H | - | - |
| 6-11 | Pt | 1 | 6 | CN | F | CN | F | H | H | H | H | H | H | pic | |
| 6-11X | Pt | 1 | 6 | CN | F | CN | F | H | H | H | H | H | H | acac | |
| 6-11 Y | Pt | 0 | 6 | CN | F | CN | F | H | H | H | H | H | H | - | - |
| 6-12 | Pt | 1 | 6 | CF₃ | CN | CF₃ | CN | H | H | H | H | H | H | pic | |
| 6-12X | Pt | 1 | 6 | CF₃ | CN | CF₃ | CN | H | H | H | H | H | H | acac | |
| 6-12Y | Pt | 0 | 6 | CF₃ | CN | CF₃ | CN | H | H | H | H | H | H | - | - |
| 6-13 | Pt | 1 | 6 | CF₃ | H | CF₃ | CN | H | H | H | H | H | H | pic | |
| 6-13X | Pt | 1 | 6 | CF₃ | H | CF₃ | CN | H | H | H | H | H | H | acac | |
| 6-13Y | Pt | 0 | 6 | CF₃ | H | CF₃ | CN | H | H | H | H | H | H | - | - |
| 6-14 | Pt | 1 | 6 | CF₃ | H | H | CN | H | H | H | H | H | H | pic | |
| 6-14X | Pt | 1 | 6 | CF₃ | H | H | CN | H | H | H | H | H | H | acac | |
| 6-14Y | Pt | 0 | 6 | CF₃ | H | H | CN | H | H | H | H | H | H | - | - |
| 6-15 | Pt | 1 | 6 | H | CF₃ | H | CN | H | H | H | H | H | H | pic | |
| 6-15X | Pt | 1 | 6 | H | CF₃ | H | CN | H | H | H | H | H | H | acac | |
| 6-15Y | Pt | 0 | 6 | H | CF₃ | H | CN | H | H | H | H | H | H | ― | ― |
| 6-16 | Pt | 1 | 6 | H | F | CN | F | H | H | H | H | H | H | pic | |
| 6-16X | Pt | 1 | 6 | H | F | CN | F | H | H | H | H | H | H | acac | |
| 6-16Y | Pt | 0 | 6 | H | F | CN | F | H | H | H | H | H | H | ― | ― |
| 6-17 | Pt | 1 | 6 | CF₃ | F | CN | F | H | H | H | H | H | H | pic | |
| 6-17X | Pt | 1 | 6 | CF₃ | F | CN | F | H | H | H | H | H | H | acac | |
| 6-17Y | Pt | 0 | 6 | CF₃ | F | CN | F | H | H | H | H | H | H | ― | ― |
| 6-18 | Pt | 1 | 6 | CN | CN | CN | CN | H | H | H | H | H | H | pic | |
| 6-18X | Pt | 1 | 6 | CN | CN | CN | CN | H | H | H | H | H | H | acac | |
| 6-18Y | Pt | 0 | 6 | CN | CN | CN | CN | H | H | H | H | H | H | ― | ― |
| 6-19 | Pt | 1 | 6 | CN | H | H | OCH₃ | H | H | H | H | H | H | pic | |
| 6-19X | Pt | 1 | 6 | CN | H | H | OCH₃ | H | H | H | H | H | H | acac | |
| 6-19Y | Pt | 0 | 6 | CN | H | H | OCH₃ | H | H | H | H | H | H | ― | ― |
| 6-20 | Pt | 1 | 6 | CN | H | H | CH₃ | H | H | H | H | H | H | pic | |
| 6-20X | Pt | 1 | 6 | CN | H | H | CH₃ | H | H | H | H | H | H | acac | |
| 6-20Y | Pt | 0 | 6 | CN | H | H | CH₃ | H | H | H | H | H | H | ― | ― |
| 6-21 | Pt | 1 | 6 | H | H | CN | OCH₃ | H | H | H | H | H | H | pic | |
| 6-21X | Pt | 1 | 6 | H | H | CN | OCH₃ | H | H | H | H | H | H | acac | |
| 6-21Y | Pt | 0 | 6 | H | H | CN | OCH₃ | H | H | H | H | H | H | - | - |
| 6-22 | Pt | 1 | 6 | H | H | CN | CH₃ | H | H | H | H | H | H | pic | |
| 6-22X | Pt | 1 | 6 | H | H | CN | CH₃ | H | H | H | H | H | H | acac | |
| 6-22Y | Pt | 0 | 6 | H | H | CN | CH₃ | H | H | H | H | H | H | - | - |
| 6-23 | Pt | 1 | 6 | CN | H | H | CN | H | H | H | H | H | H | pic | |
| 6-23X | Pt | 1 | 6 | CN | H | H | CN | H | H | H | H | H | H | acac | |
| 6-23Y | Pt | 0 | 6 | CN | H | H | CN | H | H | H | H | H | H | - | - |
| 6-24 | Pt | 1 | 6 | CN | H | H | F | H | H | H | H | H | H | pic | |
| 6-24X | Pt | 1 | 6 | CN | H | H | F | H | H | H | H | H | H | acac | |
| 6-24Y | Pt | 0 | 6 | CN | H | H | F | H | H | H | H | H | H | ― | ― |
| 6-25 | Pt | 1 | 6 | CN | F | H | F | H | H | H | H | H | H | pic | |
| 6-25X | Pt | 1 | 6 | CN | F | H | F | H | H | H | H | H | H | acac | |
| 6-25Y | Pt | 0 | 6 | CN | F | H | F | H | H | H | H | H | H | ― | ― |
| 6-26 | Pt | 1 | 6 | CN | H | CF₃ | CH₃ | H | H | H | H | H | H | pic | |
| 6-26X | Pt | 1 | 6 | CN | H | CF₃ | CH₃ | H | H | H | H | H | H | acac | |
| 6-26Y | Pt | 0 | 6 | CN | H | CF₃ | CH₃ | H | H | H | H | H | H | ― | ― |
| 6-27 | Pt | 1 | 6 | CN | H | H | CF₃ | H | H | H | H | H | H | pic | |
| 6-27X | Pt | 1 | 6 | CN | H | H | CF₃ | H | H | H | H | H | H | acac | |
| 6-27Y | Pt | 0 | 6 | CN | H | H | CF₃ | H | H | H | H | H | H | - | - |
| 6-28 | Pt | 1 | 6 | CN | F | CN | H | H | H | H | H | H | H | pic | |
| 6-28X | Pt | 1 | 6 | CN | F | CN | H | H | H | H | H | H | H | acac | |
| 6-28Y | Pt | 0 | 6 | CN | F | CN | H | H | H | H | H | H | H | - | - |
| 6-29 | Pt | 1 | 6 | CN | CF₃ | CN | H | H | H | H | H | H | H | pic | |
| 6-29X | Pt | 1 | 6 | CN | CF₃ | CN | H | H | H | H | H | H | H | acac | |
| 6-29Y | Pt | 0 | 6 | CN | CF₃ | CN | H | H | H | H | H | H | H | - | - |

**Table 12**

| No. | M | m | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 6'-1 | Pd | 1 | 6 | H | CN | H | H | H | H | H | H | H | H | pic | |
| 6'-1X | Pd | 1 | 6 | H | CN | H | H | H | H | H | H | H | H | acac | |
| 6'-1Y | Pd | 0 | 6 | H | CN | H | H | H | H | H | H | H | H | ― | ― |
| 6'-2 | Pd | 1 | 6 | H | H | CN | H | H | H | H | H | H | H | pic | |
| 6'-2X | Pd | 1 | 6 | H | H | CN | H | H | H | H | H | H | H | acac | |
| 6'-2Y | Pd | 0 | 6 | H | H | CN | H | H | H | H | H | H | H | ― | ― |
| 6'-3 | Pd | 1 | 6 | H | H | H | CN | H | H | H | H | H | H | pic | |
| 6'-3X | Pd | 1 | 6 | H | H | H | CN | H | H | H | H | H | H | acac | |
| 6'-3Y | Pd | 0 | 6 | H | H | H | CN | H | H | H | H | H | H | ― | ― |
| 6'-4 | Pd | 1 | 6 | CN | H | H | H | H | H | H | H | H | H | pic | |
| 6'-4X | Pd | 1 | 6 | CN | H | H | H | H | H | H | H | H | H | acac | |
| 6'-4Y | Pd | 0 | 6 | CN | H | H | H | H | H | H | H | H | H | ― | ― |
| 6'-5 | Pd | 1 | 6 | H | CN | H | CN | H | H | H | H | H | H | pic | |
| 6'-5X | Pd | 1 | 6 | H | CN | H | CN | H | H | H | H | H | H | acac | |
| 6'-5Y | Pd | 0 | 6 | H | CN | H | CN | H | H | H | H | H | H | ― | ― |
| 6'-6 | Pd | 1 | 6 | CN | H | CN | H | H | H | H | H | H | H | pic | |
| 6'-6X | Pd | 1 | 6 | CN | H | CN | H | H | H | H | H | H | H | acac | |
| 6'-6Y | Pd | 0 | 6 | CN | H | CN | H | H | H | H | H | H | H | ― | ― |
| 6'-7 | Pd | 1 | 6 | CF₃ | CN | CF₃ | H | H | H | H | H | H | H | pic | |
| 6'-7X | Pd | 1 | 6 | CF₃ | CN | CF₃ | H | H | H | H | H | H | H | acac | |
| 6'-7Y | Pd | 0 | 6 | CF₃ | CN | CF₃ | H | H | H | H | H | H | H | ― | ― |
| 6'-8 | Pd | 1 | 6 | H | CN | CF₃ | H | H | H | H | H | H | H | pic | |
| 6'-8X | Pd | 1 | 6 | H | CN | CF₃ | H | H | H | H | H | H | H | acac | |
| 6'-8Y | Pd | 0 | 6 | H | CN | CF₃ | H | H | H | H | H | H | H | ― | ― |
| 6'-9 | Pd | 1 | 6 | H | CN | H | F | H | H | H | H | H | H | pic | |
| 6'-9X | Pd | 1 | 6 | H | CN | H | F | H | H | H | H | H | H | acac | |
| 6'-9Y | Pd | 0 | 6 | H | CN | H | F | H | H | H | H | H | H | - | - |
| 6'-10 | Pd | 1 | 6 | H | F | H | CN | H | H | H | H | H | H | pic | |
| 6'-10X | Pd | 1 | 6 | H | F | H | CN | H | H | H | H | H | H | acac | |
| 6'-10Y | Pd | 0 | 6 | H | F | H | CN | H | H | H | H | H | H | - | - |
| 6'-11 | Pd | 1 | 6 | CN | F | CN | F | H | H | H | H | H | H | pic | |
| 6'-11X | Pd | 1 | 6 | CN | F | CN | F | H | H | H | H | H | H | acac | |
| 6'-11Y | Pd | 0 | 6 | CN | F | CN | F | H | H | H | H | H | H | - | - |
| 6'-12 | Pd | 1 | 6 | CF₃ | CN | CF₃ | CN | H | H | H | H | H | H | pic | |
| 6'-12X | Pd | 1 | 6 | CF₃ | CN | CF₃ | CN | H | H | H | H | H | H | acac | |
| 6'-12Y | Pd | 0 | 6 | CF₃ | CN | CF₃ | CN | H | H | H | H | H | H | ― | ― |
| 6'-13 | Pd | 1 | 6 | CF₃ | H | CF₃ | CN | H | H | H | H | H | H | pic | |
| 6'-13X | Pd | 1 | 6 | CF₃ | H | CF₃ | CN | H | H | H | H | H | H | acac | |
| 6'-13Y | Pd | 0 | 6 | CF₃ | H | CF₃ | CN | H | H | H | H | H | H | ― | ― |
| 6'-14 | Pd | 1 | 6 | CF₃ | H | H | CN | H | H | H | H | H | H | pic | |
| 6'-14X | Pd | 1 | 6 | CF₃ | H | H | CN | H | H | H | H | H | H | acac | |
| 6'-14Y | Pd | 0 | 6 | CF₃ | H | H | CN | H | H | H | H | H | H | ― | ― |
| 6'-15 | Pd | 1 | 6 | H | CF₃ | H | CN | H | H | H | H | H | H | pic | |
| 6'-15X | Pd | 1 | 6 | H | CF₃ | H | CN | H | H | H | H | H | H | acac | |
| 6'-15Y | Pd | 0 | 6 | H | CF₃ | H | CN | H | H | H | H | H | H | - | - |
| 6'-16 | Pd | 1 | 6 | H | F | CN | F | H | H | H | H | H | H | pic | |
| 6'-16X | Pd | 1 | 6 | H | F | CN | F | H | H | H | H | H | H | acac | |
| 6'-16Y | Pd | 0 | 6 | H | F | CN | F | H | H | H | H | H | H | - | - |
| 6'-17 | Pd | 1 | 6 | CF₃ | F | CN | F | H | H | H | H | H | H | pic | |
| 6'-17X | Pd | 1 | 6 | CF₃ | F | CN | F | H | H | H | H | H | H | acac | |
| 6'-17Y | Pd | 0 | 6 | CF₃ | F | CN | F | H | H | H | H | H | H | - | - |
| 6'-18 | Pd | 1 | 6 | CN | CN | CN | CN | H | H | H | H | H | H | pic | |
| 6'-18X | Pd | 1 | 6 | CN | CN | CN | CN | H | H | H | H | H | H | acac | |
| 6'-18Y | Pd | 0 | 6 | CN | CN | CN | CN | H | H | H | H | H | H | - | - |

**Table 13**

| No. | M | m | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 7-1 | Pt | 1 | 7 | H | CN | H | H | H | H | H | H | H | H | pic | |
| 7-1X | Pt | 1 | 7 | H | CN | H | H | H | H | H | H | H | H | acac | |
| 7-1Y | Pt | 0 | 7 | H | CN | H | H | H | H | H | H | H | H | - | - |
| 7-2 | Pt | 1 | 7 | H | H | CN | H | H | H | H | H | H | H | pic | |
| 7-2X | Pt | 1 | 7 | H | H | CN | H | H | H | H | H | H | H | acac | |
| 7-2Y | Pt | 0 | 7 | H | H | CN | H | H | H | H | H | H | H | - | - |
| 7-3 | Pt | 1 | 7 | H | H | H | CN | H | H | H | H | H | H | pic | |
| 7-3X | Pt | 1 | 7 | H | H | H | CN | H | H | H | H | H | H | acac | |
| 7-3Y | Pt | 0 | 7 | H | H | H | CN | H | H | H | H | H | H | - | - |
| 7-4 | Pt | 1 | 7 | CN | H | H | H | H | H | H | H | H | H | pic | |
| 7-4X | Pt | 1 | 7 | CN | H | H | H | H | H | H | H | H | H | acac | |
| 7-4Y | Pt | 0 | 7 | CN | H | H | H | H | H | H | H | H | H | - | - |
| 7-5 | Pt | 1 | 7 | H | CN | H | CN | H | H | H | H | H | H | pic | |
| 7-5X | Pt | 1 | 7 | H | CN | H | CN | H | H | H | H | H | H | acac | |
| 7-5Y | Pt | 0 | 7 | H | CN | H | CN | H | H | H | H | H | H | - | - |
| 7-6 | Pt | 1 | 7 | CN | H | CN | H | H | H | H | H | H | H | pic | |
| 7-6X | Pt | 1 | 7 | CN | H | CN | H | H | H | H | H | H | H | acac | |
| 7-6Y | Pt | 0 | 7 | CN | H | CN | H | H | H | H | H | H | H | - | - |
| 7-7 | Pt | 1 | 7 | CF₃ | CN | CF₃ | H | H | H | H | H | H | H | pic | |
| 7-7X | Pt | 1 | 7 | CF₃ | CN | CF₃ | H | H | H | H | H | H | H | acac | |
| 7-7Y | Pt | 0 | 7 | CF₃ | CN | CF₃ | H | H | H | H | H | H | H | - | - |
| 7-8 | Pt | 1 | 7 | H | CN | CF₃ | H | H | H | H | H | H | H | pic | |
| 7-8X | Pt | 1 | 7 | H | CN | CF₃ | H | H | H | H | H | H | H | acac | |
| 7-8Y | Pt | 0 | 7 | H | CN | CF₃ | H | H | H | H | H | H | H | - | - |
| 7-9 | Pt | 1 | 7 | H | CN | H | F | H | H | H | H | H | H | pic | |
| 7-9X | Pt | 1 | 7 | H | CN | H | F | H | H | H | H | H | H | acac | |
| 7-9Y | Pt | 0 | 7 | H | CN | H | F | H | H | H | H | H | H | - | - |
| 7-10 | Pt | 1 | 7 | H | F | H | CN | H | H | H | H | H | H | pic | |
| 7-10X | Pt | 1 | 7 | H | F | H | CN | H | H | H | H | H | H | acac | |
| 7-10Y | Pt | 0 | 7 | H | F | H | CN | H | H | H | H | H | H | - | - |
| 7-11 | Pt | 1 | 7 | CN | F | CN | F | H | H | H | H | H | H | pic | |
| 7-11X | Pt | 1 | 7 | CN | F | CN | F | H | H | H | H | H | H | acac | |
| 7-11Y | Pt | 0 | 7 | CN | F | CN | F | H | H | H | H | H | H | - | - |
| 7-12 | Pt | 1 | 7 | CF₃ | CN | CF₃ | CN | H | H | H | H | H | H | pic | |
| 7-12X | Pt | 1 | 7 | CF₃ | CN | CF₃ | CN | H | H | H | H | H | H | acac | |
| 7-12Y | Pt | 0 | 7 | CF₃ | CN | CF₃ | CN | H | H | H | H | H | H | ― | ― |
| 7-13 | Pt | 1 | 7 | CF₃ | H | CF₃ | CN | H | H | H | H | H | H | pic | |
| 7-13X | Pt | 1 | 7 | CF₃ | H | CF₃ | CN | H | H | H | H | H | H | acac | |
| 7-13Y | Pt | 0 | 7 | CF₃ | H | CF₃ | CN | H | H | H | H | H | H | ― | ― |
| 7-14 | Pt | 1 | 7 | CF₃ | H | H | CN | H | H | H | H | H | H | pic | |
| 7-14X | Pt | 1 | 7 | CF₃ | H | H | CN | H | H | H | H | H | H | acac | |
| 7-14Y | Pt | 0 | 7 | CF₃ | H | H | CN | H | H | H | H | H | H | ― | ― |
| 7-15 | Pt | 1 | 7 | H | CF₃ | H | CN | H | H | H | H | H | H | pic | |
| 7-15X | Pt | 1 | 7 | H | CF₃ | H | CN | H | H | H | H | H | H | acac | |
| 7-15Y | Pt | 0 | 7 | H | CF₃ | H | CN | H | H | H | H | H | H | ― | ― |
| 7-16 | Pt | 1 | 7 | H | F | CN | F | H | H | H | H | H | H | pic | |
| 7-16X | Pt | 1 | 7 | H | F | CN | F | H | H | H | H | H | H | acac | |
| 7-16Y | Pt | 0 | 7 | H | F | CN | F | H | H | H | H | H | H | ― | ― |
| 7-17 | Pt | 1 | 7 | CF₃ | F | CN | F | H | H | H | H | H | H | pic | |
| 7-17X | Pt | 1 | 7 | CF₃ | F | CN | F | H | H | H | H | H | H | acac | |
| 7-17Y | Pt | 0 | 7 | CF₃ | F | CN | F | H | H | H | H | H | H | ― | ― |
| 7-18 | Pt | 1 | 7 | CN | CN | CN | CN | H | H | H | H | H | H | pic | |
| 7-18X | Pt | 1 | 7 | CN | CN | CN | CN | H | H | H | H | H | H | acac | |
| 7-18Y | Pt | 0 | 7 | CN | CN | CN | CN | H | H | H | H | H | H | ― | ― |
| 7-19 | Pt | 1 | 7 | CN | H | H | OCH₃ | H | H | H | H | H | H | pic | |
| 7-19X | Pt | 1 | 7 | CN | H | H | OCH₃ | H | H | H | H | H | H | acac | |
| 7-19Y | Pt | 0 | 7 | CN | H | H | OCH₃ | H | H | H | H | H | H | ― | ― |
| 7-20 | Pt | 1 | 7 | CN | H | H | CH₃ | H | H | H | H | H | H | pic | |
| 7-20X | Pt | 1 | 7 | CN | H | H | CH₃ | H | H | H | H | H | H | acac | |
| 7-20Y | Pt | 0 | 7 | CN | H | H | CH₃ | H | H | H | H | H | H | ― | ― |
| 7-21 | Pt | 1 | 7 | H | H | CN | OCH₃ | H | H | H | H | H | H | pic | |
| 7-21X | Pt | 1 | 7 | H | H | CN | OCH₃ | H | H | H | H | H | H | acac | |
| 7-21Y | Pt | 0 | 7 | H | H | CN | OCH₃ | H | H | H | H | H | H | - | - |
| 7-22 | Pt | 1 | 7 | H | H | CN | CH₃ | H | H | H | H | H | H | pic | |
| 7-22X | Pt | 1 | 7 | H | H | CN | CH₃ | H | H | H | H | H | H | acac | |
| 7-22Y | Pt | 0 | 7 | H | H | CN | CH₃ | H | H | H | H | H | H | - | - |
| 7-23 | Pt | 1 | 7 | CN | H | H | CN | H | H | H | H | H | H | pic | |
| 7-23X | Pt | 1 | 7 | CN | H | H | CN | H | H | H | H | H | H | acac | |
| 7-23Y | Pt | 0 | 7 | CN | H | H | CN | H | H | H | H | H | H | - | - |
| 7-24 | Pt | 1 | 7 | CN | H | H | F | H | H | H | H | H | H | pic | |
| 7-24X | Pt | 1 | 7 | CN | H | H | F | H | H | H | H | H | H | acac | |
| 7-24Y | Pt | 0 | 7 | CN | H | H | F | H | H | H | H | H | H | - | - |
| 7-25 | Pt | 1 | 7 | CN | F | H | F | H | H | H | H | H | H | pic | |
| 7-25X | Pt | 1 | 7 | CN | F | H | F | H | H | H | H | H | H | acac | |
| 7-25Y | Pt | 0 | 7 | CN | F | H | F | H | H | H | H | H | H | - | - |
| 7-26 | Pt | 1 | 7 | CN | H | CF₃ | CH₃ | H | H | H | H | H | H | pic | |
| 7-26X | Pt | 1 | 7 | CN | H | CF₃ | CH₃ | H | H | H | H | H | H | acac | |
| 7-26Y | Pt | 0 | 7 | CN | H | CF₃ | CH₃ | H | H | H | H | H | H | ― | ― |
| 7-27 | Pt | 1 | 7 | CN | H | H | CF₃ | H | H | H | H | H | H | pic | |
| 7-27X | Pt | 1 | 7 | CN | H | H | CF₃ | H | H | H | H | H | H | acac | |
| 7-27Y | Pt | 0 | 7 | CN | H | H | CF₃ | H | H | H | H | H | H | - | - |
| 7-28 | Pt | 1 | 7 | CN | F | CN | H | H | H | H | H | H | H | pic | |
| 7-28X | Pt | 1 | 7 | CN | F | CN | H | H | H | H | H | H | H | acac | |
| 7-28Y | Pt | 0 | 7 | CN | F | CN | H | H | H | H | H | H | H | - | - |
| 7-29 | Pt | 1 | 7 | CN | CF₃ | CN | H | H | H | H | H | H | H | pic | |
| 7-29X | Pt | 1 | 7 | CN | CF₃ | CN | H | H | H | H | H | H | H | acac | |
| 7-29Y | Pt | 0 | 7 | CN | CF₃ | CN | H | H | H | H | H | H | H | - | - |

**Table 14**

| No. | M | m | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 7'-1 | Pd | 1 | 7 | H | CN | H | H | H | H | H | H | H | H | pic | |
| 7'-1X | Pd | 1 | 7 | H | CN | H | H | H | H | H | H | H | H | acac | |
| 7'-1Y | Pd | 0 | 7 | H | CN | H | H | H | H | H | H | H | H | ― | ― |
| 7'-2 | Pd | 1 | 7 | H | H | CN | H | H | H | H | H | H | H | pic | |
| 7'-2X | Pd | 1 | 7 | H | H | CN | H | H | H | H | H | H | H | acac | |
| 7'-2Y | Pd | 0 | 7 | H | H | CN | H | H | H | H | H | H | H | ― | ― |
| 7'-3 | Pd | 1 | 7 | H | H | H | CN | H | H | H | H | H | H | pic | |
| 7'-3X | Pd | 1 | 7 | H | H | H | CN | H | H | H | H | H | H | acac | |
| 7'-3Y | Pd | 0 | 7 | H | H | H | CN | H | H | H | H | H | H | ― | ― |
| 7'-4 | Pd | 1 | 7 | CN | H | H | H | H | H | H | H | H | H | pic | |
| 7'-4X | Pd | 1 | 7 | CN | H | H | H | H | H | H | H | H | H | acac | |
| 7'-4Y | Pd | 0 | 7 | CN | H | H | H | H | H | H | H | H | H | ― | ― |
| 7'-5 | Pd | 1 | 7 | H | CN | H | CN | H | H | H | H | H | H | pic | |
| 7'-5X | Pd | 1 | 7 | H | CN | H | CN | H | H | H | H | H | H | acac | |
| 7'-5Y | Pd | 0 | 7 | H | CN | H | CN | H | H | H | H | H | H | ― | ― |
| 7'-6 | Pd | 1 | 7 | CN | H | CN | H | H | H | H | H | H | H | pic | |
| 7'-6X | Pd | 1 | 7 | CN | H | CN | H | H | H | H | H | H | H | acac | |
| 7'-6Y | Pd | 0 | 7 | CN | H | CN | H | H | H | H | H | H | H | ― | ― |
| 7'-7 | Pd | 1 | 7 | CF₃ | CN | CF₃ | H | H | H | H | H | H | H | pic | |
| 7'-7X | Pd | 1 | 7 | CF₃ | CN | CF₃ | H | H | H | H | H | H | H | acac | |
| 7'-7Y | Pd | 0 | 7 | CF₃ | CN | CF₃ | H | H | H | H | H | H | H | ― | ― |
| 7'-8 | Pd | 1 | 7 | H | CN | CF₃ | H | H | H | H | H | H | H | pic | |
| 7'-8X | Pd | 1 | 7 | H | CN | CF₃ | H | H | H | H | H | H | H | acac | |
| 7'-8Y | Pd | 0 | 7 | H | CN | CF₃ | H | H | H | H | H | H | H | ― | ― |
| 7'-9 | Pd | 1 | 7 | H | CN | H | F | H | H | H | H | H | H | pic | |
| 7'-9X | Pd | 1 | 7 | H | CN | H | F | H | H | H | H | H | H | acac | |
| 7'-9Y | Pd | 0 | 7 | H | CN | H | F | H | H | H | H | H | H | - | - |
| 7'-10 | Pd | 1 | 7 | H | F | H | CN | H | H | H | H | H | H | pic | |
| 7'-10X | Pd | 1 | 7 | H | F | H | CN | H | H | H | H | H | H | acac | |
| 7'-10Y | Pd | 0 | 7 | H | F | H | CN | H | H | H | H | H | H | - | - |
| 7'-11 | Pd | 1 | 7 | CN | F | CN | F | H | H | H | H | H | H | pic | |
| 7'-11X | Pd | 1 | 7 | CN | F | CN | F | H | H | H | H | H | H | acac | |
| 7'-11Y | Pd | 0 | 7 | CN | F | CN | F | H | H | H | H | H | H | - | - |
| 7'-12 | Pd | 1 | 7 | CF₃ | CN | CF₃ | CN | H | H | H | H | H | H | pic | |
| 7'-12X | Pd | 1 | 7 | CF₃ | CN | CF₃ | CN | H | H | H | H | H | H | acac | |
| 7'-12Y | Pd | 0 | 7 | CF₃ | CN | CF₃ | CN | H | H | H | H | H | H | ― | ― |
| 7'-13 | Pd | 1 | 7 | CF₃ | H | CF₃ | CN | H | H | H | H | H | H | pic | |
| 7'-13X | Pd | 1 | 7 | CF₃ | H | CF₃ | CN | H | H | H | H | H | H | acac | |
| 7'-13Y | Pd | 0 | 7 | CF₃ | H | CF₃ | CN | H | H | H | H | H | H | ― | ― |
| 7'-14 | Pd | 1 | 7 | CF₃ | H | H | CN | H | H | H | H | H | H | pic | |
| 7'-14X | Pd | 1 | 7 | CF₃ | H | H | CN | H | H | H | H | H | H | acac | |
| 7'-14Y | Pd | 0 | 7 | CF₃ | H | H | CN | H | H | H | H | H | H | ― | ― |
| 7'-15 | Pd | 1 | 7 | H | CF₃ | H | CN | H | H | H | H | H | H | pic | |
| 7'-15X | Pd | 1 | 7 | H | CF₃ | H | CN | H | H | H | H | H | H | acac | |
| 7'-15Y | Pd | 0 | 7 | H | CF₃ | H | CN | H | H | H | H | H | H | - | - |
| 7'-16 | Pd | 1 | 7 | H | F | CN | F | H | H | H | H | H | H | pic | |
| 7'-16X | Pd | 1 | 7 | H | F | CN | F | H | H | H | H | H | H | acac | |
| 7'-16Y | Pd | 0 | 7 | H | F | CN | F | H | H | H | H | H | H | - | - |
| 7'-17 | Pd | 1 | 7 | CF₃ | F | CN | F | H | H | H | H | H | H | pic | |
| 7'-17X | Pd | 1 | 7 | CF₃ | F | CN | F | H | H | H | H | H | H | acac | |
| 7'-17Y | Pd | 0 | 7 | CF₃ | F | CN | F | H | H | H | H | H | H | - | - |
| 7'-18 | Pd | 1 | 7 | CN | CN | CN | CN | H | H | H | H | H | H | pic | |
| 7'-18X | Pd | 1 | 7 | CN | CN | CN | CN | H | H | H | H | H | H | acac | |
| 7'-18Y | Pd | 0 | 7 | CN | CN | CN | CN | H | H | H | H | H | H | - | - |

**Table 15**

| No. | M | m | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 8-1 | Pt | 1 | 8 | H | CN | H | H | H | H | H | H | H | H | pic | |
| 8-1X | Pt | 1 | 8 | H | CN | H | H | H | H | H | H | H | H | acac | |
| 8-1Y | Pt | 0 | 8 | H | CN | H | H | H | H | H | H | H | H | ― | ― |
| 8-2 | Pt | 1 | 8 | H | H | CN | H | H | H | H | H | H | H | pic | |
| 8-2X | Pt | 1 | 8 | H | H | CN | H | H | H | H | H | H | H | acac | |
| 8-2Y | Pt | 0 | 8 | H | H | CN | H | H | H | H | H | H | H | ― | ― |
| 8-3 | Pt | 1 | 8 | H | H | H | CN | H | H | H | H | H | H | pic | |
| 8-3X | Pt | 1 | 8 | H | H | H | CN | H | H | H | H | H | H | acac | |
| 8-3Y | Pt | 0 | 8 | H | H | H | CN | H | H | H | H | H | H | ― | ― |
| 8-4 | Pt | 1 | 8 | CN | H | H | H | H | H | H | H | H | H | pic | |
| 8-4X | Pt | 1 | 8 | CN | H | H | H | H | H | H | H | H | H | acac | |
| 8-4Y | Pt | 0 | 8 | CN | H | H | H | H | H | H | H | H | H | ― | ― |
| 8-5 | Pt | 1 | 8 | H | CN | H | CN | H | H | H | H | H | H | pic | |
| 8-5X | Pt | 1 | 8 | H | CN | H | CN | H | H | H | H | H | H | acac | |
| 8-5Y | Pt | 0 | 8 | H | CN | H | CN | H | H | H | H | H | H | ― | ― |
| 8-6 | Pt | 1 | 8 | CN | H | CN | H | H | H | H | H | H | H | pic | |
| 8-6X | Pt | 1 | 8 | CN | H | CN | H | H | H | H | H | H | H | acac | |
| 8-6Y | Pt | 0 | 8 | CN | H | CN | H | H | H | H | H | H | H | - | - |
| 8-7 | Pt | 1 | 8 | CF₃ | CN | CF₃ | H | H | H | H | H | H | H | pic | |
| 8-7X | Pt | 1 | 8 | CF₃ | CN | CF₃ | H | H | H | H | H | H | H | acac | |
| 8-7Y | Pt | 0 | 8 | CF₃ | CN | CF₃ | H | H | H | H | H | H | H | - | - |
| 8-8 | Pt | 1 | 8 | H | CN | CF₃ | H | H | H | H | H | H | H | pic | |
| 8-8X | Pt | 1 | 8 | H | CN | CF₃ | H | H | H | H | H | H | H | acac | |
| 8-8Y | Pt | 0 | 8 | H | CN | CF₃ | H | H | H | H | H | H | H | - | - |
| 8-9 | Pt | 1 | 8 | H | CN | H | F | H | H | H | H | H | H | pic | |
| 8-9X | Pt | 1 | 8 | H | CN | H | F | H | H | H | H | H | H | acac | |
| 8-9Y | Pt | 0 | 8 | H | CN | H | F | H | H | H | H | H | H | - | - |
| 8-10 | Pt | 1 | 8 | H | F | H | CN | H | H | H | H | H | H | pic | |
| 8-10X | Pt | 1 | 8 | H | F | H | CN | H | H | H | H | H | H | acac | |
| 8-10Y | Pt | 0 | 8 | H | F | H | CN | H | H | H | H | H | H | - | - |
| 8-11 | Pt | 1 | 8 | CN | F | CN | F | H | H | H | H | H | H | pic | |
| 8-11X | Pt | 1 | 8 | CN | F | CN | F | H | H | H | H | H | H | acac | |
| 8-11Y | Pt | 0 | 8 | CN | F | CN | F | H | H | H | H | H | H | - | - |
| 8-12 | Pt | 1 | 8 | CF₃ | CN | CF₃ | CN | H | H | H | H | H | H | pic | |
| 8-12X | Pt | 1 | 8 | CF₃ | CN | CF₃ | CN | H | H | H | H | H | H | acac | |
| 8-12Y | Pt | 0 | 8 | CF₃ | CN | CF₃ | CN | H | H | H | H | H | H | - | - |
| 8-13 | Pt | 1 | 8 | CF₃ | H | CF₃ | CN | H | H | H | H | H | H | pic | |
| 8-13X | Pt | 1 | 8 | CF₃ | H | CF₃ | CN | H | H | H | H | H | H | acac | |
| 8-13Y | Pt | 0 | 8 | CF₃ | H | CF₃ | CN | H | H | H | H | H | H | - | - |
| 8-14 | Pt | 1 | 8 | CF₃ | H | H | CN | H | H | H | H | H | H | pic | |
| 8-14X | Pt | 1 | 8 | CF₃ | H | H | CN | H | H | H | H | H | H | acac | |
| 8-14Y | Pt | 0 | 8 | CF₃ | H | H | CN | H | H | H | H | H | H | - | - |
| 8-15 | Pt | 1 | 8 | H | CF₃ | H | CN | H | H | H | H | H | H | pic | |
| 8-15X | Pt | 1 | 8 | H | CF₃ | H | CN | H | H | H | H | H | H | acac | |
| 8-15Y | Pt | 0 | 8 | H | CF₃ | H | CN | H | H | H | H | H | H | ― | ― |
| 8-16 | Pt | 1 | 8 | H | F | CN | F | H | H | H | H | H | H | pic | |
| 8-16X | Pt | 1 | 8 | H | F | CN | F | H | H | H | H | H | H | acac | |
| 8-16Y | Pt | 0 | 8 | H | F | CN | F | H | H | H | H | H | H | ― | ― |
| 8-17 | Pt | 1 | 8 | CF₃ | F | CN | F | H | H | H | H | H | H | pic | |
| 8-17X | Pt | 1 | 8 | CF₃ | F | CN | F | H | H | H | H | H | H | acac | |
| 8-17Y | Pt | 0 | 8 | CF₃ | F | CN | F | H | H | H | H | H | H | ― | ― |
| 8-18 | Pt | 1 | 8 | CN | CN | CN | CN | H | H | H | H | H | H | pic | |
| 8-18X | Pt | 1 | 8 | CN | CN | CN | CN | H | H | H | H | H | H | acac | |
| 8-18Y | Pt | 0 | 8 | CN | CN | CN | CN | H | H | H | H | H | H | ― | ― |
| 8-19 | Pt | 1 | 8 | CN | H | H | OCH₃ | H | H | H | H | H | H | pic | |
| 8-19X | Pt | 1 | 8 | CN | H | H | OCH₃ | H | H | H | H | H | H | acac | |
| 8-19Y | Pt | 0 | 8 | CN | H | H | OCH₃ | H | H | H | H | H | H | - | - |
| 8-20 | Pt | 1 | 8 | CN | H | H | CH₃ | H | H | H | H | H | H | pic | |
| 8-20X | Pt | 1 | 8 | CN | H | H | CH₃ | H | H | H | H | H | H | acac | |
| 8-20Y | Pt | 0 | 8 | CN | H | H | CH₃ | H | H | H | H | H | H | - | - |
| 8-21 | Pt | 1 | 8 | H | H | CN | OCH₃ | H | H | H | H | H | H | pic | |
| 8-21X | Pt | 1 | 8 | H | H | CN | OCH₃ | H | H | H | H | H | H | acac | |
| 8-21Y | Pt | 0 | 8 | H | H | CN | OCH₃ | H | H | H | H | H | H | ― | ― |
| 8-22 | Pt | 1 | 8 | H | H | CN | CH₃ | H | H | H | H | H | H | pic | |
| 8-22X | Pt | 1 | 8 | H | H | CN | CH₃ | H | H | H | H | H | H | acac | |
| 8-22Y | Pt | 0 | 8 | H | H | CN | CH₃ | H | H | H | H | H | H | ― | ― |
| 8-23 | Pt | 1 | 8 | CN | H | H | CN | H | H | H | H | H | H | pic | |
| 8-23X | Pt | 1 | 8 | CN | H | H | CN | H | H | H | H | H | H | acac | |
| 8-23Y | Pt | 0 | 8 | CN | H | H | CN | H | H | H | H | H | H | - | - |
| 8-24 | Pt | 1 | 8 | CN | H | H | F | H | H | H | H | H | H | pic | |
| 8-24X | Pt | 1 | 8 | CN | H | H | F | H | H | H | H | H | H | acac | |
| 8-24Y | Pt | O | 8 | CN | H | H | F | H | H | H | H | H | H | - | - |
| 8-25 | Pt | 1 | 8 | CN | F | H | F | H | H | H | H | H | H | pic | |
| 8-25X | Pt | 1 | 8 | CN | F | H | F | H | H | H | H | H | H | acac | |
| 8-25Y | Pt | 0 | 8 | CN | F | H | F | H | H | H | H | H | H | - | - |
| 8-26 | Pt | 1 | 8 | CN | H | CF₃ | CH₃ | H | H | H | H | H | H | pic | |
| 8-26X | Pt | 1 | 8 | CN | H | CF₃ | CH₃ | H | H | H | H | H | H | acac | |
| 8-26Y | Pt | 0 | 8 | CN | H | CF₃ | CH₃ | H | H | H | H | H | H | ― | ― |
| 8-27 | Pt | 1 | 8 | CN | H | H | CF₃ | H | H | H | H | H | H | pic | |
| 8-27X | Pt | 1 | 8 | CN | H | H | CF₃ | H | H | H | H | H | H | acac | |
| 8-27Y | Pt | 0 | 8 | CN | H | H | CF₃ | H | H | H | H | H | H | - | - |
| 8-28 | Pt | 1 | 8 | CN | F | CN | H | H | H | H | H | H | H | pic | |
| 8-28X | Pt | 1 | 8 | CN | F | CN | H | H | H | H | H | H | H | acac | |
| 8-28Y | Pt | 0 | 8 | CN | F | CN | H | H | H | H | H | H | H | - | - |
| 8-29 | Pt | 1 | 8 | CN | CF₃ | CN | H | H | H | H | H | H | H | pic | |
| 8-29X | Pt | 1 | 8 | CN | CF₃ | CN | H | H | H | H | H | H | H | acac | |
| 8-29Y | Pt | 0 | 8 | CN | CF₃ | CN | H | H | H | H | H | H | H | ― | ― |

**Table 16**

| No. | M | m | BSS | R¹ | R² | R³ | R⁴ | R⁵ | R⁶ | R⁷ | R⁸ | R⁹ | R¹⁰ | L¹ | L² |
|---|---|---|---|---|---|---|---|---|---|---|---|---|---|---|---|
| 8'-1 | Pd | 1 | 8 | H | CN | H | H | H | H | H | H | H | H | pic | |
| 8'-1X | Pd | 1 | 8 | H | CN | H | H | H | H | H | H | H | H | acac | |
| 8'-1Y | Pd | 0 | 8 | H | CN | H | H | H | H | H | H | H | H | ― | ― |
| 8'-2 | Pd | 1 | 8 | H | H | CN | H | H | H | H | H | H | H | pic | |
| 8'-2X | Pd | 1 | 8 | H | H | CN | H | H | H | H | H | H | H | acac | |
| 8'-2Y | Pd | 0 | 8 | H | H | CN | H | H | H | H | H | H | H | ― | ― |
| 8'-3 | Pd | 1 | 8 | H | H | H | CN | H | H | H | H | H | H | pic | |
| 8'-3X | Pd | 1 | 8 | H | H | H | CN | H | H | H | H | H | H | acac | |
| 8'-3Y | Pd | 0 | 8 | H | H | H | CN | H | H | H | H | H | H | ― | ― |
| 8'-4 | Pd | 1 | 8 | CN | H | H | H | H | H | H | H | H | H | pic | |
| 8'-4X | Pd | 1 | 8 | CN | H | H | H | H | H | H | H | H | H | acac | |
| 8'-4Y | Pd | 0 | 8 | CN | H | H | H | H | H | H | H | H | H | ― | ― |
| 8'-5 | Pd | 1 | 8 | H | CN | H | CN | H | H | H | H | H | H | pic | |
| 8'-5X | Pd | 1 | 8 | H | CN | H | CN | H | H | H | H | H | H | acac | |
| 8'-5Y | Pd | 0 | 8 | H | CN | H | CN | H | H | H | H | H | H | ― | ― |
| 8'-6 | Pd | 1 | 8 | CN | H | CN | H | H | H | H | H | H | H | pic | |
| 8'-6X | Pd | 1 | 8 | CN | H | CN | H | H | H | H | H | H | H | acac | |
| 8'-6Y | Pd | 0 | 8 | CN | H | CN | H | H | H | H | H | H | H | ― | ― |
| 8'-7 | Pd | 1 | 8 | CF₃ | CN | CF₃ | H | H | H | H | H | H | H | pic | |
| 8'-7X | Pd | 1 | 8 | CF₃ | CN | CF₃ | H | H | H | H | H | H | H | acac | |
| 8'-7Y | Pd | 0 | 8 | CF₃ | CN | CF₃ | H | H | H | H | H | H | H | ― | ― |
| 8'-8 | Pd | 1 | 8 | H | CN | CF₃ | H | H | H | H | H | H | H | pic | |
| 8'-8X | Pd | 1 | 8 | H | CN | CF₃ | H | H | H | H | H | H | H | acac | |
| 8'-8Y | Pd | 0 | 8 | H | CN | CF₃ | H | H | H | H | H | H | H | ― | ― |
| 8'-9 | Pd | 1 | 8 | H | CN | H | F | H | H | H | H | H | H | pic | |
| 8'-9X | Pd | 1 | 8 | H | CN | H | F | H | H | H | H | H | H | acac | |
| 8'-9Y | Pd | 0 | 8 | H | CN | H | F | H | H | H | H | H | H | ― | ― |
| 8'-10 | Pd | 1 | 8 | H | F | H | CN | H | H | H | H | H | H | pic | |
| 8'-10X | Pd | 1 | 8 | H | F | H | CN | H | H | H | H | H | H | acac | |
| 8'-10Y | Pd | 0 | 8 | H | F | H | CN | H | H | H | H | H | H | ― | ― |
| 8'-11 | Pd | 1 | 8 | CN | F | CN | F | H | H | H | H | H | H | pic | |
| 8'-11X | Pd | 1 | 8 | CN | F | CN | F | H | H | H | H | H | H | acac | |
| 8'-11Y | Pd | 0 | 8 | CN | F | CN | F | H | H | H | H | H | H | ― | ― |
| 8'-12 | Pd | 1 | 8 | CF₃ | CN | CF₃ | CN | H | H | H | H | H | H | pic | |
| 8'-12X | Pd | 1 | 8 | CF₃ | CN | CF₃ | CN | H | H | H | H | H | H | acac | |
| 8'-12Y | Pd | 0 | 8 | CF₃ | CN | CF₃ | CN | H | H | H | H | H | H | ― | ― |
| 8'-13 | Pd | 1 | 8 | CF₃ | H | CF₃ | CN | H | H | H | H | H | H | pic | |
| 8'-13X | Pd | 1 | 8 | CF₃ | H | CF₃ | CN | H | H | H | H | H | H | acac | |
| 8'-13Y | Pd | 0 | 8 | CF₃ | H | CF₃ | CN | H | H | H | H | H | H | ― | ― |
| 8'-14 | Pd | 1 | 8 | CF₃ | H | H | CN | H | H | H | H | H | H | pic | |
| 8'-14X | Pd | 1 | 8 | CF₃ | H | H | CN | H | H | H | H | H | H | acac | |
| 8'-14Y | Pd | 0 | 8 | CF₃ | H | H | CN | H | H | H | H | H | H | ― | ― |
| 8'-15 | Pd | 1 | 8 | H | CF₃ | H | CN | H | H | H | H | H | H | pic | |
| 8'-15X | Pd | 1 | 8 | H | CF₃ | H | CN | H | H | H | H | H | H | acac | |
| 8'-15Y | Pd | 0 | 8 | H | CF₃ | H | CN | H | H | H | H | H | H | ― | ― |
| 8'-16 | Pd | 1 | 8 | H | F | CN | F | H | H | H | H | H | H | pic | |
| 8'-16X | Pd | 1 | 8 | H | F | CN | F | H | H | H | H | H | H | acac | |
| 8'-16Y | Pd | 0 | 8 | H | F | CN | F | H | H | H | H | H | H | ― | ― |
| 8'-17 | Pd | 1 | 8 | CF₃ | F | CN | F | H | H | H | H | H | H | pic | |
| 8'-17X | Pd | 1 | 8 | CF₃ | F | CN | F | H | H | H | H | H | H | acac | |
| 8'-17Y | Pd | 0 | 8 | CF₃ | F | CN | F | H | H | H | H | H | H | ― | ― |
| 8'-18 | Pd | 1 | 8 | CN | CN | CN | CN | H | H | H | H | H | H | pic | |
| 8'-18X | Pd | 1 | 8 | CN | CN | CN | CN | H | H | H | H | H | H | acac | |
| 8'-18Y | Pd | 0 | 8 | CN | CN | CN | CN | H | H | H | H | H | H | ― | ― |

The metal-complex compound of the present invention is preferably a material for a light emitting element and in particular, more preferably a material. for the organic EL device.

The present invention provides an organic EL device which comprises at least one organic thin film layer sandwiched between a pair of electrode consisting of an anode and a cathode, wherein the organic thin film layer comprises the above metal-complex compound, which emits light by applying an electric voltage between the pair of electrode.

With regard to the amount of the metal complex compound of the present invention contained in the organic thin film layer, it is usually 0.1 to 100 % by weight, preferably 1 to 30 % by weight of total mass of the light emitting layer.

It is preferable for the organic EL device of the present invention that the light emitting layer comprises the metal-complex compound of the present invention. Further, the light emitting layer is usually formed to a thin film by means of vapor deposition process or coating process, however, it is preferable that the layer comprising the metal-complex compound of the present invention is formed into film by coating orocess because it simplifies the production process.

In the organic EL device of the present invention, a monolayer-type organic thin layer consists of a light emitting layer, which comprises the metal-complex compound of the present invention. Typical examples of the construction of a multilayer-type organic EL device include (an anode / a hole injecting layer (a hole transporting layer) / a light emitting layer / a cathode); (an anode / a light emitting layer / an electron injecting layer (an electron transporting layer) / a cathode); (an anode / a hole injecting layer (a hole transporting layer) / a light emitting layer / an electron injecting layer (an electron transporting layer) / a cathode).

The anode in the organic EL device covers a role of supplying holes into a hole injecting layer, a hole transporting layer or into a light emitting layer, and it is effective that the anode has a work function of 4.5 eV or greater. As the material for the anode, metals, alloys, metal oxides, electroconductive compounds, or these mixtures may be employable. Specific examples of the material for the anode include electroconductive metal oxides such as tin oxide, zinc oxide, indium oxide, indium oxide tin (ITO), etc.; metals such as gold, silver, chromium, nickel, etc.; mixtures or laminated materials of these electroconductive metal oxide and metals; inorganic electroconductive substance such as copper iodide, chalocite, etc.; organic electroconductive materials such as polyaniline, polythiophene, polypyrrole, etc.; and laminated materials of the above materials with ITO; preferably are the electroconductive metal oxides. Particularly, it is preferable to employ ITO from viewpoints such as productivity, enhanced electroconductivity, transparency, etc. Regarding with a film thickness of the anode, it is possible to be appropriately selected depending on the material.

The cathode in the organic EL device covers a role of supplying electronds into an electrom injecting layer, an electron transporting layer or into a light emitting layer and examples of the material for the cathode include alkali metals (for example, Li, Na, K, etc.) and their fluoride or oxidate, alkaline earth metals (for example, Mg, Ca, etc.) and their fluoride or oxidate, gold, silver, lead, aluminium, sodium-potassium alloy or sodium-potassium mixed metals, lithium-aluminium alloy or lithium-aluminium mixed metals, magnesium-silver alloy or the magnesium-silver mixed metals, or rare earth metals such as indium, ytterbium, etc. Among those, preferable examples are aluminum, lithium-aluminum alloy or lithium-aluminum mixed metals, magnesium-silver alloy or magnesium-silver mixed metals, etc. The cathode may be a monolayer structure of the above material, and may be a laminated structure of the layer containing the above material. For example, the laminated structure such as aluminum / lithium fluoride, aluminum / lithium oxide or the like is preferable.

It may be appropriate that the hole injecting layer and the hole transporting layer of the organic EL device of the present invention have any function of injecting holes from the anode, transporting holes, or barriering the electrons injected from the cathode. Specific examples include carbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino substituted chalcone derivatives, styryl anthracene derivatives, fluorenone derivatives, hydrazone derivatives, stilbene derivatives, silazane derivatives, aromatic tertiary amine compounds, styryl amine compound, aromaticdimethylidene-based compounds, porphyrin-based compounds, polysilane-based compounds, poly(N-vinyl carbazole) derivatives, aniline-based copolymer; electroconductive polymer oligomer such as thiophene oligomer, polythiophene, etc.; organosilane derivatives, metal-complex compound of the present invention, etc. The hole injecting layer and the hole transporting layer may be composed of single layer comprising one or more kind of these hole injecting materials and these hole transporting materials or may be laminated with themselves or a layer comprising another kind of compound.

It may be appropriate that the electron injecting layer and the electron transporting layer of the organic EL device of the present invention have any function of injecting electrons from the cathode, transporting electrons, or barriering the holes injected from the anode. Specific examples include triazole derivatives, oxazole derivatives, oxadiazole derivatives, midazole derivatives, fluorenone derivatives, anthraquinodimethane derivatives, anthrone derivatives, diphenylchinone derivatives, thiopyrandioxide derivatives, carbodiimide derivatives, fluorenylidene methane derivatives, distyrylpyrazine derivatives; aromatic ring tetracarboxylic acid anhydride such as naphthalene, perylene, etc.; phthalocyanine derivatives, various metallic complexes represented by metallic complexes of 8-quinolinol derivatives or metallic complexes having benz oxazole or benzothiazole as ligand; organosilane derivatives, metal-complex compound of the present invention, etc. The electron injecting layer may be composed of single layer comprising one or more kind of these electron injecting materials or may be laminated with an electron injecting layer comprising another kind of compound.

Further, following compounds are employable for the electron injecting layer and the electron transporting layer.

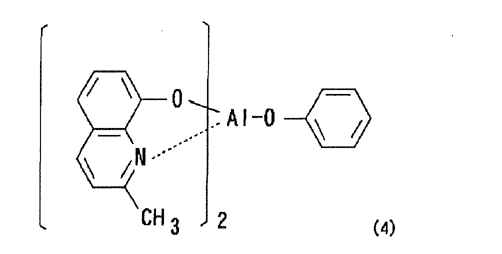

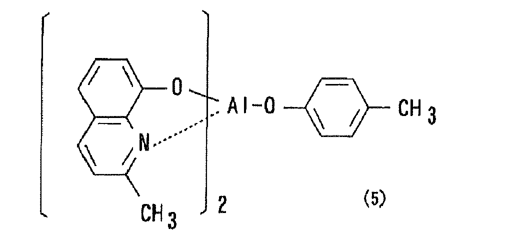

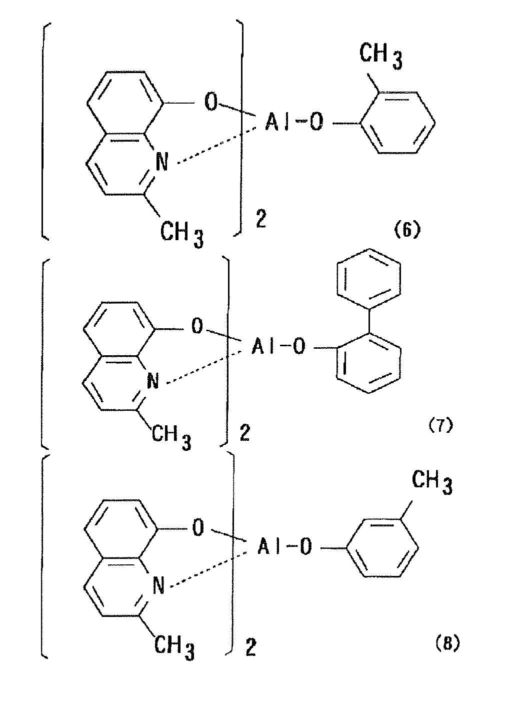

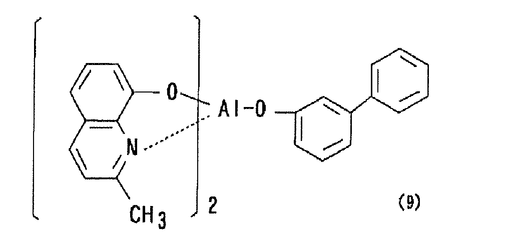

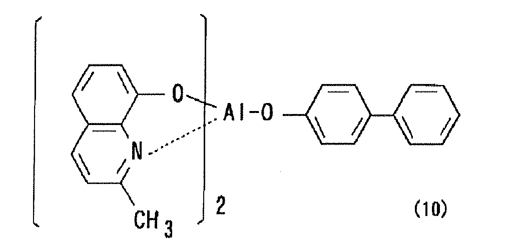

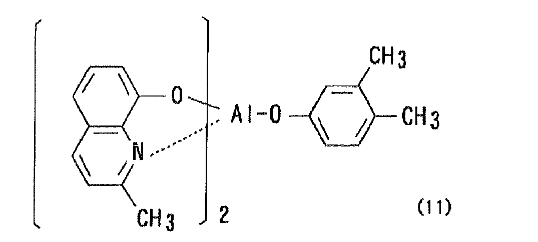

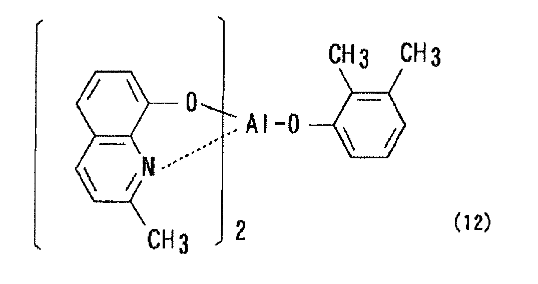

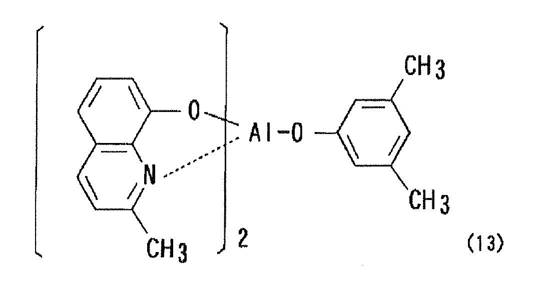

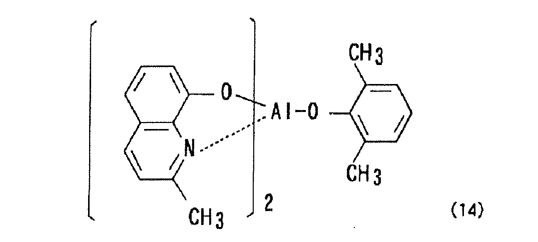

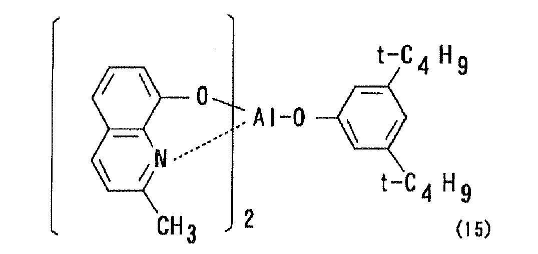

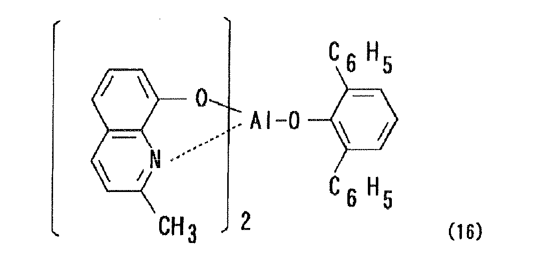

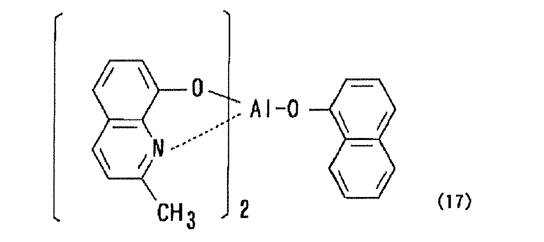

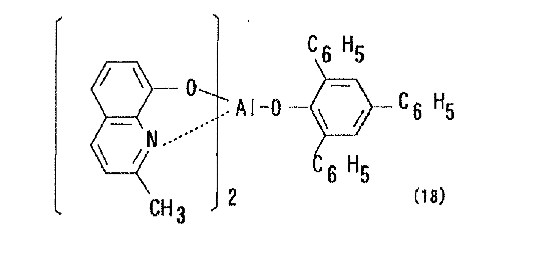

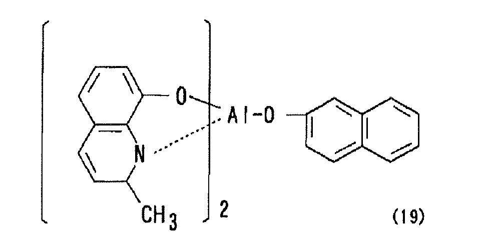

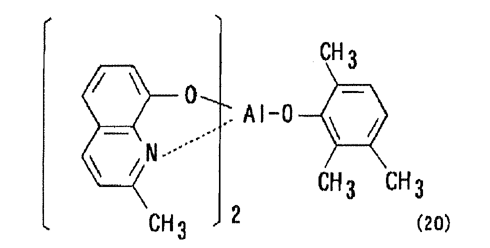

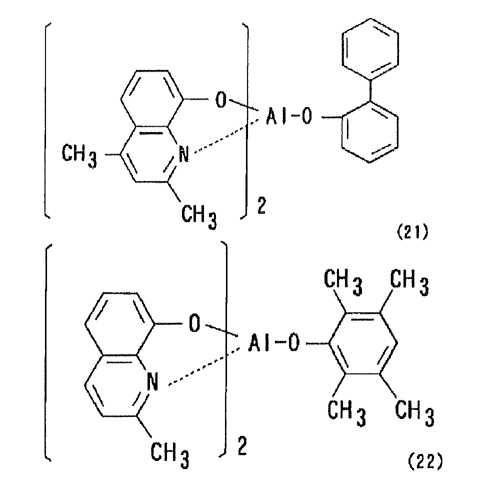

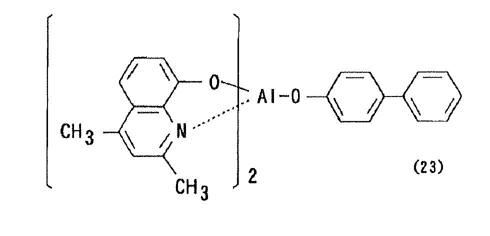

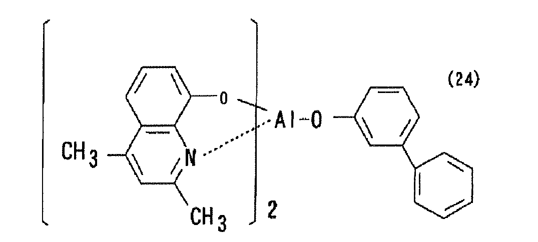

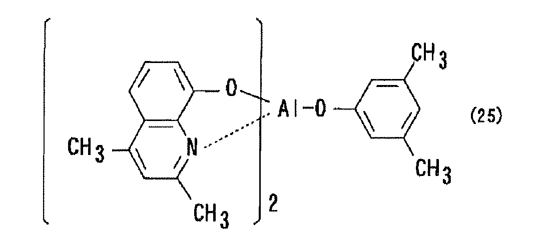

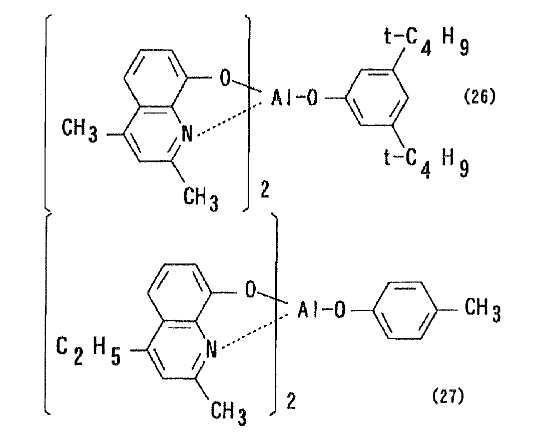

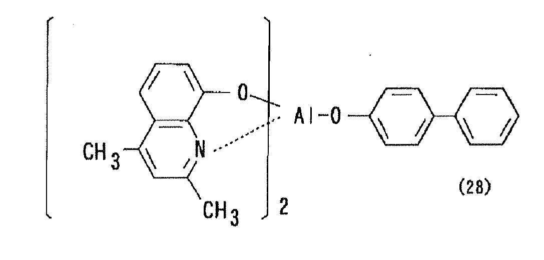

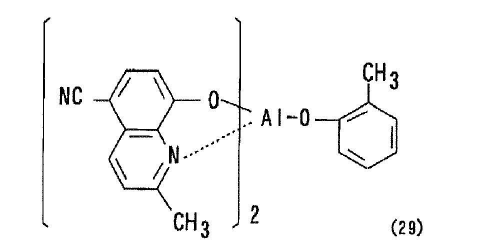

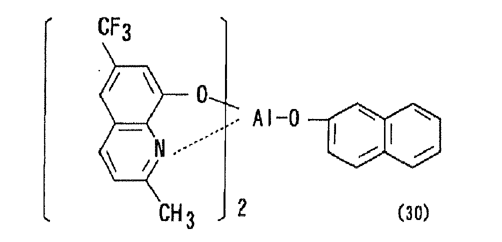

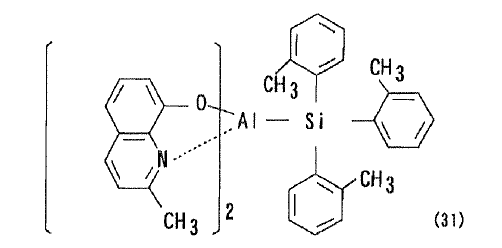

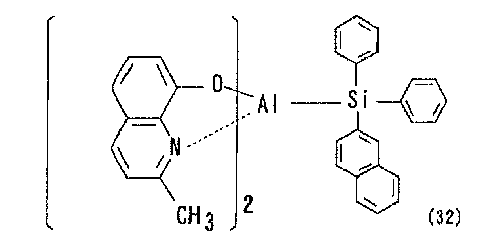

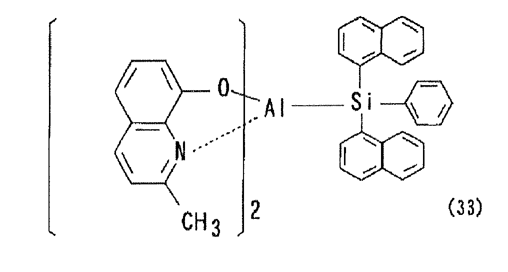

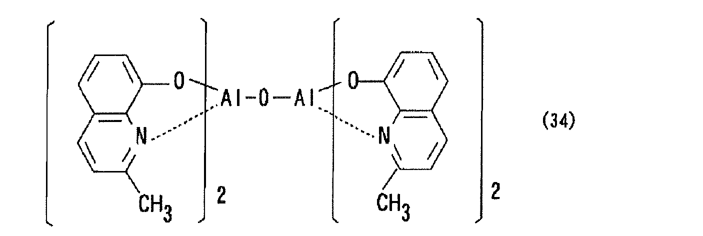

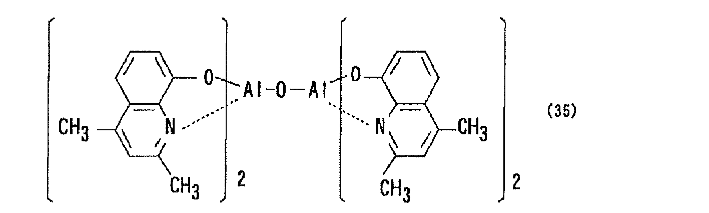

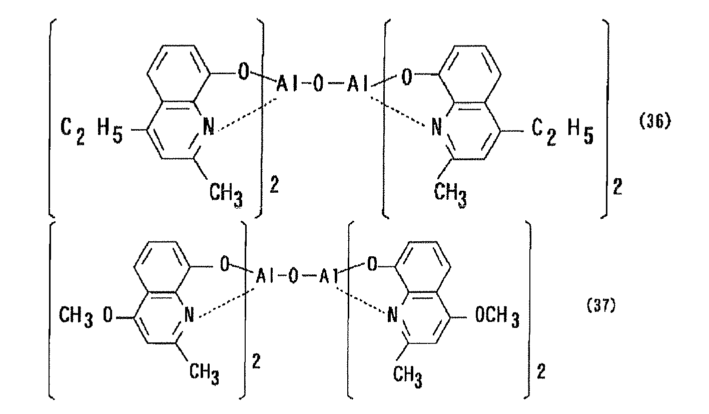

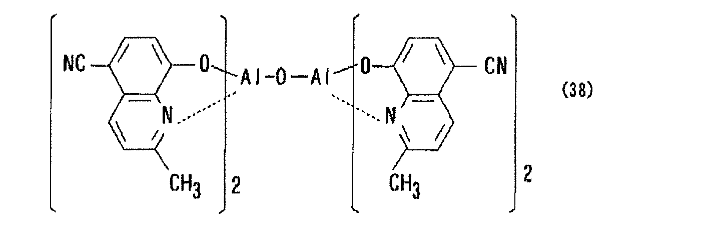

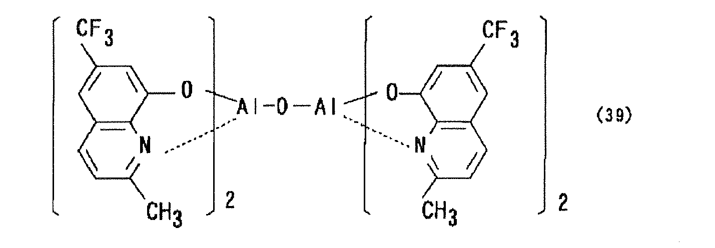

It is preferable for the organic EL device of the present invention that the electron injectiing layer and / or the electron transporting layer emprises a π -electron lacking heteroring derivative having a nitrogen atom as its essential component.

Preferable examples of the π -electron lacking heteroring derivative having a nitrogen atom include derivatives of five-member ring having a nitrogen atom selected from among benzimidazole ring, benztriazole ring, pyridino imidazole ring, pyrimidino imidazole ring, pyridazino imidazole ring; or derivatives of six-member ring having a nitrogen atom consisting of pyridine ring, pyrimidine ring, pyrazine ring or triazine ring. A structure expressed by general formula B-I below is preferable as the derivatives of five-member ring having a nitrogen atom. Structures expressed by general formulae C-I, C-II, C-III, C-IV, C-V and C-VI below are preferable as the derivatives of six-member ring having a nitrogen atom, while general formulae C-I and C-11 are more preferable.

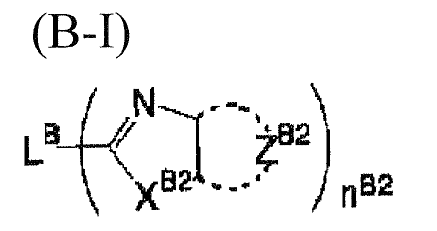

In general formula (B-1), L^{B} represents a connecting group of divalent or more, preferably the connecting group formed with a carbon atom, a silicon atom, a nitrogen atom, a boron atom, an oxygen atom, a sulfur atom, metals, metal ions, etc.; more preferably the connecting group formed with a carbon atom, a silicon atom, a nitrogen atom, a boron atom, an oxygen atom, a sulfur atom, an aromatic hydrocarbon ring, an aromatic heteroring; and the most preferably the connecting group formed with a carbon atom, a silicon atom, an aromatic hydrocarbon ring and an aromatic heteroring.

L^{b} may have a substituent, and preferable examples of the substituent are alkyl group, alkenyl group, alkynyl group, aromatic hydrocarbon group, amino group, alkoxy group, aryloxy group, acyl group, alkoxycarbonyl group, aryloxy carbonyl group, acyl oxy group, acylamino group, alkoxycarbonylamino group, aryloxy carbonylamino group, sulfonyl amino group, sulfamoyl group, carbamoyl group, alkylthio group, arylthio group, sulphonyl group, halogen atom, cyano group and aromatic heterocycle group; more preferable examples are alkyl group, aryl group, alkoxy group, aryloxy group, halogen atom, cyano group and aromatic heterocycle group; furthermore preferable examples are alkyl group, aryl group, alkoxy group, aryloxy group and aromatic heterocycle group; and particularly preferable examples are alkyl group, aryl group, alkoxy group and aromatic heterocycle group.

Followings are the specific examples of the connecting group represented by Lb:

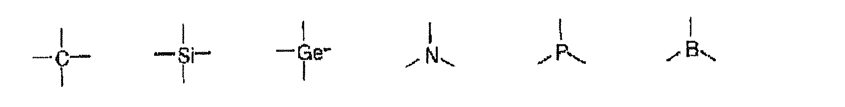

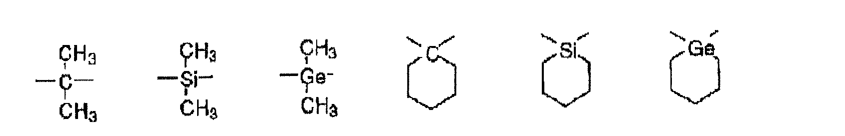

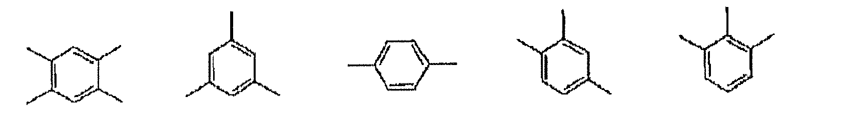

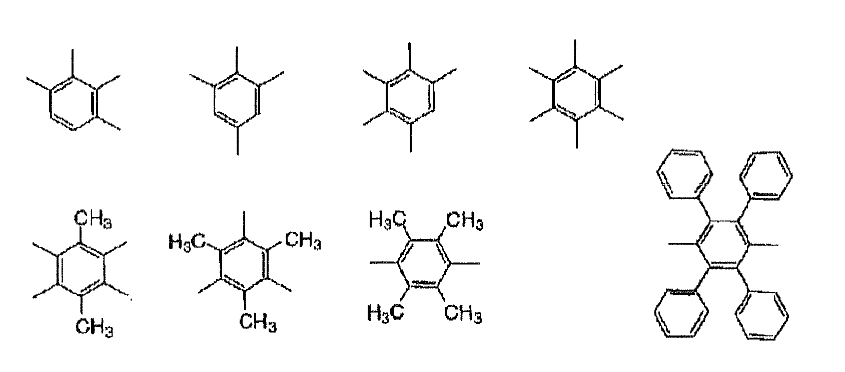

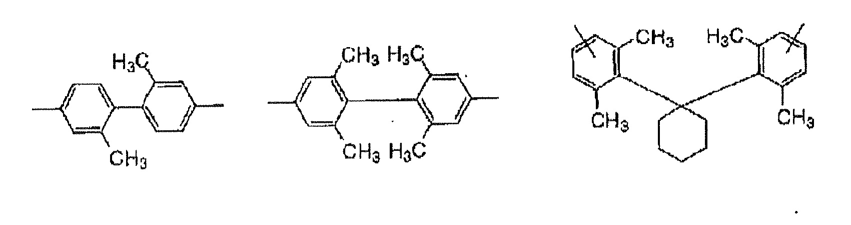

In the general formula (B-1), X^{B2} represents ―O―, ―S― or = N―R^{B2}. R^{B2} represents a hydrogen atom, an aliphatic hydrocarbon group, an aryl group and a heteroring group.

The aliphatic hydrocarbon group represented by R^{B2} is straight chain, branched or circular alkyl group (alkyl group preferably having 1 to 20 carbon atoms, more preferably having 1 to 12 carbon atoms and particularly preferably having 1 to 8 carbon atoms; examples include methyl group, ethyl group, iso-propyl group, tert-butyl group, n-octyl group, n-decyl group, n-hexadecyl group, cyclopropyl group, cyclopentyl group, cyclohexyl group, etc.); alkenyl group (alkenyl group preferably having 2 to 20 carbon atoms, more preferably having 2 to 12 carbon atoms and particularly preferably having 2 to 8 carbon atoms; examples include vinyl group, allyl group, 2-butenyl group, 3-pentenyl group, etc.); and alkynyl group (alliynyl group preferably having 2 to 20 carbon atoms, more preferably having 2 to 12 carbon atoms and particularly preferably having 2 to 8 carbon atoms; examples include propargyl group, 3-pentynyl group, etc.), while the above alkyl group being more preferable.

The aryl group represented by R^{B2} is a monocyclic or condensed ring aryl group, preferably the aryl group having 6 to 30 carbon atoms, more preferably 6 to 20 carbon atoms and further preferably 6 to 12 carbon atoms; examles include phenyl group, 2-methylphenyl group, 3-methylphenyl group, 4-methylphenyl group, 2-methoxyphenyl group, 3-trifluoromethylphenyl group, pentafluorophenyl group, 1-naphthyl group, 2-naphthyl group, etc.

The heteroring group represented by R^{B2} is a monocyclic or condensed ring heteroring group, preferably the hetero ring group having 1 to 20 carbon atoms, more preferably 1 to 12 carbon atoms and further preferably 2 to 10 carbon atoms; examles include pyrrolidine, piperidine, piperazine, morpholine, thiophene, selenophene, furan, pyrrole, imidazole, pyrazole, pyridine, pyrazine, pyridazine, pyrimidine, triazole, triazine, indole, indazole, purine, thiazoline, thiazole, thiadiazole, oxazoline, oxazole, oxadi azole, chinoline, isoquinoline, phthalazine, naphthyridine, quinoxaline, quinazoline, quinoliine, pteridine, acridine, phenanthroline, phenazine, tetrazole, benzimidazole, benz oxazole, benzothiazole, benz triazole, tetrazaindene, carbazole, azepin, etc.; while furan, thiophene, pyridine, pyrazine, pyrimidine, pyridazine, triazine, quinoline, phthalazine, naphthyridine, quinoxaline and quinazoline are preferable; furan, thiophene, pyridine and chinoline are more preferable; and quinoline is further more preferable.

The aliphatic hydrocarbon group, the aryl group and the heteroring group represented by R^{B2} may have a substituent whose examples are the same as the above preferable examples of the substituent of L^{B}.

The aliphatic hydrocarbon group, the aryl group and the heteroring group are preferable as R^{B2}, the aryl group and the heteroring group are more preferable and the aryl group is further more preferable.

In the general formula (B-I), X^{B2} is preferably ―O― or = N―R^{B2}, more preferably = N―R^{B2} and particularly prferably = N―Ar^{B2}; while Ar^{B2} is an aryl group (preferably the aryl group having 6 to 30 carbon atoms, more preferably 6 to 20 carbon atoms and further more preferably 6 to 12 carbon atoms); or an aromatic hetero ring group (preferably the aromatic hetero ring group having 1 to 20 carbon atoms, more preferably 1 to 12 carbon atoms and further more preferably 2 to 10 carbon atoms).

In the general formula (B-I), Z^{B2} represents an atomic group necessary for forming aromatic group rings. The aromatic group ring formed by Z^{B2} may be any of an aromatic hydrocarbon ring or an aromatic heteroring; examplles include benzene ring, pyridine ring, pyrazine ring, pyrimidine ring, pyridazine ring, triazine ring, pyrrole ring, furan ring, thiophene ring, selenophene ring, tellurophene ring, imidazole ring, thiazole ring, selenazole ring, tellurazole ring, thiadiazole ring, oxadi azole ring, pyrazole ring, etc.; while benzene ring, pyridine ring, pyrazine ring, pyrimidine ring and pyridazine ring are preferable; benzene ring, pyridine ring and pyrazine ring are more preferable; benzene ring and pyridine ring are further more preferable; and pyridine ring is particularly preferable. The aromatic group ring formed by Z^{B2} may form a condensed ring with other ring or may have a substituent. Preferable substituents for Z^{B2} are alkyl group, alkenyl group, alkynyl group, aryl group, amino group, alkoxy group, aryloxy group, acyl group, alkoxycarbonyl group, aryloxy carbonyl group, acyl oxy group, acylamino group, alkoxycarbonylamino group, aryloxy carbonylamino group, sulfonyl amino group, sulfamoyl group, carbamoyl group, alkylthio group, arylthio group, sulphonyl group, halogen atom, cyano group and heteroring group; more preferable substituents for Z^{B2} are alkyl group, aryl group, alkoxy group, aryloxy group, halogen atom, cyano group and heteroring group; further more preferable substituents for Z^{B2} are alkyl group, aryl group, alkoxy group, aryloxy group and heteroring group; particularly preferable substituents for Z^{B2} are alkyl group, aryl group, alkoxy group and heteroring group.

In the general formula (B-I), n^{B2} represents an integer of 1 to 4, preferably an integer of 2 or 3.

Among the compounds expressed by the general formula (B-I), further preferable compounds are expresed by a following general formula (B- II):

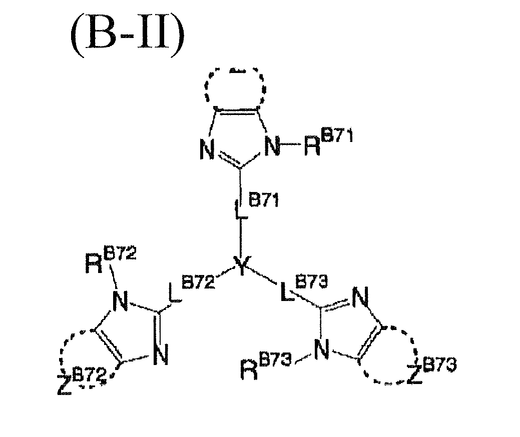

In the general formula (B-II), R^{B71}, R^{B72} and R^{B73} each represents the same as R^{B2} in the general formula (B-I), wherein the preferable range is also the same.

In the general formula (B-II), Z^{B71}, Z^{B72} and Z^{B73} each represents the same as Z^{B2} in the general formula (B-I), wherein the preferable range is also the same.

In general formula (B-II), L^{B71}, L^{B72} and L^{B73} each represents a connecting group of divalent or more described as the examples of L^{B} in the general formula (B-I); preferably a single bond, a divalent aromatic hydrocarbon ring group, a divalent aromatic heteroring group; and the connecting group formed by those combination; more preferably a single bond. L^{B71}, L^{B72} and L^{B73} may have a substituent, whose examples are the same as described as the substituent for L^{B} in the general formula (B-I).

Y represents a nitrogen atom, a 1,3,5-benzenetriyl group or a 2,4,6-triazinetriyl group. The 1,3,5-benzenetriyl group may have substituent at 2, 4 and 6-positions, examples of the substituent include alkyl group, aromatic hydrocarbon ring group, halogen atom, etc. Specific examples of the derivatives of five-member ring having a nitrogen atom represented by the general formula (B-I) or the general formula (B-II) include the following compounds, though not limited thereto. 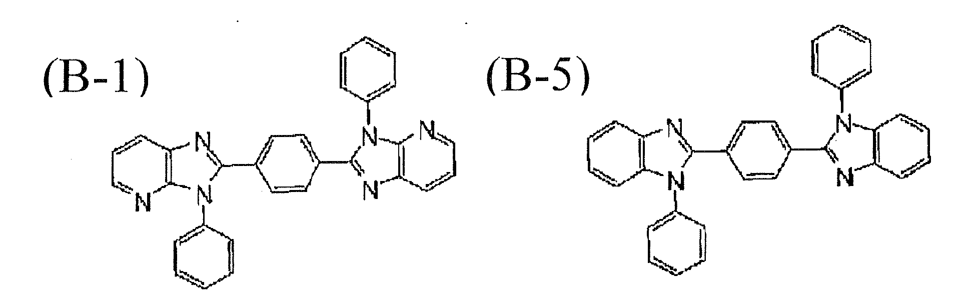 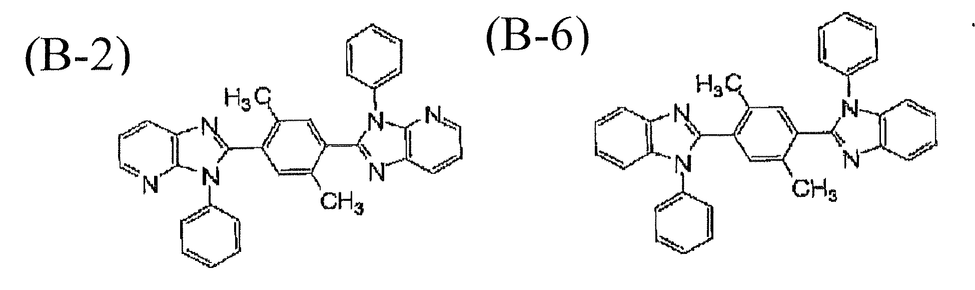 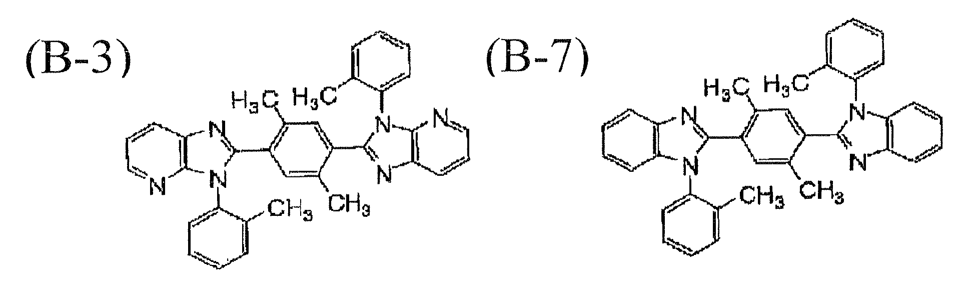 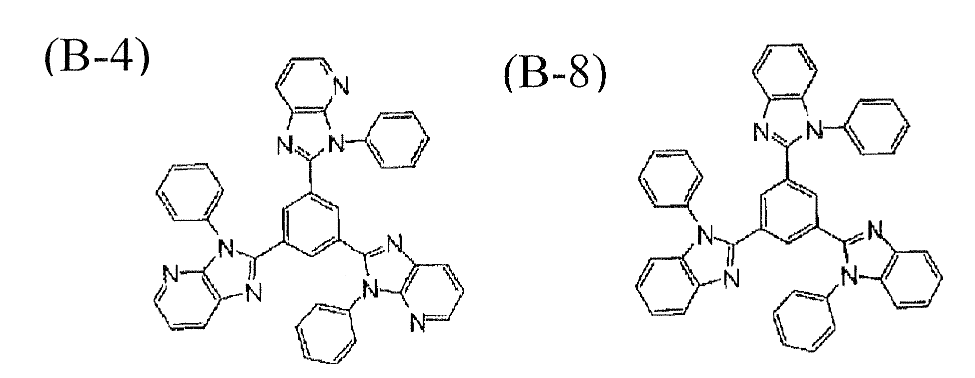 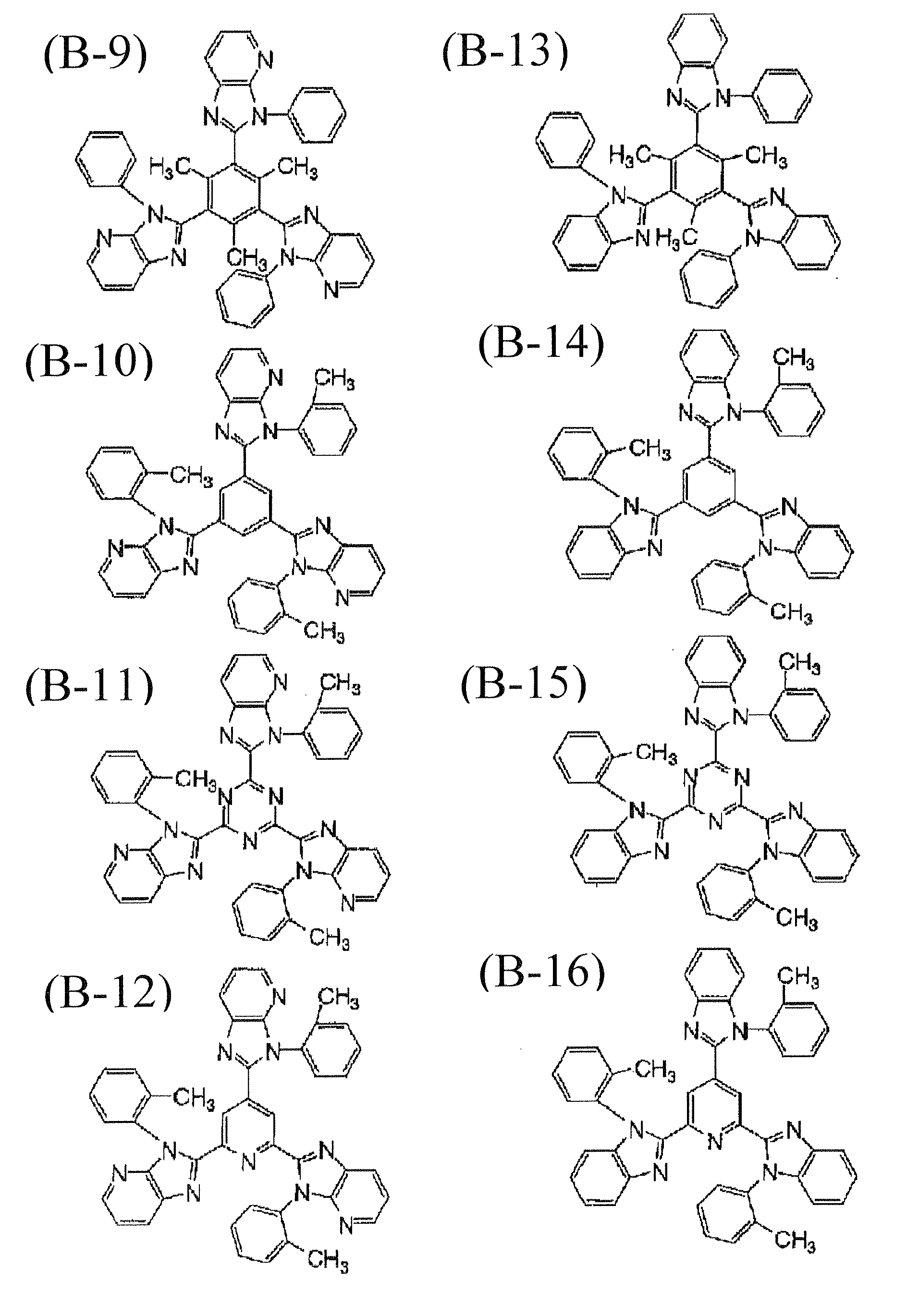 (Cz-)nA (C-I)

Cz(-A)m (C-II)

wherein Cz represents a substituted or unsubstituted carbazolyl group, an aryl carbazolyl group or a carbazolylalkylene group;
**A** represents a group formed by a portion expressed by a following general formula (A); and **n** and **m** each represents an integer of 1 to 3.

(M)p―(L) q―(M')r (A)

wherein **M** and **M'** each independently represents a heteroaromatic ring having a nitrogen atom and further having 2 to 40 carbon atoms, which forms a ring with or without a substituent; further, **M** and **M'** may be the same with or different from each other. **L** represents a single bond, an arylene group having 6 to 30 carbon atoms, a cycloalkylene group of having 5 to 30 carbon atoms or a heteroaromatic ring having 2 to 30 carbon atoms, each may have or may not have a substituent which bonds to the ring. **p** represents an integer of 0 to 2, **q** represents an integer of 1 or 2 and **r** represents an integer of 0 to 2. However, **p** + **r** is 1 or greater.

Coupling styles of the general formula (C-I) and the general formula (C -II) are expressed in the following table concretely depending on a number of parameters **n** and **m.**

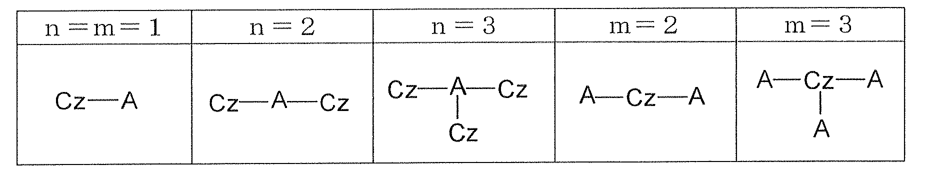

Further, coupling styles of the groups represented by general formula (A) are expressed in the following tables (1) to (16) concretely depending on a number of parameters **p, q** and **r.**

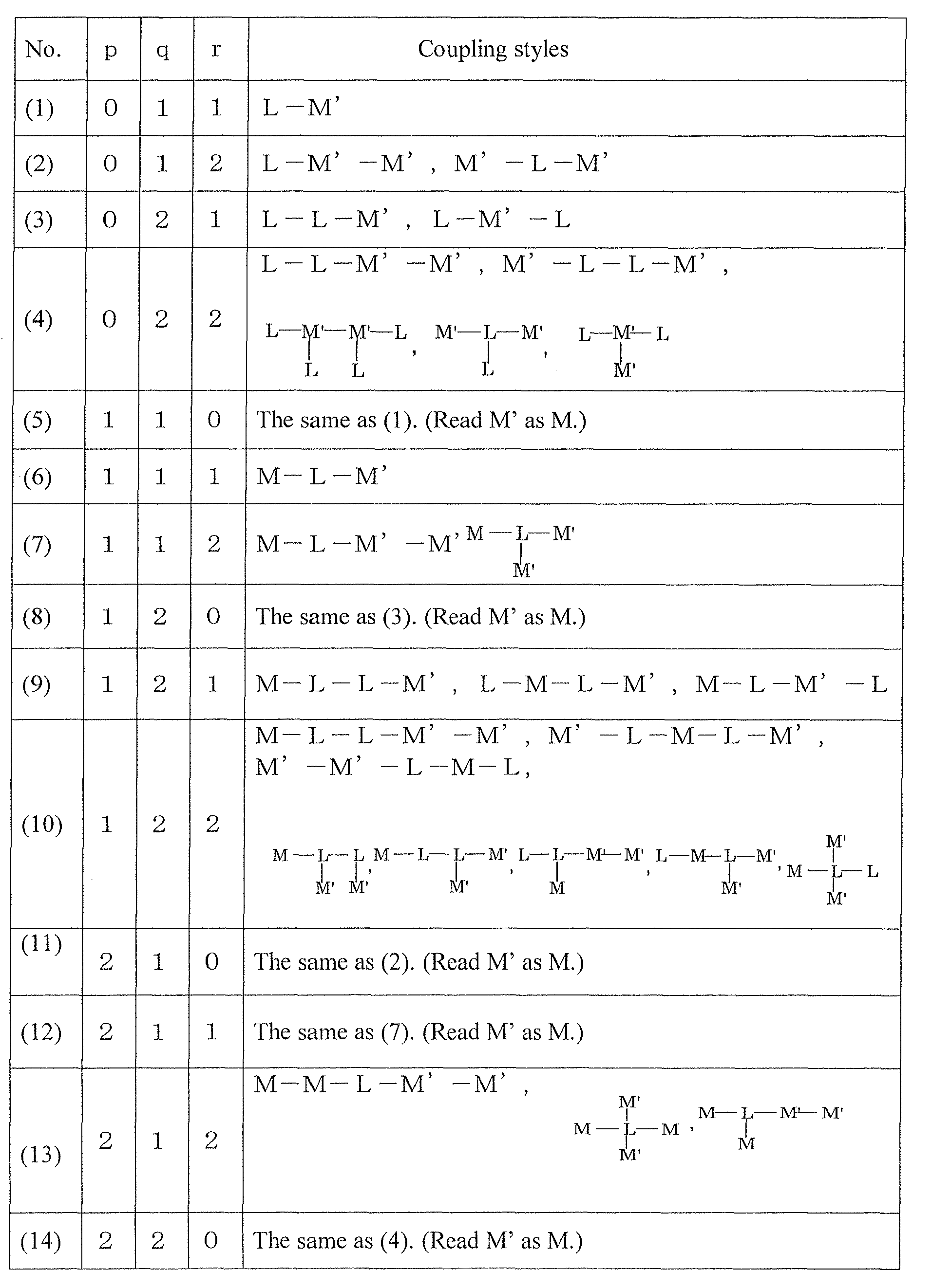

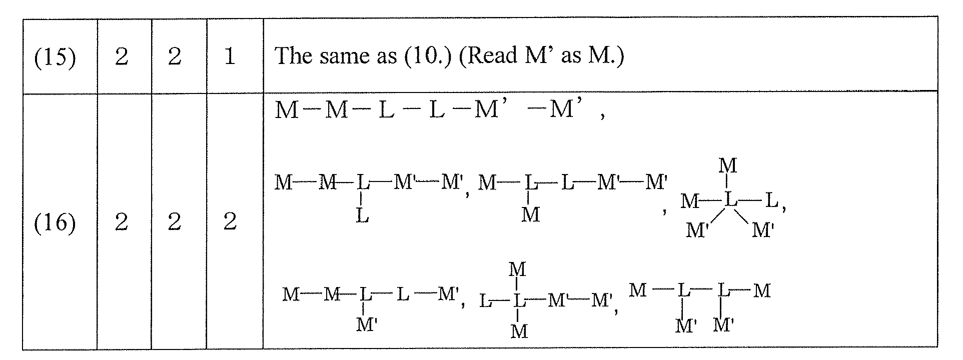

In the general formulae (C-I) and (C-II), when Cz couples with **A,** Cz may bond at any portion of **M, L** or **M'** expressing **A.** For example, in a case where **p** = **q** = **r** = 1 ((6) in table) in Cz―A wherein m = n =1, **A** becomes as **M-L** ―M' and expressed by three coupling styles of Cz―M―L―M', M―L (―Cz)―M' and M―L―M'―Cz. In the same way, in a case where **p** = **q** = 1 and **r** = 2 ((7) in table) in Cz―A―Cz wherein n= 2 in the general formula (C-I), **A** becomes as **M** ―L―M'―M' or M―L(―M')―M' and expressed by following coupling styles:

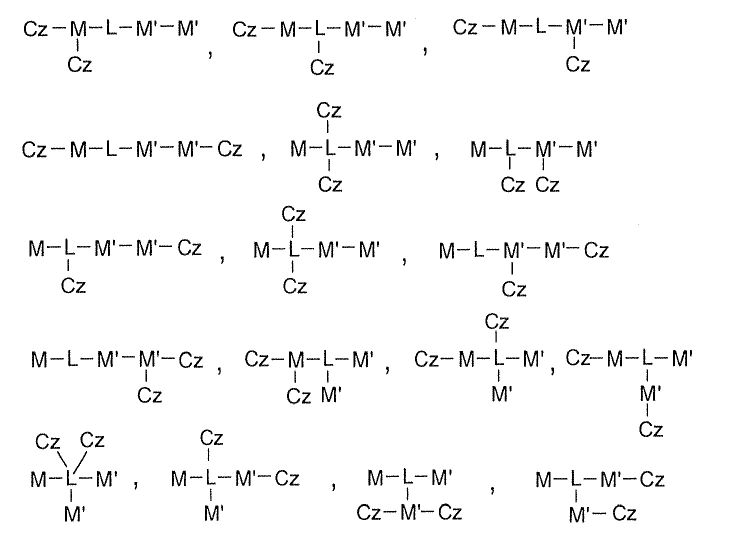

Specific examples of the structure represented by general formulae (C-I) and (C-II) include the structures shown in the following, however, they are not limited to the following. 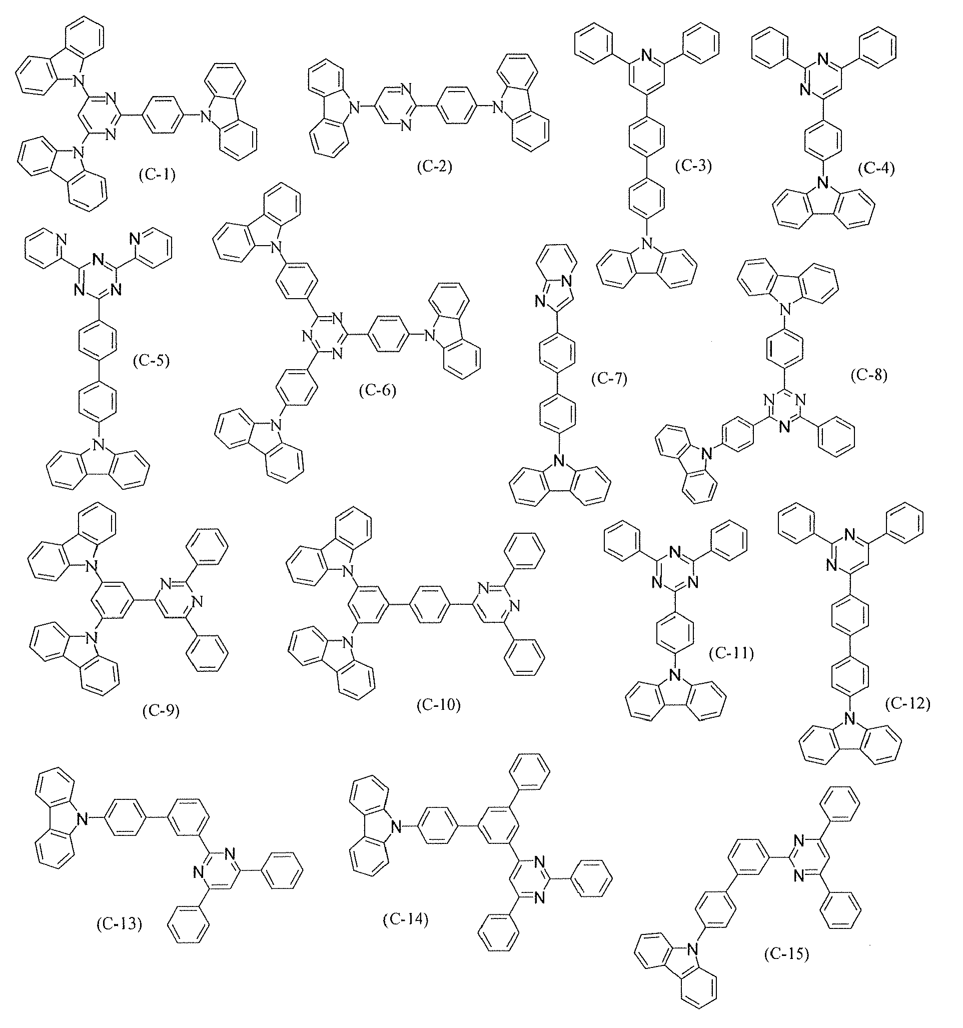 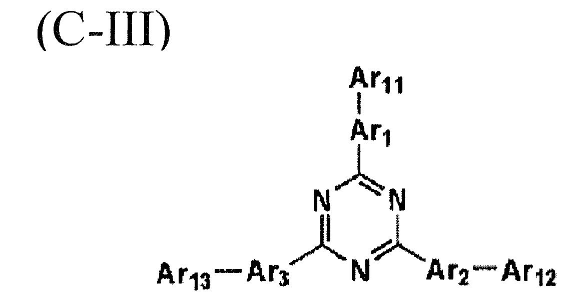 wherein Aru to Aris each represents the same group as R^{B2} in the general formula (B-I), specific examples are also same as those of R^{B2}; Ar₁ to Ar₃ represents a divalent group of the same group as R^{B2} in the general formula (BI), specific examples being the same.

Specific examples of the structure represented by general formula (C-III) include the structures shown in the following, however, they are not limited to the following. 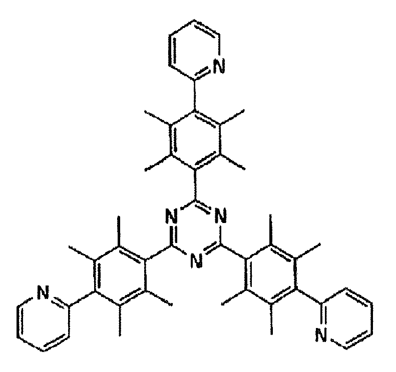 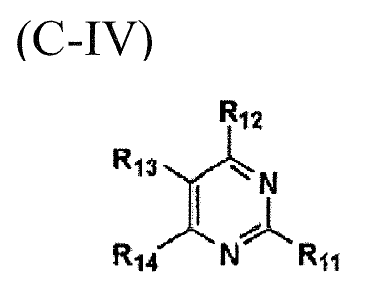 wherein R¹¹ to R¹⁴ each represents the same group as R^{B2} in the general formula (B-I), specific examples are also same as those of R^{B2}

Specific examples of the structure represented by general formula (C-IV) include the structures shown in the following, however, they are not limited to them. 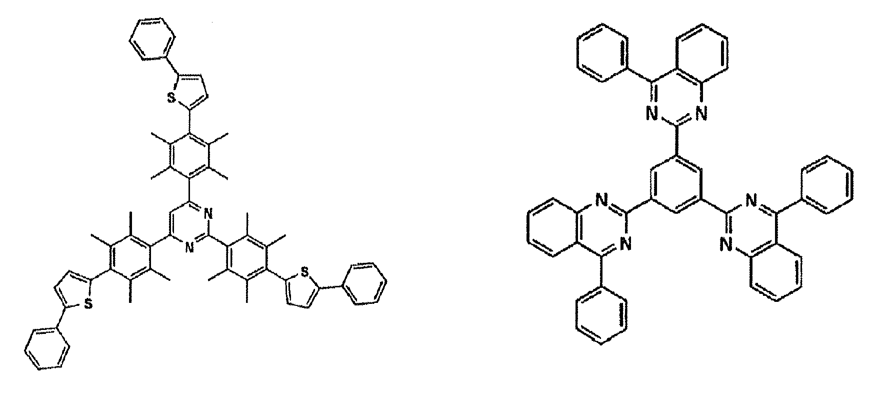 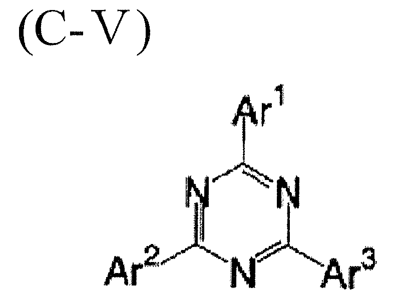 wherein Ar¹ to Ar³ each represents the same group as R^{B2} in the general formula (B―I), specific examples are also same as those of R^{B2}

Specific examples of the structure represented by general formula (C―V) include the structures shown in the following, however, they are not limited to them. 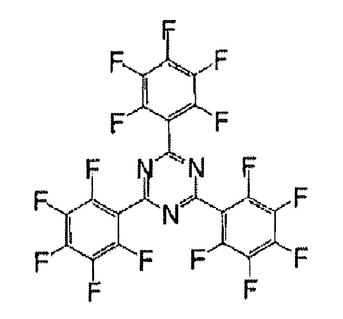 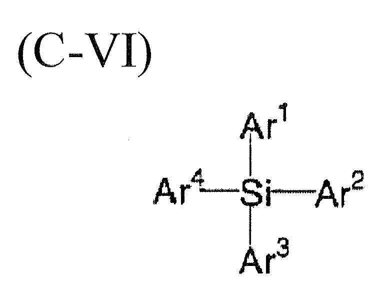 wherein Ar¹ to Ar⁴ each represents the same group as R^{B2} in the general formula (B-I), specific examples are also same as those of R^{B2},

Specific examples of the structure represented by general formula (C-VI) include the structures shown in the following; however, they are not limited to them.

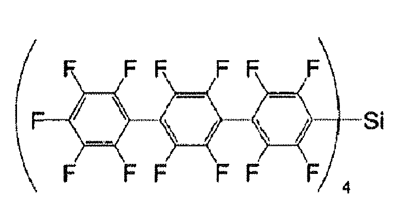

Further in the organic EL device of the present invention, it is preferable to employ an inorganic compound such as an insulating material or a semiconductor for an electron injecting or transporting layer. The electron injecting or transporting layer employing an insulating material or a semiconductor effectively prevents leak in the electric current and improves the electron injecting capability. It is preferable that at least one metal compound selected from the group consisting of alkali metal chalcogenides, alkaline earth metal chalcogenides, alkali metal halides and alkaline earth metal halides is used as the insulating material. It is preferable that the electron injecting or transporting layer is constituted with the above alkali metal chalcogenide since the electron injecting property can be improved.

Preferable examples of the alkali metal chalcogenide include Li₂O, LiO, Na₂S and Na₂Se and NaO. Preferable examples of the alkaline earth metal chalcogenide include CaO, BaO, SrO, BeO, BaS and CaSe. Preferable examples of the alkali metal halide include LiF, NaF, KF, LiCl, KCl, NaCl, etc. Preferable examples of the alkaline earth metal halide include fluorides such as CaF₂, BaF₂, SrF₂, MgF₂ and BeF₂ and halides other than the fluorides.

Examples of the semiconductor constituting the electron injecting or transporting layer include oxides, nitrides and nitriding oxides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound constituting the electron injecting or transporting layer is in the form of a fine crystalline or amorphous insulating thin film. When the electron injecting or transporting layer is constituted with the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.

In the present invention, a reductive dopant with a work function of 2.9 eV or smaller may be added in the electron injecting or transporting layer. The reductive dopant used in the present invention is defined as a substance which reduces the electron transporting compound. In the present invention, it is preferable that the reductive dopant is added in the interfacial zone between the cathode and the organic thin film layer of the organic EL device, and the reductive dopant reduces at least a part of the organic layer resultantly making it anion. Examples of the reductive dopant include at least one compound selected from alkali metals, alkali metal complexes, alkali metal compounds, alkaline earth metals, alkaline earth metal complexes, alkaline earth metal compounds, rare earth metals, rare earth metal complexes and rare earth metal compounds. Examples of the preferable reductive dopant include at least one alkali metal selected from a group consisting of Li (the work function: 2.93 ev), Na (the work function: 2.36 eV), K (the work function: 2.28 eV), Rb (the work function: 2.16 eV) and Cs (the work function: 1.95 eV) or at least one alkaline earth metals selected from a group consisting of Ca (the work function: 2.9 eV), Sr (the work function: 2.0 to 2.5 eV) and Ba (the work function: 2.52 eV); whose work function of 2.9 eV is particularly preferable. Among those, more preferable reductive dopants include at least one kind selected from the group consisting of K, Rb and Cs, the latter Rb or Cs being farther more preferable and the last Cs being the most preferable. Those alkaline metals have particularly high reducing capability, and only an addition of relatively small amount of them into an electron injection zone enables to expect both improvement of luminance and lifetime extension of the organic EL device.

Examples of the alkaline earth metal compound described above include BaO, SrO, CaO and mixtures thereof such as BaₓSr₁₋ₓO (0<x<1) and BaₓCa_{1·x}O (0<x<1). Examples of the alkali oxide or alkali fluoride include LiF, Li₂O, Na F, etc. The alkali metal complex, the alkaline earth metal complex and the rare earth metal complex are not particularly limited as long as the complexes contain at least one of the alkali metal ions, the alkaline earth metal ions and rare earth metal ions, respectively, as the metal ion. As the ligand, quinolinol, benzoquinolinol, acridinol, phenanthridinol, hydroxyphenyloxazole, hydroxyphenylthiazole, hydroxydiaryloxadiazoles, hydroxydiarylthiadiazoles, hydroxyphenylpyridine, hydroxyphenyl- benzimidazole, hydroxybenzotriazole, hydroxyfulvorane, bipyridyl, phenanthroline, phthalocyanine, porphyrin, cyclopentadiene, β-diketones, azomethines and derivatives of these compounds are preferable. However, the ligand is not limited to the ligands described above.

As for the addition form of the reductive dopant, it is preferable that the reductive dopant is added in a manner such that a layer or islands are formed in the interfacial zone described above. In the case where the layer of the reductive dopant is formed, a preferable film thickness is from 0.05 to 8 nm.

As the process for adding the reductive dopant, it is preferable that an organic material which is the light emitting material or the electron injecting material forming the interfacial region is vaporized while the reductive dopant is simultaneously vapor deposited in accordance with the resistance heating deposition method so that the reductive dopant is dispersed in the organic material. The concentration of the dispersion expressed as the ratio of the amounts by mole of the organic substance to the reductive dopant is in the range of 100:1 to 1:100 and preferably in the range of 5:1 to 1:5. When the reductive dopant is added to form a layer, the reductive dopant alone is vapor deposited in accordance with the resistance heating deposition method to form a layer preferably having a thickness of 0.1 to 15 nm after a layer of the organic material such as the light emitting material and the electron injecting material is formed as the interfacial region. When the reductive dopant is added to form islands, the reductive dopant alone is vapor deposited in accordance with the resistance heating deposition method to form islands preferably having a thickness of 0.1 to 15 nm after islands of the organic material such as the light emitting material and the electron injecting material were formed as the interfacial region.

It is preferable that the light emitting layer in the organic EL device of the present invention has functions capable of injecting holes from the anode or the hole injecting layer when an electric field is applied, of injecting electrons from the cathode or the electron injecting layer, of mobilizing the injected electric charges (electrons and holes) by means of the electric field, and of providing a space for recombination of the electrons and holes thereby urging the light emission. It is preferable for the organic EL device of the present invention that the light emitting layer at least comprises the metal complex compound of the present invention, and it may comprise a host material which employs the metal complex compound as a guest material. Examples of the above host material include such as those having a carbazole skeletal structure, those having a diarylamine skeletal structure, those having a pyridine skeletal structure, those having a pyrazine skeletal structure, those having a triazine skeletal structure, those having an arylsilane skeletal structure, etc. It is preferable that T1 (energy level in the minimum triplet excitation state) of the host material is larger than T1 level of the guest material. The host material may be either a low molecular weight compound or a high molecular weight compound. Further, the light emitting layer in which the above light emitting materials are doped into thr above host materials can be formed by codeposition of the host materials and the light emitting materials such as the above metal-complex compound, etc.

In the organic EL device of the present invention, although a process for forming each layers are not particularly specified, various kinds of process such as a vacuum vapor deposition process, a LB process, a resistance heating deposition process, an electron beam process, a sputtering process, a molecular lamination process, a coating process (a spin coating process, a casting process, a dip coating process), an ink-jet process, a printing process are employable and the coating process of applying the materials over a substrate is preferable in the present invention.

The organic thin film layer comprising the metal-complex compound of the present invention can be formed in accordance with the vacuum vapor deposition process, a molecular beam epitaxy process (the MBE process) or, using a solution prepared by dissolving the compound into a solvent, in accordance with a conventional coating process such as the dip coating process, the spin with a conventional coating process such as the dip coating process, the spin coating process, the casting process, a bar coating process and a roller coating process.

In the above coating process, preparing a coating solution by dissolving the metal-complex compound of the present invention into a solvent, and by applying the coating solution over the surface of a predetermined layer (or, electrode), followed by drying may form the organic thin film layer. In the coating solution, a resin may be contained either by dissolved in a solvent or by dispersing into the solvent. Regarding with the resin, both non-conjugate high polymer (for example, polyvinylcarbazole) and conjugate high polymer (for example, polyolefin-based high polymer) are employable. Specific examples include polyvinylchloride, polycarbonate, polystyrene, polymethyl methacrylate, poly butyhnethacrylate, polyester, polysulfone, polyphenylene oxide, polybutadiene, poly (N-vinyl carbazole), hydrocarbon resin, ketone resin, phenoxy resin, polyamide, ethyl cellulose, vinyl acetate, ABS resin, polyurethane, melamine resin, unsaturated polyester resin, alkyd resin, epoxide resin, silicone resin, etc.

The thickness of each layer in the organic thin film layer in the organic EL device of the present invention is not particularly limited. In general, an excessively thin layer tends to have defects such as pin holes, and an excessively thick layer requires a high applied voltage results in decreasing the efficiency. Therefore, a thickness within the range of several nanometers to 1 *µ*m is preferable.

The present invention will be described in more detail by reference to the following examples. However, it should be noted that these examples are only illustrative and not intended to limit the invention thereto.

### Synthesis Example 1 (Synthesis of Metal-Complex Compound 1-1)

The route for synthesis of Metal-Complex Compound 1-1 is shown as the following.

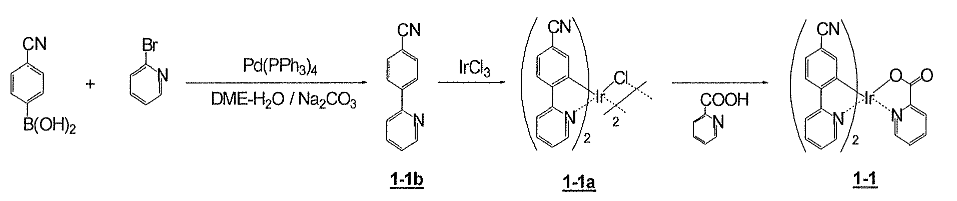

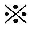 Structural formula of Intermediate 1-1a expresses dimer configuration.

(1) Synthesis of Intermediate 1-1b
   Placing 4-cyanophenyl boronic acid in an amount of 11.7 g (75.3 mmol) and Pd(PPh₃) in an amount of 42.26 g (1.95 mmol) into a flask, replacing the atmosphere with argon gas, and adding ethylene glycol dimethylether in an amount of 500 milliliter, 1.3M Na₂CO₃ aqueous solution in an amount of 150 milliliter, followed by further adding 2-bromopyridine in an amount of 11.9 g (75.3 mmol), the resultant mixture was reacted for 7 hours under refluxing. Distillating away the solvent from the reacted solution, dichloromethane was extracted. After washing the separated organic layer with water several times, the layer was dried with the use of magnesium sulfate. After concentration, the resultant crystals were purified by means of a silica column chromatography (developing solution: CH₂Cl₂ / hexane= 1/1) and as a result, 9.9 g of Intermediate 1-1b as white crystals was obtained (yield: 73 %). The white crystals were confirmed as the aimed compound from ¹H-NMR spectrum. The measurement result is shown as follows:
   ¹H-NMR (CDCl₃): δ 8.69-8.77 (m,1H), δ 7.7-8.2 (m,6H), δ 7.24-7.39 (m, 1H)
(2) Synthesis of Intermediate 1-1a
   Placing Intermediate 1-1b in an amount of 4.34 g (24.1 mmol) and IrCl₃ hydrate (available from Strem Chemicals, Inc.) in an amount of 1.8 g (6.02 mmol) into a flask, replacing the atmosphere with argon gas, and after pouring 2-ethoxyethanol in an amount of 30 milliliter, the resultant mixture was reacted for 7 hours under refluxing. A resultant brown precipitation was separated by filtration, followed by washing twice with the use of ethanol in an amount of 5 milliliter. Further, it was dissolved in 400 milliliter of methylene chloride, and the resultant solution was washed with the use of HCl aqueous solution in an amount of 200 milliliter, followed by washing twice with the use of water in an amount of 200 milliliter. After drying the resultant solution with the use of magnesium sulfate, the solvent was distillated away and as a result, 1.72 g of Intermediate 1-1a as brown crystals was obtained (yield: 49 %).
(3) Synthesis of Metal-Complex Compound 1-1
   Placing Intermediate 1-1a in an amount of 1.72 g (1.46 mmol) and 2-pyridinecarboxylic acid in an amount of 0.722 g (5.86 mmol) into a flask, replacing the atmosphere with argon gas, and after pouring 1,2-dichloroethane in an amount of 35 milliliter, the resultant mixture was reacted for 7 hours under refluxing. Distillating away the solvent from the reacted solution and dissolving the resultant dark yellow crystals into 300 milliliter of methylene chloride, the resultant solution was washed with the use of water in an amount of 150 milliliter and then, an organic layer was dried and after distillating away the solvent, yellow crystals were obtained. Washing the resultant crystals with the use of extremely slight amount of methylene chloride, and after drying them, 0.4 g of Metal-Complex Compound 1-1 was obtained (yield: 20 %). The yellow crystals were confirmed as the aimed compound from ¹H-NMR spectrum and from the result in accordance with Field Desorption Mass Spectrum (FD-MS) analysis. The measurement result is shown as follows:
   ¹H-NMR (CD₂Cl₂): δ 8.75 (d,1H), δ 7.0-8.25 (m,15H), δ 6.52 (d, 2H)
   FD-MS: m/z= 673
   Further, by measuring phosphorus light (methylene chloride solution) of the resultant Metal-Complex Compound 1-1, it was found that λ max (wave length of peak light emission intensity) of phosphorus light was 528 nm.

### Synthesis Example 2 (Synthesis of Metal-Complex Compound 1-6)

The route of synthesis of Metal-Complex Compound 1-6 is shown in the following. 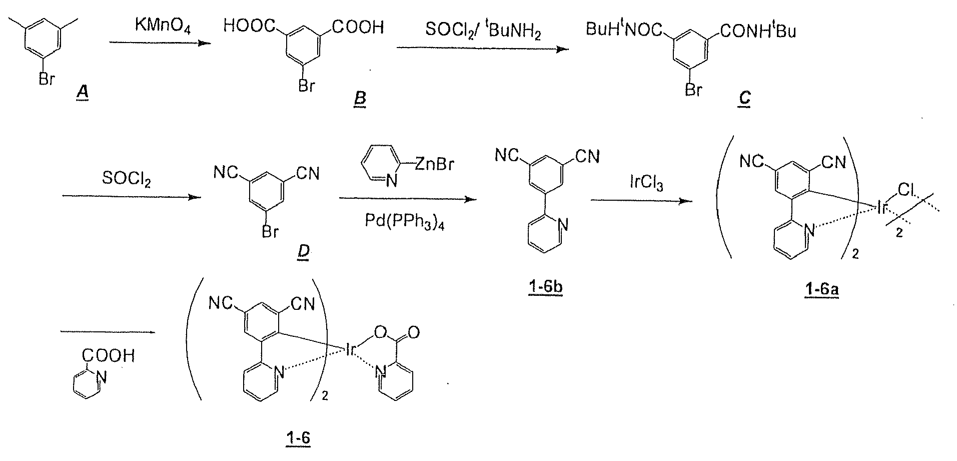
(1) Synthesis of Intermediate Product B
   Placing Compound A in an amount of 25.2 g (136 mmol) and KMnO₄ in an amount of 86.1 g (544 mmol) into a flask, and the resultant solution was reacted in water for 9 hours under refluxing. After the reaction terminated, a precipitate was filtered and the filtrate was processed with 6N hydrochloric acid at room temperature. A deposit was washed with the use of 0. 1N hydrochloric acid and dried, resultantly obtaining 11.4 g of Intermediate B (yield: 34 %).
(2) Synthesis of Intermediate C
   Placing Intermediate B in an amount of 25.0 g (102mmol) and thionyl chloride in an amount of 250 g into a flask, the resultant solution was reacted for 7 hours under refluxing. Dissolving a resultant solid into 250 milliliter of toluene, a solution prepared by dissolving t-butylamine in an amount of 37.7 g (515 mmol) into toluene was dripped into the former solution at a temperature of 5 to 18 °C. The temperature of the resultant solution was elevated little by little up to the room temperature, and adding 200 milliliter of water, the solution was stirred. A deposit was separated by filtration and as a result, 35.0 g of Compound C was obtained (yield: 96 %).
(3) Synthesis of Intermediate D
   Placing Intermediate C in an amount of 34.5 g (97.1 mmol) and thionyl chloride in an amount of 250 g into a flask, the resultant solution was refluxed for about 1.5 hours. Distillating away an excesst of thionyl chloride, a resultant yellow solid was purified by means of silica column (toluene). Distillating away the solvent from a resultant mixture, 14.7 g of Intermediate D as white crystals was obtained (yield: 73.1 %).
(4) Synthesis of Intermediate 1-6b
   Placing 2-bromopyridine in an amount of 58.9 g (373 mmol) and tetrahydrofuran (THF) in an amount of 58.9 g into a flask, a THF solution of 2.5M isopropyl magnesium chloride in an amount of 150 g (375 mmol) was dripped under cooling with ice, and the resultant solution was reacted at room temperature for 2 hours. Subsequently, Grignard agent prepared above was added into THF solution of anhydrous zinc bromide in an amount of 84.8g (377 mmol) spending 15 minutes, the resultant solution was further reacted for 2.5 hours at room temperature. Throwing Compound D in an amount of 13.9 g (67.1 mmol) and Pd(PPh₃)₄ in an amount of 0.97g (0.84 mmol) into the reacted solution, the resultant solution was further reacted at a temperature of 50 °C for 6 hours. After condensing the resultant reacted solution, it was extracted with the use of methylen chloride and a separated organic layer was washed several times with the use of water, followed by drying with the use of magnesium sulfate. After condensation, the resultant crystals were purified by means of a silica column chromatography (developing solution: toluene / ethyl acetate =10/1), and as a result, 6.5 g of Intermediate 1-6b as white crystals were obtained (yield: 47 %). Further, the white crystals were confirmed as the aimed compound from the result in accordance with Gas Chromatography-Mass Spectrometry (GC-MS). The measurement result is shown as follows:
   GC-MS: m/z= 206(H⁺)
(5) Synthesis of Intermediate 1-6a
   Placing Intermediate 1-6b in an amount of 41 g (20.0 mmol) and IrCl₃ hydrate (available from Strem Chemicals, Inc.) in an amount of 1.5 g (5.0 mmol) into a flask, the atmosphere was replaced with argon gas. Pouring 25 milliliter of 2-ethoxyethanol into the resultant solution, it was reacted for 16 hours under refluxing. A resultant yellow precipitation was separated by filtration, followed by washing with the use of methanol in an amount of 100 milliliter. Drying the separated precipitation, 1.74 g of Intermediate 1-6a as yellow crystals wwas obtained (yield: 55 %).
(6) Synthesis of Metal-Complex Compound 1-6
   Placing Intermediate 1-6a in an amount of 1.72 g (1.35mmol) and 2-pyridinecarboxylic acid in an amount of 0.666 g (5.4mmol) into a flask, the atmosphere was replaced with argon gas. Pouring 50 milliliter of 1,2-dichloroethane into the resultant solution, it was reacted for 18 hours under refluxing. Distillating away the solvent from the reacted solution and dissolving the resultant dark yellow crystals into 800 milliliter of methylene chloride, the resultant solution was washed with the use of water in an amount of 200 milliliter and then, an organic layer was dried and after distillating away the solvent, pale yellow crystals were obtained. Washing the resultant crystals with the use of extremely slight amount of methylene chloride, and after drying them, 0.5 g of Compound 1-6 was obtained (yield: 40 %). It was confirmed in accordance with FD-MS that Compound 1-6 was the aimed compound. The measurement result is shown as follows:
   FD-MS: m/z = 723

Further, by measuring phosphorus light (methylene chloride solution) of the resultant Metal-Complex Compound 1-6, it was found that λ max of phosphorus light was 471 nm.

### Synthesis Example 3 (Synthesis of Metal-Complex Compound 1-5)

The route of synthesis of Metal-Complex Compound 1-5 is shown in the following. 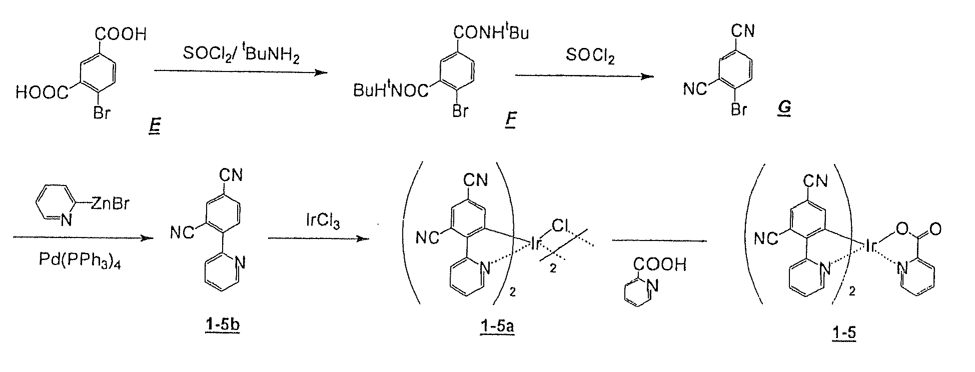
(1) Synthesis of Intermediate F
   Placing Compound E in an amount of 35.1 g(143 mmol) and thionyl chloride in an amount of 407 g into a flask, the resultant solution was reacted for 4.5 hours under refluxing. Dissolving the resultant yellow oil into 700 milliliter of toluene, a toluene solution of t-n-butylamine in an amount of 52.4 g (716mmol) was dripped slowly under cooling with ice, the resultant solution was warmed up to room temperature, and as a result, a resultant precipitate was separated by filtration. After drying, 50.2 g of Intermediate F was obtained (yield: 98 %).
(2) Synthesis of Intermediate G
   Placing Compound F in an amount of 42.0 g(118 mmol) and thionyl chloride in an amount of 285 milliliter into a flask, the resultant solution was reacted for 4.5 hours under refluxing. Distillating away an excess of thionyl chloride, a resultant yellow solid was washed with a use of ethyl acetate and toluene. After drying, 20.1 g of Intermediate G as white crystals was obtained (yield: 82.1 %).
(3) Synthesis of Intermediate 1-5b
   Placing 2-bromopyridine in an amount of 83.7 g (530 mmol) and tetrahydrofuran (THF) in an amount of 83.7 g into a flask, a THF solution of 2.5M isopropyl magnesium chloride in an amount of 214 g (530 mmol) was dripped under cooling with ice, and the resultant solution was reacted at room temperature for 3 hours. Subsequently, Grignard agent prepared above was added into THF solution of anhydrous zinc bromide in an amount of 120.5 g (535 mmol), the resultant solution was further reacted for 2 hours at room temperature. Throwing Conpound G in an amount of 18.5 g (89.4 mmol) and Pd(PPh₃)₄ in an amount of 1.2 g (1.0 mmol) into the reacted solution, the resultant solution was further reacted at a temperature of 50°C for 10 hours. After condensing the resultant reacted solution, it was extracted with the use of methylen chloride and a separated organic layer was washed several times with the use of water, followed by drying with the use of magnesium sulfate. After condensation, the resultant crystals were purified by means of a silica column chromatography (developing solution: toluene / ethyl acetate =10/1), and as a result, 8.28 g of Intermediate 1-5b as white crystals were obtained (yield: 45.2 %). It was confirmed in accordance with GC-MS that Intermediate 1-5b was the aimed compound. The measurement result is shown as follows:
   GC-MS: m/z=205
(4) Synthesis of Intermediate 1-5a
   Placing Intermediate 1-5b in an amount of 41 g (20.0 mmol) and IrC13 hydrate (available from Strem Chemicals, Inc.) in an amount of 15 g (5.0 mmol) into a flask, the atmosphere was replaced with argon gas. Pouring 25 milliliter of 2-ethoxyethanol into the resultant solution, it was reacted for 24 hours under refluxing. A resultant yellow ocher precipitation was separated by filtration, followed by washing with the use of methanol in an amount of 200 milliliter. Drying the separated precipitation, 1.1 g of Intermediate 1-5a as yellow crystals was obtained (yield: 35 %).
(5) Synthesis of Metal-Complex Compound 1-5
   Placing Intermediate 1-5a in an amount of 1.1 g (0.86 mmol) and 2-pyridinecarboxylic acid in an amount of 0.43 g (35 mmol) into a flask, the atmosphere was replaced with argon gas. Pouring 60 milliliter of 1,2-dichloroethane into the resultant solution, it was reacted for 24 hours under refluxing. Distillating away the solvent from the reacted solution and dissolving the resultant orange crystals into 900 milliliter of methylene chloride, the resultant solution was washed with the use of water in an amount of 300 milliliter and then, an organic layer was dried with the use of magnesium sulfate and after distillating away the solvent, orange crystals were obtained. After drying, 0.45 g of Compound 1-5 was obtained (yield: 36 %). It was confirmed in accordance with FD-MS that Compound 1-5 was the aimed compound. The measurement result is shown as follows:
   FD-MS: m/z= 723

Further, by measuring phosphorus light (methylene chloride solution) of the resultant Metal-Complex Compound 1-5, it was found that λ max of phosphorus light was 545 nm

### Synthesis Example 4 (Synthesis of Metal-Complex Compound 1-6X)

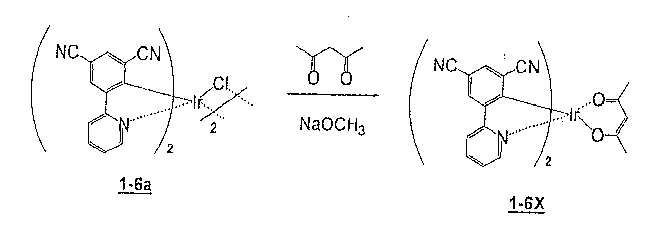

Placing the above Intermediate 1-6a in an amount of 320 g (2.52 mmol) into a flask, replacing the atmosphere with argon gas, and after pouring 1,2-dichloroethane in an amount of 150 milliliter, the resultant mixture was stirred at the room temperature. On the other hand, dissolving acetylacetone in an amount of 0.55 g (5.5 mmol) into 10 milliliter of ethanol was reacted with 28 % sodium methoxide methanol solution in an amount of 10 g (5.5 mmol equivalent) at the room temperature. Adding the resultant solution into the above Intermediate 1-6a solution, it was reacted for 17 hours under refluxing. Distillating away the solvent from the reacted solution and dissolving the resultant dark yellow crystals into 1000 milliliter of methylene chloride, the resultant solution was washed with the use of water in an amount of 500 milliliter and then, an organic layer was dried with the use of magnesium sulfate and again, the solvent was distillated away. The resultant crystals were purified by means of a silica column chromatography (developing solution: ethyl acetate / methylene chloride =1/4) and as a result, 0.7 g of pale yellow Compound 1-6X was obtained (yield: 20 %). It was confirmed in accordance with FD-MS that Compound 1-6X was the aimed compound. The measurement result is shown as follows:
FD-MS : m/z= 700

Further, by measuring phosphorus light (methylene chloride solution) of the resultant Metal-Complex Compound 1-6X, it was found that λ max of phosphorus light was 478 nm

### Example 1 (Fabrication of organic EL device)

A glass substrate of 25 mm×75 mm×1.1 mm thickness having an ITO transparent electrode was cleaned by application of ultrasonic wave in isopropyl alcohol for 5 minutes and then by exposure to ozone generated by ultraviolet light for 30 minutes. The cleaned substrate having the transparent electrode lines was attached to a substrate holder, and on the surface of the cleaned substrate at the side having the transparent electrode, a film of 4,4'-bis [N-(4-biphenyl)-N-(4-biphenyl)amino] bipheny having a thickness of 40 nm was formed so that the formed film covered the transparent electrode. The formed film worked as the hole transporting layer. Further, a host material (CBP) below together with additioning a photoluminescent Ir-Complex Compound 1-1 as a dopant were deposited obtaining a film thickness of 30 nm on the formed fimd. The formed film worked as a light emitting layer. A concentration of Compound 1-1 in the light emitting layer was 5 % by weight. On the light emitting layer, a film of (1,1'-bisphenyl)-4-olate)bis(2-methyl-8-quinolinolato) aluminium (BAlq) below having a thickness of 10 nm was formed.The formed film of BAlq worked as the hole barrier layer. On the film formed above, a film of 8-hydroxyquinoline (Alq) having a thickness of 30 nm was formed. The film of Alq worked as the electron injecting layer. Subsequently, lithium fluoride being alkali metal halide was deposited up to 0.15 nm in thickness and then, aluminum was deposited up to 150 nm in thickness. The Al/LiF worked as a cathode. An organic EL device was fabricated in the manner described above. The device fabricated above was examined by passing electric current. Yellowish green light was emitted at a luminance of 107 cd/m² under a voltage of 6.6 V and a current density of 4.15 mA/cm². The CIE chromaticity coordinates were (0.401, 0.568), and the current efficiency was 2.58 cd/A. Additionally, λ max of light emission was 531 nm.

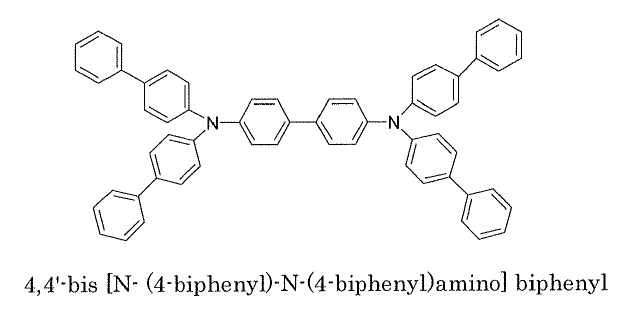

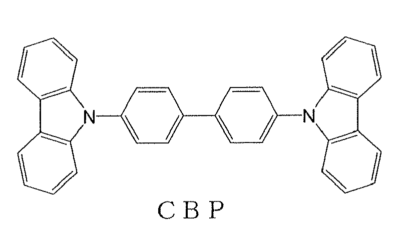

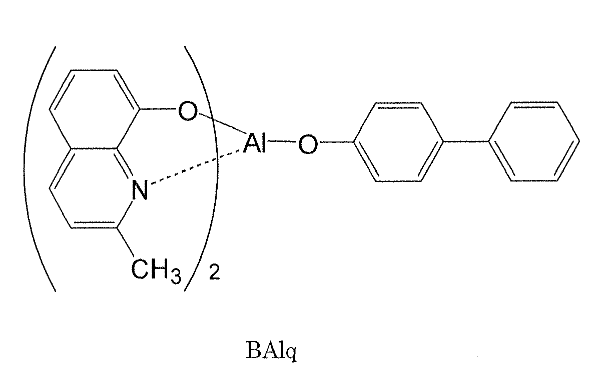

### Example 2 (Fabrication of organic EL device)

A glass substrate of 25 mm×75 mm×1.1 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode which had been cleaned was attached to a substrate holder of a vacuum vapor deposition apparatus. On the surface of the cleaned substrate at the side having the transparent electrode, a film of TPD232 having a thickness of 100 nm was formed so that the formed film covered the transparent electrode. The formed film of TPD232 worked as the hole injecting layer. Further on the formed film, 4,4',4"-tris(carbazole-9-yl)-triphenylamine (TCTA) below was film-formed obtaining a film thickness of 10 nm. The formed film of TCTA film worked as a hole transporting layer. Further, Compound (A) below was deposited obtaining a film thickness of 30 nm on the above TCTA film, simultaneously adding a phosphorous photoluminescent Ir Metal-Complex Compound 1-6X as a dopant. A concentration of Compound 1-6X in the light emitting layer was 7.5 % by weight. The formed film worked as a light emitting layer. On the formed film, Compound (C-12) below was film-formed obtaining a film thickness of 25 nm. The formed film worked as an electron transport assisting layer. On the film formed above, a film of Alq having a thickness of 5 nm was formed. The film of Alq worked as the electron injecting layer. Subsequently, lithium fluoride was deposited up to 0.1 nm in thickness and then, aluminum was deposited up to 150 nm in thickness. The Al/LiF worked as a cathode. An organic EL device was fabricated in the manner descried above.

The device fabricated above was examined by passing electric current. Blueish green light was emitted at a luminance of 104 cd/m² under a voltage of 3.9 V and a current density of 0.26 mA/cm², and the current efficiency was 39.6 cd/A.

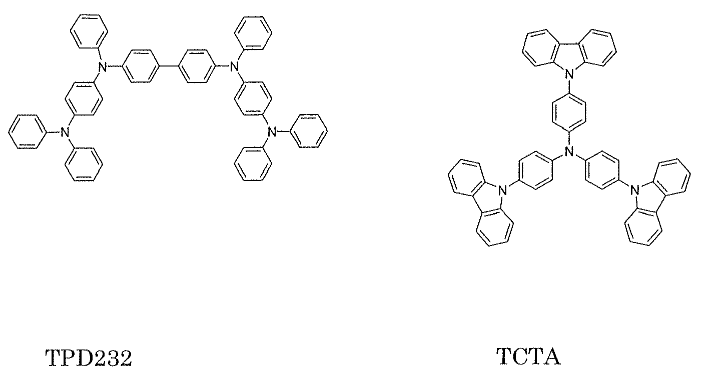

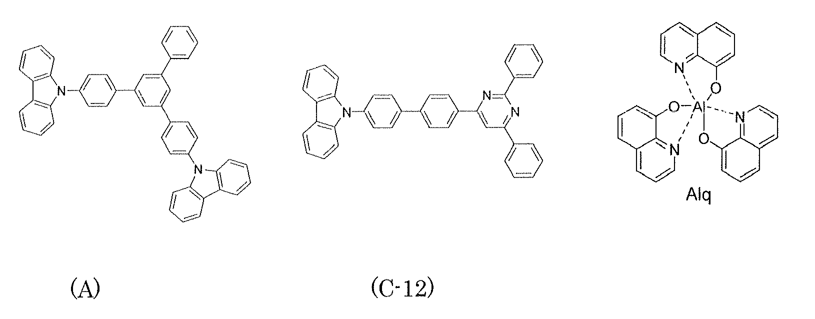

### Comparative Example 1

An organic EL devices was fabricated similarly as Example 2 except that CFIracac below was used instead of the phosphorus photoluminescent Metal-Complex Compound 1-6X. Measuring voltage, current density, luminance and current efficiency, the measured results are shown in Table 1 together with colors of light emission.

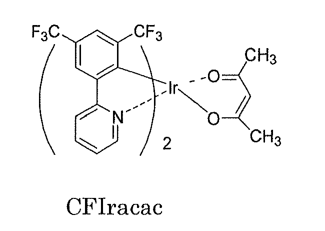

### Comparative Example 2

An organic EL devices was fabricated similarly as Example 2 except that FIracac below was used instead of the phosphorus photoluminescent Metal-Complex Compound 1-6 X. Measuring voltage, current density, luminance and current efficiency, the measured results are shown in Table 1 together with colors of light emission.

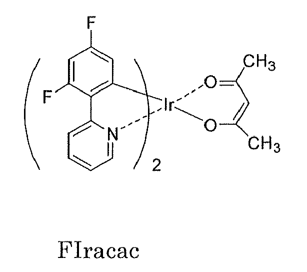

### Example 3

An organic EL devices was fabricated similarly as Example 2 except that phosphorus photoluminescent Metal-Complex Compound 1-6 was used instead of the phosphorus photoluminescent Metal-Complex Compound 1-6X. Measuring voltage, current density, luminance and current efficiency, the measured results are shown in Table 1 together with colors of light emission.

### Comparative Example 3

An organic EL devices was fabricated similarly as Example 2 except that FIrpic below was used instead of the phosphorus photoluminescent Metal-Complex Compound 1-6 X. Measuring voltage, current density, luminance and current efficiency, the measured results are shown in Table 1 together with colors of light emission.

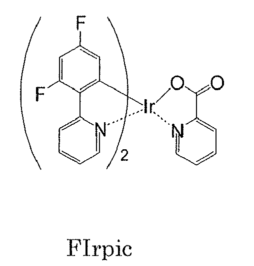

### Comparative Example 4

An organic EL devices was fabricated similarly as Example 2 except that CFIrpic below was used instead of the phosphorus photoluminescent Metal-Complex Compound 1-6 X.

Measuring voltage, current density, luminance and current efficiency, the measured results are shown in Table 1 together with colors of light emission.

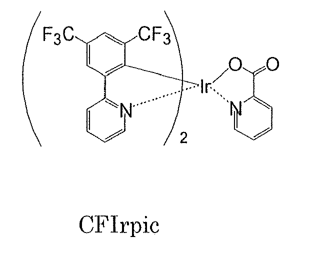

**Table 1**

| | Dopant in Light emitting layer | Voltage (V) | Current Dencity (mA/cm²) | Luminance (cd/m²) | Current efficiency (cd/A) | Color of Light Emission |
|---|---|---|---|---|---|---|
| Ex. 2 | 1-6X | 3.9 | 0.26 | 103 | 39.6 | Bluish Green |
| Ex. 3 | 1-6 | 5.0 | 0.33 | 104 | 31.5 | Bluish Green |
| Co. Ex. 1 | CFI r acac | 7.2 | 0.51 | 100 | 19.6 | Bluish Green |
| Co. Ex. 2 | FIracac | 7.0 | 0.50 | 103 | 19.5 | Bluish Green |
| Co. Ex. 3 | FIrpic | 6. 1 | 0.75 | 106 | 13.8 | Bluish Green |
| Co. Ex. 4 | CFIrpic | 6.6 | 0.63 | 98 | 15.5 | Bluish Green |

As shown in Table 1, it is verified that the organic EL devices of Examples 2 to 3 employing of Metal-Complex Compound of the present invention become capable of working at low driving voltage and emitting bluish green light with enhanced current efficiency relative to Comparative Examples 1-4.

### INDUSTRIAL APPLICABILITY

As described above in detail, the organic EL device employing the novel metal-complex compound of the present invention emits various phosphorous lights including blue light having an enhanced current efficiency and prolonged lifetime. Accordingly, the present invention is applicable for a field such as various display devices, display panels, backlights, illuminating light sources, beacon lights, signboards, and interior designs, particularly suitable as display device for color displays.

## Claims

1. A metal-complex compound having a partial structure represented by a following general formula (I): 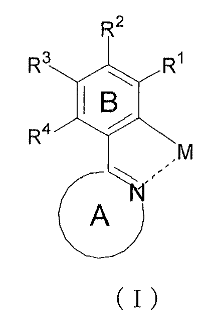 wherein Structure B represents a benzene ring structure having R¹ to R⁴; R¹ to R⁴ each independently represents a hydrogen atom, a cyano group, a halogen atom, a substituted or unsubstituted alkyl group having 1 to 20 carbon atoms, a substituted or unsubstituted amino group, a substituted or unsubstituted alkoxyl group having 1 to 20 carbon atoms, a substituted or unsubstituted aromatic group having 1 to 30 carbon atoms; at least one among R¹ to R⁴ is a cyano group; and a couple of R¹ and R², a couple of R² and R³, and a couple of R³ and R⁴ may bond each other to form a ring structure;
Structure A represents a ring structure having 3 to 20 carbon atoms, further having at least one carbon-nitrogen double bond and may have a substituent; which may form a ring structure having the foregoing R⁴; and
M represents any one metal atom selected from iridium (Ir) atom, rhodium (Rh) atom, platinum (Pt) atom or palladium (Pd) atom.

2. The metal-complex compound according to Claim 1, which is a material for a light emitting element.

3. The metal-complex compound according to Claim 1, wherein said Structure B represents a substituted benzene ring moiety represented by any one of following formulae:

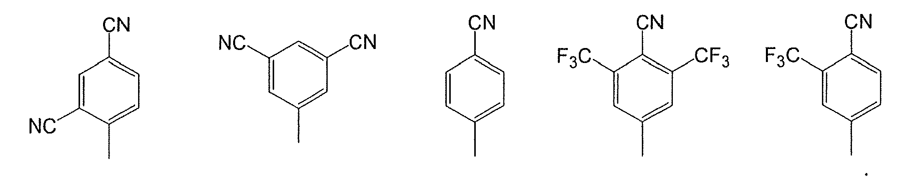

4. The metal-complex compound according to Claim 1, wherein said Structure A represents a group represented by any one of following formulae:

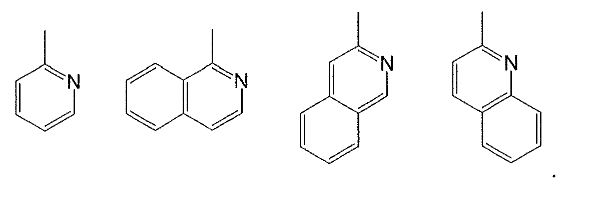

5. The metal-complex compound according to Claim 1, wherein said partial structure represented by the general formula (I) is expressed by any one of following formulae:

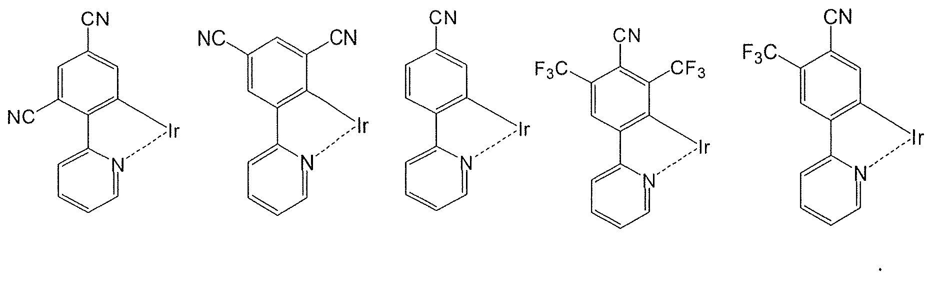

6. The metal-complex compound according to Claim 1, which is expressed by any one of following general formulae 1 to 8: 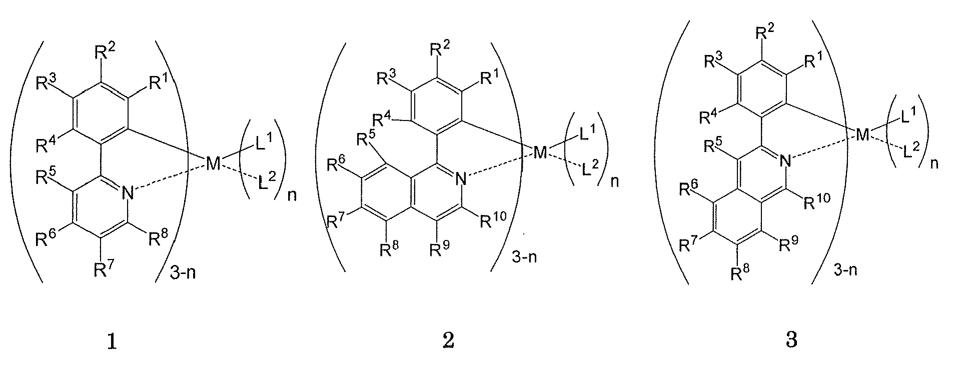 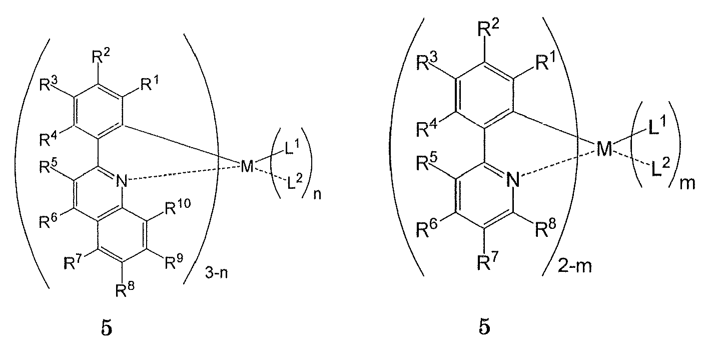 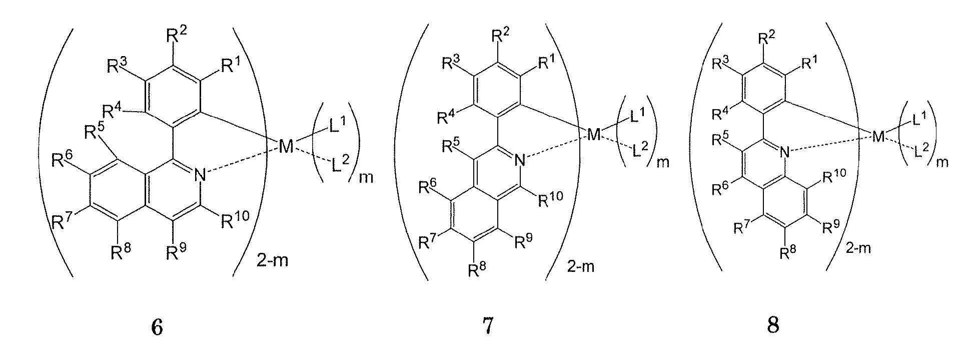 wherein R¹ to R¹⁰ each independently represents a hydrogen atom, a cyano group, a halogen atom, a substituted or unsubstituted alkyl group having 1 to 20 carbon atoms, a substituted or unsubstituted amino group, a substituted or unsubstituted alkoxyl group having 1 to 20 carbon atoms, a substituted or unsubstituted aromatic group having 1 to 30 carbon atoms; at least one among R¹ to R⁴ is a cyano group;
and a couple of R¹ and R², a couple of R² and R³, a couple of R³ and R⁴, a couple of R⁴ and R⁵, a couple of R⁵ and R⁶, a couple of R⁶ and R⁷, a couple of R⁸ and R⁹, and a couple of R⁹ and R¹⁰ may bond each other to form a ring structure;
M represents any one metal atom selected from iridium (Ir) atom, rhodium (Rh) atom, platinum (Pt) atom or palladium (Pd) atom; and
L¹ and L² each independently represents any one structure expressed by following structures: 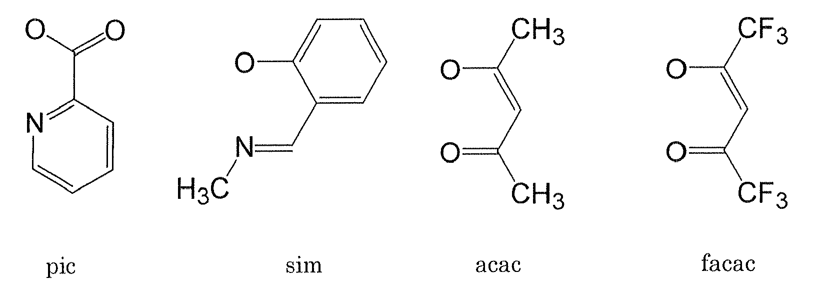 and
wherein n represents an integer of 0 to 2, and m represents an integer of 0 or 1.

7. An organic electroluminescence device which comprises at least one organic thin film layer sandwiched between a pair of electrode consisting of an anode and a cathode, wherein the organic thin film layer comprises the metal-complex compound according to any one of Claims 1 to 6, which emits light by applying an electric voltage between the pair of electrode.

8. The organic electroluminescence device according to Claim 7, wherein said light emitting layer comprises said metal-complex compound.

9. The organic electroluminescence device according to Claim 7, wherein at least one of an electron injecting layer or an electron transporting layer with a π -electron lacking heteroring derivative having a nitrogen atom as its essential component sandwiched between said light emitting layer and said cathode.

10. The organic electroluminescence device according to Claim 7, wherein a reductive dopant is added in an interfacial region between said cathode and said organic thin film layer.

11. The organic electroluminescence device according to Claim 7, wherein said organic thin film layer comprising the metal-complex compound is formed by coating.
